(19) Europäisches Patentamt
European Patent Office
Office européen des brevets

(11) **EP 3 250 644 B1**

(12) **EUROPÄISCHE PATENTSCHRIFT**

(45) Veröffentlichungstag und Bekanntmachung des Hinweises auf die Patenterteilung:
**19.08.2020   Patentblatt 2020/34**

(21) Anmeldenummer: **16705423.8**

(22) Anmeldetag: **28.01.2016**

(51) Int Cl.:
*C09C 1/62* (2006.01)         *C09C 1/64* (2006.01)
*C09C 1/66* (2006.01)         *A61K 8/02* (2006.01)
*A61K 8/26* (2006.01)         *A61K 8/81* (2006.01)
*A61K 8/891* (2006.01)       *A61Q 1/02* (2006.01)
*C08K 9/08* (2006.01)         *C09D 5/03* (2006.01)
*C09D 5/29* (2006.01)         *C09D 7/62* (2018.01)
*C09D 7/40* (2018.01)

(86) Internationale Anmeldenummer:
**PCT/EP2016/000143**

(87) Internationale Veröffentlichungsnummer:
**WO 2016/120015 (04.08.2016 Gazette 2016/31)**

(54) **BESCHICHTETE PIGMENTE, VERFAHREN ZU DEREN HERSTELLUNG UND DEREN VERWENDUNG, BESCHICHTUNGSMITTEL UND GEGENSTAND**

COATED PIGMENTS, METHOD FOR THEIR PREPARATION AND THEIR USE, COATING AGENT AND ITEM

PIGMENTS REVÊTUS, SON PROCÉDÉ DE FABRICATION ET D'UTILISATION, AGENT DE REVÊTEMENT ET OBJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.01.2015   EP 15152898**

(43) Veröffentlichungstag der Anmeldung:
**06.12.2017   Patentblatt 2017/49**

(73) Patentinhaber: **Eckart GmbH**
**91235 Hartenstein (DE)**

(72) Erfinder:
• **STEIN-HOFMANN, Simone**
**91220 Schnaittach (DE)**
• **BEDFORD, Oliver**
**64372 Ober-Ramstadt (DE)**
• **STRUCK, Oliver**
**91239 Henfenfeld (DE)**
• **SCHNEIDER, Ralph**
**91207 Lauf a.d. Pegnitz (DE)**

(74) Vertreter: **Altana IP Department**
**Altana Management Services GmbH**
**Abelstraße 45**
**46483 Wesel (DE)**

(56) Entgegenhaltungen:
**EP-B1- 1 874 874         CN-A- 101 580 653**
**CN-A- 102 516 831       DE-A1-102005 037 611**

## Beschreibung

Die Erfindung betrifft Pigmente mit metallischem Substrat und Beschichtung sowie Verfahren zu deren Herstellung und deren Verwendung. Die Erfindung betrifft weiterhin Beschichtungsmittel umfassend derartige Pigmente sowie beschichtete Gegenstände.

Die WO 2013/064643 A1 und WO 2014/048887 A1 betreffen eine zweistufige Beschichtung, wobei eine Kombination aus anorganischer Beschichtung und organischer Beschichtung zur Erzielung verbesserter Eigenschaften genutzt wird. Die Verwendung einer anorganisch/organischen Hybridschicht und insbesondere der erfindungsgemäße spezifische Aufbau werden jedoch nicht offenbart.

Die US 2008/0249209 A1 beschreibt die Verwendung von anorganisch/organischen Mischschichten zur Beschichtung von Metalleffektpigmenten. Jedoch wird keine Beschichtung der erfindungsgemäßen spezifischen Zusammensetzung offenbart oder nahegelegt, dass hiermit verbesserte Eigenschaften erzielt werden können.

CN 102 516 831 offenbart plättchenförmige Aluminiumpigmente, die mit einer anorganisch/organischen Hybridschicht überzogen sind und Metalloxide enthalten.

DE102005037611 offenbart Metalleffektpigmente bestehend aus plättchenförmigen Metallsubstraten (z.B. Aluminium), die mit einer anorganisch/organischen Hybridschicht beschichtet sind.

Die vorgenannten Beschichtungsvarianten weisen zumindest teilweise bereits sehr gute Stabilitäten in typischen Stabilitätstests auf. Zur Vereinfachung der Formulierung entsprechender Beschichtungszusammensetzungen wäre es jedoch vorteilhaft deren Stabilität weiter zu verbessern, um den Einsatz neuer Stoffe zu ermöglichen und eine größere Sicherheit bei der Verwendung neuer Kombinationen bekannter Bestandteile zu gewährleisten. So zeigten die bisherigen Metallpigmente beispielsweise bei einem erhöhten Zusatz von Eisenoxid zu bestehenden Gasungstests nur eine unzureichende Stabilität. Auch erwies es sich als schwierig die erforderliche Gasungsstabilität bei deutlich erhöhten pH-Werten zu gewährleisten. Da Einsatzgebiete wie die Lackierung von Automobilen jedoch eine extrem hohe Zuverlässigkeit der Stabilität verlangen, besteht ein großes Bedürfnis Pigmente mit einer noch höheren Stabilität bereitzustellen. Gleichzeitig sollen umweltbedenkliche oder gesundheitsbedenkliche Bestandteile vermieden werden. Zugleich sollten jedoch die optischen Eigenschaften wie der Flopindex der Pigmente idealerweise beibehalten werden.

Somit ist es eine Aufgabe der vorliegenden Erfindung Pigmente zur Verfügung zu stellen, welche eine verbesserte Stabilität aufweisen. Ferner ist es eine Aufgabe der vorliegenden Erfindung, Verfahren zu deren Herstellung sowie deren Verwendungen bereitzustellen.

Ferner sollen vorteilhafterweise die optischen Eigenschaften von hiermit beschichteten Metalleffektpigmenten, insbesondere deren Helligkeitsflop, verbessert werden. Zudem sollen vorteilhafterweise andere anwendungsspezifische Eigenschaften wie die Schwitzwasserbeständigkeit zumindest beibehalten werden. Auch Verbesserungen hinsichtlich der Agglomerationsneigung von insbesondere PVD-Pigmenten wären vorteilhaft.

Überraschenderweise zeigte sich, dass beispielsweise die Stabilität von Pigmenten mit metallischem Substrat mittels einer anorganisch/organischen Hybridschicht einer spezifischen Zusammensetzung erhöht werden kann. Diese Aufgabe der vorliegenden Erfindung wird gelöst durch ein Pigment umfassend ein metallisches Substrat und mindestens eine anorganisch/organische Hybridschicht,
wobei die anorganisch/organische Hybridschicht mindestens ein Metalloxid, mindestens einen Netzwerkbildner und mindestens ein organisches Polymer umfasst, wobei das mindestens eine Metalloxid kein Oxidationsprodukt des metallischen Substrats darstellt und der Begriff Metalloxid Oxide, Hydroxide und Oxidhydrate der Metalle und Halbmetalle umfasst,
der Netzwerkbildner mindestens teilweise kovalent mit dem Metalloxid und dem organischen Polymer verbunden ist, wobei das Verhältnis der Menge an Metalloxid der Beschichtung zur spezifischen Oberfläche des unbeschichteten Metallpigments in einem Bereich von 16,1 mg/m$^2$ bis 25 mg/m$^2$ liegt und

das Verhältnis der Menge an organischem Polymer der anorganisch/organischen Hybridschicht zur spezifischen Oberfläche des unbeschichteten Metallpigments in einem Bereich von 3,9 mg/m$^2$ bis 10,1 mg/m$^2$ liegt. Besonders bevorzugt ist es, dass plättchenförmige metallische Substrate verwendet werden. Die vorgenannte Metalloxidmenge der Beschichtung bezieht sich auf die Beschichtung ohne gegebenenfalls aufgebrachte Nachbeschichtungen.

Unter einer anorganisch/organischen Hybridschicht wird im Sinne der vorliegenden Erfindung verstanden, dass hierin eine weitgehend homogene Schicht der anorganischen und organischen Bestandteile vorliegt, ohne dass sich diskrete aufeinander folgende Schichten ausbilden. Jedoch können sich beispielweise infolge von Entmischungsprozessen Einschlüsse oder matrixartige Strukturen entsprechender Bestandteile ausbilden. Vorzugsweise werden sehr homogene Hybridschichten erhalten, welche nur äußerst kleine Einschlüsse mit einer Größe von beispielsweise weniger als 50 nm, mehr bevorzugt weniger als 25 nm, aufweisen, wobei die Bestimmung der Größe der Einschlüsse vorzugsweise mittels Rasterelektronenmikroskopie erfolgt. Überraschenderweise hat sich gezeigt, dass sich derartige Hybridschichten im erfindungsgemäßen Bereich als besonders stabil erweisen.

Bevorzugte Formen der erfindungsgemäßen Pigmente finden sich in den Ansprüchen 1 bis 13 und den Aspekten 1 bis 22.

Ferner betrifft die vorliegende Erfindung ein Verfahren zur Herstellung von Metallpigmenten mit metallischem Substrat und Beschichtung, umfassend folgende Schritte:

    i) Umsetzen mindestens eines Metalloxidedukts, mindestens eines Edukts eines organischen Polymers und mindestens eines Netzwerkbildners in einer Flüssigphase unter Ausbildung einer Beschichtungszusammensetzung,
    ii) Aufbringen der Beschichtungszusammensetzung auf metallische Substrate unter Ausbildung eines anorganisch/organischen Hybridschicht,

wobei die anorganisch/organische Hybridschicht mindestens ein anorganisches Netzwerk umfassend mindestens ein Metalloxid und mindestens ein organisches Polymer aufweist und das anorganische Netzwerk und das organische Polymer kovalent miteinander verbunden sind,

wobei das mindestens eine Metalloxid kein Oxidationsprodukt des metallischen Substrats darstellt und der Begriff Metalloxid Oxide, Hydroxide und Oxidhydrate der Metalle und Halbmetalle umfasst,

der Netzwerkbildner zumindest teilweise mit dem Metalloxid und dem organischen Polymer verbunden ist,

das Verhältnis der Menge an Metalloxid der Beschichtung zur spezifischen Oberfläche des unbeschichteten Metallpigments in einem Bereich von 16,1 mg/m$^2$ bis 25 mg/m$^2$ liegt und

das Verhältnis der Menge organischem Polymer der anorganisch/organischen Hybridschicht zur spezifischen Oberfläche des unbeschichteten Metallpigments in einem Bereich von 3,9 mg/m$^2$ bis 10,1 mg/m$^2$ liegt. Hierbei muss verstanden werden, das die Schritte i) und ii) nicht getrennt durchgeführt werden müssen. Vielmehr ist es typischerweise bevorzugt, dass Schritt i) in Gegenwart des metallischen Substrats stattfindet, so dass direkt eine Abscheidung der anorganisch/organischen Hybridschicht auf der Oberfläche des metallischen Substrats stattfindet.

Bevorzugte Formen des erfindungsgemäßen Verfahrens finden sich in den Ansprüchen 14 bis 16 und den Aspekten 23 bis 32.

Ferner betrifft die vorliegende Erfindung die Verwendung der erfindungsgemäßen Pigmente in einem Kosmetikum, einem Kunststoff oder einem Beschichtungsmittel.

Bevorzugte Formen der erfindungsgemäßen Verwendungen sind in Anspruch 17 und den Aspekten 34 bis 35 angegeben.

Ferner betrifft die vorliegende Erfindung ein Beschichtungsmittel umfassend erfindungsgemäße Pigmente.

Ferner betrifft die vorliegende Erfindung einen Gegenstand, der die erfindungsgemäßen Pigmente oder die erfindungsgemäßen Beschichtungsmittel umfasst.

Der Begriff "Pigment umfassend ein metallisches Substrat" im Sinne der vorliegenden Erfindung umfasst sphärische und plättchenförmige metallhaltige Partikel, sofern dies nicht anders spezifiziert ist. Dies umfasst insbesondere Partikel bei denen mindestens eine Metallschicht auf ein nichtmetallisches Substrat aufgebracht ist und Partikel bestehend im Wesentlichen oder vorzugsweise vollständig aus mindestens einem Metall oder mindestens einer Metalllegierung.

Soweit nicht anderweitig spezifiziert, ist es in der vorliegenden Erfindung besonders bevorzugt, dass "im Wesentlichen" mindestens 95 %, mehr bevorzugt mindestens 99 %, bedeutet. Insbesondere ist es bevorzugt, dass etwas vollständig daraus besteht. Soweit es sich auf ein stoffliches Merkmal bezieht, wie beispielsweise der Gehalt eines spezifischen Metalls oder die Menge einer Beschichtung, so sind hiermit Gewichts-% gemeint, soweit keine anderweitige Spezifikation vorliegt.

Der Begriff "Metalleffektpigment" im Sinne der vorliegenden Erfindung bezeichnet plättchenförmige metallhaltige Partikel. Die plättchenförmigen metallhaltigen Partikel umfassen plättchenförmige Partikel bei denen mindestens eine Metallschicht auf ein nichtmetallisches plättchenförmiges Substrat aufgebracht ist und plättchenförmige Partikel bestehend im Wesentlichen oder vorzugsweise vollständig aus mindestens einem Metall oder mindestens einer Metalllegierung. Sofern nicht anders spezifiziert, umfassen die Begriffe "Pigment", "Metallpigment" und "Metalleffektpigment" bei der vorliegenden Erfindung auch eine Mehrzahl von Pigmenten, insbesondere falls die darauf bezogene Größe eine statistische Größe darstellt, welche nur an einer größeren Zahl an Partikeln in gemittelter Form erhalten werden kann. Sofern keine spezifischen Angaben hinsichtlich einer derartigen Mittelung angegeben sind, bezieht sich die entsprechende gemittelte Größe auf das arithmetische Mittel der betreffenden Größe. Der Begriff "nichtmetallisches Substrat" umfasst im Sinne der vorliegenden Erfindung beispielsweise Polymersubstrate, Glassubstrate, beispielsweise Glasplättchen, und rein metalloxidische Substrate, beispielsweise Siliciumoxidplättchen, Aluminiumoxidplättchen oder Titanoxidplättchen. Als nichtmetallisches Substrat können auch natürliche oder synthetische Glimmerplättchen verwendet werden. Insbesondere ist es jedoch bevorzugt, dass die metallischen Substrate der erfindungsgemäßen Pigmente im Wesentlichen oder vorzugsweise vollständig aus Metall oder Metalllegierungen bestehen.

Insbesondere ist es bei weiteren Ausführungsformen bevorzugt, dass das metallische Substrat plättchenförmig ist.

Der Begriff "Metallkern" im Sinne der vorliegenden Erfindung bezeichnet metallische Substrate, welche im Wesentlichen, vorzugsweise vollständig, aus Metall oder Metalllegierungen bestehen.

Der Begriff "plättchenförmig" im Sinne der vorliegenden Erfindung bedeutet, dass das betreffende Partikel in einer Dimensionen bedeutend kleiner ist verglichen mit den beiden verbleibenden. Das heißt, dass beispielsweise die mittlere Höhe des Partikels mindestens 10-mal kleiner ist als die mittlere Breite und Länge. Die Bestimmung der Ausmaße der Pigmente erfolgt mithilfe dem Fachmann geläufiger Verfahren, beispielsweise Rasterelektronenmikroskopie. Derartige Pigmente weisen besondere vorteilhafte Eigenschaften auf und stehen im Fokus der vorliegenden Erfindung. Beispielsweise erlaubt eine plättchenförmige Ausformung die gerichtete Reflexion von elektromagnetischer Strahlung wie sichtbarem Licht. Hierdurch können wiederum besondere optische Effekte erzielt werden. Eine möglichst einheitlich plane Ausformung erweist sich beispielsweise zur Erzielung einer besonders hohen Brillanz als vorteilhaft, da hiermit eine räumlich gleichgerichtete Reflexion der Pigmente erreicht werden kann und nur wenig Streuung auftritt.

Typischerweise werden die erfindungsgemäßen Pigmente aufgrund ihrer optischen Eigenschaften eingesetzt. Funktionelle Effekte wie beispielsweise Veränderungen der Erwärmung eines Gegenstands infolge von Sonneneinstrahlung können jedoch auch genutzt werden.

Die Aufarbeitung, Probenvorbereitung und Analyse der Pigmente erfolgt mittels dem Fachmann geläufiger Verfahren. Beispiele entsprechender Verfahren sind HPLC (Hochleistungsflüssigkeitschromatographie), GC-MS (Gaschromatographie mit Massenspektrometrie-Kopplung), NMR (Kernspinresonanzspektroskopie), XPS (Röntgenphotoelektronenspektroskopie), Elementaranalyse oder Kombinationen der vorgenannten untereinander oder mit anderen routinemäßig genutzten Verfahren. Beschreibungen entsprechender Einsatzmöglichkeiten finden sich beispielsweise in wissenschaftlichen Standardwerken, wissenschaftlichen Publikationen und Patentschriften wie der US 8,574,357 B2. Beispielsweise zur Bestimmung der Metalloxidmenge oder Polymermenge ist es essentiell Nachbeschichtungen oder Bestandteile beispielsweise einer Lackzusammensetzung, in der die Pigmente formuliert waren, zu entfernen. Derartige Reste werden beispielsweise vor der Messung durch Waschen der Pigmente in dem Fachmann bekannten Lösungsmitteln entfernt.

Die Bestimmung der spezifischen Oberfläche (BET-Oberfläche) erfolgt mittels handelsüblicher Messgeräte. Beispielsweise kann zur Messung ein BELSORP-mini II der Firma BEL Japan, Inc.verwendet werden. Bevorzugte Messmethode ist die 5-Punktmessung.

Der Begriff "anorganische Oxidkomponente" bezeichnet Metalloxide, wobei der Begriff Metall auch Halbmetalle umfasst und der Begriff Oxid auch Hydroxide und Oxidhydrate umfasst. Insbesondere ist es bevorzugt, dass es sich bei der anorganischen Oxidkomponente nicht um eine anorganische Heteropolysäure handelt. Ein besonders vorteilhaft eingesetztes Metalloxid ist Siliciumoxid. Die Bestimmung der Menge der "anorganischen Oxidkomponente" erfolgt durch Bestimmung der Gesamtmenge des enthaltenen oxidierten Metalls auch in der Form von beispielsweise Hydroxiden, Oxidhydraten, etc. mithilfe dem Fachmann geläufiger Verfahren. Beispiele hierfür sind Atomabsorptionsspektroskopie, Elementaranalyse, induktiv gekoppeltes Plasma mit Atomemmisionsspektroskopie (ICP-AES / Inductively coupled plasma atomic emission spectroscopy) und insbesondere Kombinationen bekannter Verfahren wie den vorgenannten. Die Berechnung des Wertes erfolgt über die detektierte Metallmenge. Sofern die Bestimmung des genauen Metalloxids oder der Metalloxidmischung enthaltenen Mischung nicht mithilfe dem Fachmann geläufiger Verfahren wie beispielsweise XPS erfolgen kann, wird das höchstoxidierte unter Normbedingungen stabile Oxid des Metalls als Referenz genommen. Normbedingungen im Sinne der vorliegenden Erfindung sind eine Temperatur von 0 °C und ein Druck von 1,01325 bar. Beispielsweise wird für Siliciumoxid $SiO_2$ als Referenz angenommen. Sofern der Netzwerkbildner entsprechende Bestandteile aufweist, beispielsweise bei spezifischen Organosilanen als Netzwerkbildner für $SiO_2$ und bestimmten organischen Polymeren, werden dessen einzelne Bestandteile zum Metalloxid bzw. zum organischen Polymer gerechnet.

Der Begriff "organisches Polymer" bezeichnet dem Fachmann geläufige organische Polymere und Oligomere. Vorzugsweise ist hierbei jedoch kein Polyethylen eingeschlossen. Ferner wird bei weiteren Ausführungsformen vorzugsweise unter dem erfindungsgemäßen Begriff "organisches Polymer" verstanden, dass der betreffende Stoff mindestens 10 Monomereinheiten, mehr bevorzugt mindestens 15 Monomereinheiten, noch mehr bevorzugt mindestens 18 Monomereinheiten, aufweist.

Die vorliegende Erfindung betrifft eine spezifische Beschichtung, bei der die Bestandteile einer anorganisch/organischen Beschichtungsschicht derart ausgewählt werden, dass besonders vorteilhafte Effekte erzielt werden. Insbesondere erwies es sich als vorteilhaft die Metalloxidmenge bezogen auf die Gesamtmenge der Beschichtung ohne Nachbeschichtung und Polymermenge der anorganisch/organischen Hybridschicht auf die Oberfläche des zu beschichtenden Objektes abzustimmen. Hiermit konnten beispielsweise bedeutend höhere Stabilitäten erzielt werden. Die Metallpigmente der vorliegenden Erfindung sind daher gekennzeichnet durch mindestens eine anorganisch/organische Hybridschicht, wobei das Verhältnis der Menge an Metalloxid der Beschichtung zur spezifischen Oberfläche des unbeschichteten Metallpigments in einem Bereich von 16,1 mg/m$^2$ bis 25 mg/m$^2$ liegt und

das Verhältnis der Menge organischem Polymer der anorganisch/organischen Hybridschicht zur spezifischen Oberfläche des unbeschichteten Metallpigments in einem Bereich von 3,9 mg/m$^2$ bis 10,1 mg/m$^2$ liegt.

Bei weiteren Ausführungsformen ist es ferner bevorzugt, dass das Verhältnis der Menge an Metalloxid der Beschichtung zur spezifischen Oberfläche des unbeschichteten Metallpigments mindestens 17,2 mg/m$^2$, mehr bevorzugt

mindestens 17,9 mg/m$^2$, beträgt.

Ferner ist es bei weiteren Ausführungsformen bevorzugt, dass das Verhältnis der Menge an Metalloxid der Beschichtung zur spezifischen Oberfläche des unbeschichteten Metallpigments höchstens 23 mg/m$^2$, mehr bevorzugt höchstens 22,3 mg/m$^2$, beträgt.

Insbesondere ist es bei weiteren Ausführungsformen bevorzugt, dass das Verhältnis der Menge an Metalloxid der Beschichtung zur spezifischen Oberfläche des unbeschichteten Metallpigments in einem Bereich von 17,2 mg/m$^2$ bis 23 mg/m$^2$, mehr bevorzugt in einem Bereich von 17,9 mg/m$^2$ bis 22,3 mg/m$^2$, liegt.

Vorzugsweise stellt das Metalloxid der anorganisch/organischen Hybridschicht kein Oxidationsprodukt des Metallpigments dar. So weisen die meisten Metallpigmente typischerweise eine sehr dünne oberflächliche Metalloxidschicht auf. Ferner kann durch eine gezielte oberflächliche Oxidation ein besonderer Effekt, meist hinsichtlich der Farbe, hervorgerufen werden, siehe beispielsweise WO 1996/038505 A1 und WO 2012/130680 A1.

Ferner ist es bevorzugt, dass die anorganisch/organische Hybridschicht als eine das metallische Substrat umhüllende Beschichtung ausgebildet ist.

Die Mindestdicke der anorganisch/organische Hybridschicht beträgt bei weiteren Ausführungsformen ferner vorzugsweise mindestens 9 nm, mehr bevorzugt mindestens 12 nm, noch mehr bevorzugt mindestens 15 nm. So zeigte sich, dass dünnere anorganisch/organische Hybridschichten beispielsweise eine geringere Zuverlässigkeit hinsichtlich der gewünschten Stabilität aufwiesen. Es wird vermutet, dass beim Einsatz einer zu dünnen Beschichtungsschicht zumindest vereinzelt infolge von Schwankungen in der Produktion keine vollständig umhüllende Beschichtungsschicht bei einigen Pigmenten erreicht wird.

Ferner ist es bei weiteren Ausführungsformen bevorzugt, dass die Dicke der anorganisch/organischen Hybridschicht höchstens 75 nm, mehr bevorzugt höchstens 64 nm, noch mehr bevorzugt höchstens 52 nm, beträgt. Die Bestimmung der Dicke der anorganisch/organischen Hybridschicht erfolgt mittels dem Fachmann geläufiger Verfahren wie beispielsweise Rasterelektronenmikroskopie. Insbesondere wurden bei dickeren anorganisch/organischen Hybridschichten Verschlechterungen der optischen Eigenschaften beobachtet. Es wird vermutet, dass sich hierbei unter anderem eine verstärkte Neigung zur Agglomeration ausbildet, wodurch teilweise die Verschlechterung der optischen Effekte erklärbar wird.

Bei weiteren Ausführungsformen liegt die Dicke der anorganisch/organischen Hybridschicht vorteilhafterweise im Bereich von 9 nm bis 75 nm, mehr bevorzugt im Bereich von 12 nm bis 64 nm, noch mehr bevorzugt im Bereich von 15 nm bis 52 nm.

Bei weiteren Ausführungsformen ist es ferner bevorzugt, dass die Menge der anorganisch/organischen Hybridschicht mindestens 5 Gew.-%, mehr bevorzugt mindestens 6 Gew.-%, noch mehr bevorzugt mindestens 8 Gew.-%, beträgt, jeweils bezogen auf das Gesamtgewicht des Pigments.

Ferner ist es bei weiteren Ausführungsformen bevorzugt, dass die Menge der anorganisch/organischen Hybridschicht höchstens 25 Gew.-%, mehr bevorzugt höchstens 21 Gew.-%, noch mehr bevorzugt höchstens 18 Gew.-%, beträgt, jeweils bezogen auf das Gesamtgewicht des Pigments.

Insbesondere ist es bei weiteren Ausführungsformen bevorzugt, dass die Menge der anorganisch/organischen Hybridschicht im Bereich von 5 Gew.-% bis 25 Gew.-%, mehr bevorzugt im Bereich von 6 Gew.-% bis 21 Gew.-%, noch mehr bevorzugt im Bereich von 8 Gew.-% bis 18 Gew.-%, liegt, jeweils bezogen auf das Gesamtgewicht des Pigments.

Ferner ist es bei weiteren Varianten der vorliegenden Erfindung bevorzugt, dass mindestens einer der Netzwerkbildner eine Struktur gemäß Formel (NI) oder (NII) aufweist,

$$R^{an1}{}_{xn1}R^{bn1}{}_{yn1}SiX_{(4-xn1-yn1)} \qquad (NI)$$

$$(R^{an1}O)_{xn2}(R^{bn1}O)_{yn2}MX_{(zn2-xn2-yn2)} \qquad (NII)$$

wobei die X unabhängig voneinander ausgewählt werden aus hydrolysierbaren Gruppen, nach deren Hydrolyse eine kovalente Bindung von organischem Netzwerkbildner mit dem anorganischen Netzwerk ausgebildet werden kann,

die R$^{an1}$ unabhängig voneinander ausgewählt werden aus reaktiven organischen Gruppen, die mit dem organischen Polymer kovalent verbindbar sind,

die R$^{bn1}$ unabhängig voneinander ausgewählt werden aus organischen Gruppen, die mit dem organischen Polymer kovalent verbindbar sein können,

M ausgewählt wird aus der Gruppe bestehend aus Al, Zr und Ti,

xn1 eine ganze Zahl von 1 bis 3 ist, yn1 eine ganze Zahl von 0 bis (3-xn1) ist,

zn2 die formale Oxidationszahl von M ist, xn2 eine ganze Zahl von 1 bis (zn2-1) ist,

yn2 eine ganze Zahl von 0 bis (zn2-2) ist, und

xn2+yn2 $\leq$ zn2-1 ist.

Vorzugsweise werden bei den Netzwerkbildnern gemäß Formel (NI) und (NII) die X unabhängig voneinander ausgewählt aus der Gruppe bestehend aus Halogengruppen, wie Bromid, Chlorid und Iodid, der Hydroxygruppe und C1-C20 Alkoxygruppen, welche auch Heteroatome, vorzugsweise O, S und/oder N, in der Kohlenstoffkette aufweisen können. Vorzugsweise werden die X unabhängig voneinander ausgewählt aus der Gruppe bestehend aus Halogengruppen, der Hydroxygruppe und C1-C4 Alkoxygruppen ohne Heteroatome in der Kohlenstoffkette, mehr bevorzugt aus C1-C4 Alkoxygruppen ohne Heteroatome in der Kohlenstoffkette. Beispiele für C1-C4 Alkoxygruppen ohne Heteroatome in der Kohlenstoffkette sind die Methoxygruppe, Ethoxygruppe, Propoxygruppe und n-Butoxygruppe.

Ferner werden bei weiteren Ausführungsformen die $R^{an1}$ vorzugsweise ausgewählt aus der Gruppe bestehend aus C1-C40 Alkylgruppen, C2-C40 Alkenylgruppen, C2-C40 Alkinylgruppen, C6-C36-Arylgruppen, teilfluorierte C6-C36 Arylgruppen, C7-C40 Alkylarylgruppen, C7-C40 Arylalkylgruppen, C8-C40 Alkenylarylgruppen, C8-C40 Arylalkenylgruppen, C8-C40 Arylalkinylgruppen, C8-C40 Alkinylarylgruppen, C5-C40 Cycloalkylgruppen, C6-C40 Alkylcycloalkylgruppen und C6-C40 Cycloalkylalkylgruppen, mehr bevorzugt aus der Gruppe bestehend aus C1-C26 Alkylgruppen, C2-C26 Alkenylgruppen, C2-C26 Alkinylgruppen, C6-C30-Arylgruppen, C7-C31 Alkylarylgruppen, C7-C31 Arylalkylgruppen, C8-C32 Alkenylarylgruppen, C5-C20 Cycloalkylgruppen, C6-C21 Alkylcycloalkylgruppen und C6-C21 Cycloalkylalkylgruppen, noch mehr bevorzugt aus der Gruppe bestehend aus C1-C10 Alkylgruppen, C2-10 Alkenylgruppen, C6-C10 Arylgruppen, C7-C11 Alkylarylgruppen und C7-C11 Arylalkylgruppen, wobei die vorgenannten $R^{an1}$ substituiert oder unsubstituiert sein können. Da $R^{an1}$ eine reaktive organische Gruppe darstellt, die mit dem organischen Polymer kovalent verbindbar ist, müssen unreaktive Gruppen der vorgenannten Liste wie beispielsweise C1-C40-Alkygruppen funktionelle Substituenten tragen. Besonders bevorzugt handelt es sich bei $R^{an1}$ um substituierte C1-C10 Alkylgruppen.

Bei weiteren Ausführungsformen werden die gegebenenfalls vorhandenen Substituenten von $R^{an1}$ vorzugsweise ausgewählt aus der Gruppe bestehend aus Aminogruppen, Hydroxygruppe, Thiolgruppen, Epoxygruppen, Acrylatgruppen, Methacrylatgruppen, Vinylgruppen, Allylgruppen, Carboxygruppen, Carboxylanhydridgruppen, Isocyanatgruppen, Cyanatgruppen, Ureidogruppen, Carbamatgruppen und Mischungen davon, mehr bevorzugt aus der Gruppe bestehend aus Aminogruppen, Hydroxygruppe, Acrylatgruppen, Methacrylatgruppen und Mischungen davon, noch mehr bevorzugt aus der Gruppe bestehend aus Acrylatgruppen, Methacrylatgruppen und Mischungen davon. Ein Beispiel für eine entsprechend substituierte $R^{an1}$-Gruppe ist die 3-Methacryloxypropylgruppe.

Bei weiteren Ausführungsformen werden die $R^{bn1}$ unabhängig voneinander ausgewählt aus der Gruppe bestehend aus H-, C1-C40 Alkylgruppen, C2-C40 Alkenylgruppen, C2-C40 Alkinylgruppen, C6-C36-Arylgruppen, fluorierte C6-C36 Arylgruppen, teilfluorierte C6-C36 Arylgruppen, C7-C40 Alkylarylgruppen, C7-C40 Arylalkylgruppen, C8-C40 Alkenylarylgruppen, C8-C40 Arylalkenylgruppen, C8-C40 Arylalkinylgruppen, C8-C40 Alkinylarylgruppen, C5-C40 Cycloalkylgruppen, C6-C40 Alkylcycloalkylgruppen und C6-C40 Cycloalkylalkylgruppen, mehr bevorzugt aus der Gruppe bestehend aus C1-C24 Alkylgruppen, C2-C24 Alkenylgruppen, C2-C24 Alkinylgruppen, C6-C24 Arylgruppen, fluorierte C6-C24 Arylgruppen, teilfluorierte C6-C24 Arylgruppen, C7-C30 Alkylarylgruppen, C7-C30 Arylalkylgruppen, C8-C30 Alkenylarylgruppen, C8-C30 Arylalkenylgruppen, C8-C30 Arylalkinylgruppen, C8-C30 Alkinylarylgruppen, C5-C20 Cycloalkylgruppen, C6-C25 Alkylcycloalkylgruppen, noch mehr bevorzugt aus der Gruppe bestehend aus C1-C24 Alkylgruppen, C2-C24 Alkenylgruppen, C6-C18 Arylgruppen, C7-C24 Alkylarylgruppen, C7-C24 Arylalkylgruppen, C5-C16 Cycloalkylgruppen, C6-C20 Alkylcycloalkylgruppen, wobei die vorgenannten Gruppen unsubstituiert sind und in den Kohlenstoffketten und Kohlenstoffringsystemen gegebenenfalls enthaltene Heteroatome ausgewählt werden aus der Gruppe bestehend aus O, N und S. Vorzugsweise enthalten die vorgenannten Gruppen keine Heteroatome in der Kette oder im Ring.

Insbesondere ist es bei weiteren Ausführungsformen bevorzugt, dass der Netzwerkbildner eine Struktur gemäß Formel (NI) oder (NII) aufweist,

$$Ra^{n1}_{xn1}R^{bn1}_{yn1}SiX_{(4-xn1-yn1)} \qquad (NI)$$

$$(R^{an1}O)_{xn2}(R^{bn1}O)_{yn2}MX_{(zn2-xn2-yn2)} \qquad (NII)$$

wobei die X unabhängig voneinander ausgewählt werden aus der Gruppe bestehend aus Halogengruppen, der Hydroxygruppe und C1-C4 Alkoxygruppen ohne Heteroatome in der Kohlenstoffkette,
die $R^{an1}$ unabhängig voneinander ausgewählt werden aus der Gruppe bestehend aus C1-C26 Alkylgruppen, C2-C26 Alkenylgruppen, C2-C26 Alkinylgruppen, C6-C30-Arylgruppen, C7-C31 Alkylarylgruppen, C7-C31 Arylalkylgruppen, C8-C32 Alkenylarylgruppen, C5-C20 Cycloalkylgruppen, C6-C21 Alkylcycloalkylgruppen und C6-C21 Cycloalkylalkylgruppen, wobei gegebenenfalls vorhandene Substituenten ausgewählt werden aus der Gruppe bestehend aus Aminogruppen, Hydroxygruppe, Thiolgruppen, Epoxygruppen, Acrylatgruppen, Methacrylatgruppen, Vinylgruppen, Allylgruppen, Carboxygruppen, Carboxylanhydridgruppen, Isocyanatgruppen, Cyanatgruppen, Ureidogruppen, Carbamatgruppen und Mischungen davon,
die $R^{bn1}$ unabhängig voneinander ausgewählt werden aus der Gruppe bestehend aus C1-C24 Alkylgruppen, C2-C24 Alkenylgruppen, C2-C24 Alkinylgruppen, C6-C24 Arylgruppen, fluorierte C6-C24 Arylgruppen, teilfluorierte C6-C24 Arylgruppen, C7-C30 Alkylarylgruppen, C7-C30 Arylalkylgruppen, C8-C30 Alkenylarylgruppen, C8-C30 Arylalkenylgruppen, C8-C30 Arylalkinylgruppen, C8-C30 Alkinylarylgruppen, C5-C20 Cycloalkylgruppen und C6-C25 Alkylcycloalkylgruppen, noch mehr bevorzugt aus der Gruppe bestehend aus C1-C24 Alkylgruppen, C2-C24 Alkenylgruppen, C6-C18 Arylgruppen, C7-C24 Alkylarylgruppen, C7-C24 Arylalkylgruppen, C5-C16 Cycloalkylgruppen und C6-C20 Alkylcycloalkylgruppen, wobei die vorgenannten Gruppen unsubstituiert sind und in den Kohlenstoffketten und Kohlenstoffringsystemen gegebenenfalls enthaltene Heteroatome ausgewählt werden aus der Gruppe bestehend aus O, N und S,

M ausgewählt wird aus der Gruppe bestehend aus Al, Zr und Ti,

$xn1$ eine ganze Zahl von 1 bis 3 ist, $yn1$ eine ganze Zahl von 0 bis (3-$xn1$) ist,

$zn2$ die formale Oxidationszahl von M ist, $xn2$ eine ganze Zahl von 1 bis ($zn2$-1) ist, $yn2$ eine ganze Zahl von 0 bis ($zn2$-2) ist, und

$xn2+yn2 \leq zn2-1$ ist.

Als besonders vorteilhaft hat es sich erwiesen, wenn die anorganisch/organische Hybridschicht mindestens einen Netzwerkbildner umfasst, der ausgewählt wird aus der Gruppe der Netzwerkbildnern gemäß Formel (NI). Insbesondere ist es hierbei bevorzugt, dass mindestens Netzwerkbildner ausgewählt aus der Gruppe der Netzwerkbildnern Formel (NI), wobei die X unabhängig voneinander ausgewählt werden aus der Gruppe bestehend aus C1-C4 Alkoxygruppen ohne Heteroatome in der Kohlenstoffkette,

die $R^{an1}$ unabhängig voneinander ausgewählt werden aus der Gruppe bestehend aus substituierten C1-C10 Alkylgruppen, wobei die Substituenten ausgewählt werden aus der Gruppe bestehend aus Acrylatgruppen, Methacrylatgruppen und Mischungen hiervon ,

die $R^{bn1}$ unabhängig voneinander ausgewählt werden aus der Gruppe bestehend aus C1-C24 Alkylgruppen, C6-C18 Arylgruppen, C7-C24 Alkylarylgruppen, C7-C24 Arylalkylgruppen, C5-C16 Cycloalkylgruppen und C6-C20 Alkylcycloalkylgruppen, wobei die vorgenannten Gruppen unsubstituiert sind und in den Kohlenstoffketten und Kohlenstoffringsystemen keine Heteroatome aufweisen,

$xn1$ eine ganze Zahl von 1 bis 3 ist und

$yn1$ 0 oder 1 ist.

Als Netzwerkbildner geeignete organofunktionelle Silane können beispielsweise von der Fa. Evonik erworben werden. Beispiele sind 3-Methacryloxypropyltrimethoxysilan (Dynasylan MEMO), Vinyltri(m)ethoxysilan (Dynasylan VTMO bzw. VTEO), 3-Mercaptopropyltri(m)ethoxysilan (Dynasylan MTMO oder 3201), Aminopropyltrimethoxysilan (Dynasylan AMMO) oder 2-Aminoethyl-3-aminopropyltrimethoxy-silan (Dynasylan DAMO) und 3-Glycidoxy-propyltrimethoxysilan (Dynasylan GLYMO). Eine besonders geeignete Gruppe der Netzwerkbildner sind die 3-Methacryloxypropyltrialkoxysilane.

Bei weiteren Ausführungsformen ist es ferner bevorzugt, dass $R^{an1}$ mindestens eine Funktionalität aufweist, die dem organischen Polymer der anorganisch/organischen Hybridschicht entspricht. Soll das organische Polymer beispielsweise durch die Polymerisation von olefinischen Monomeren erhalten werden oder ein vorpolymerisiertes organisches Polymer über darin enthaltene olefinische Gruppen an den Netzwerkbildner gebunden werden, so sind Netzwerkbildner mit einer olefinischen Gruppe bevorzugt. Insbesondere ist es bevorzugt, dass ein Teil des Netzwerkbildner als Teil der Polymerkette des organischen Polymers betrachtet werden kann, so dass beispielsweise mindestens eine (Meth)acrylatkette des organischen Polymers über (Meth)acrylatgruppen an $R^{an1}$ gebunden werden.

Es hat sich als besonders vorteilhaft erwiesen, wenn der Netzwerkbildner mindestens zwei Funktionalitäten aufweist, wobei sich mindestens eine Funktionalität in das organische Polymer einbindet und sich mindestens eine Funktionalität in das anorganische Netzwerk einbindet.

Weitere Beispiele für organofunktionelle Silane mit beispielsweise Vinyl- bzw. (Meth)acrylatfunktionalitäten sind: Isocyanatotriethoxysilan, 3-Isocyanatopropoxyltriethoxysilan, Vinylethyldichlorsilan, Vinylmethyldichlorsilan, Vinylmethyldiacetoxysilan, Vinylmethyldiethoxysilan, Vinyltriacetoxysilan, Vinyltrichlorsilan, Phenylvinyldiethoxysilan, Phenylallyldiethoxy-silan, Phenylallyldichlorsilan, 3-Methacryloxypropyltriethoxysilan, Methacryloxypropyltrimethoxysilan, 3-Acryloxypropyltrimethoxysilan, 2-Methacryloxyethyltri(m)ethoxysilan, 2-Acryloxyethyltri(m)ethoxysilan, 3-Methacryloxypropyltris(methoxy-ethoxy)silan, 3-Methacryloxypropyltris(butoxyethoxy)silan, 3-Methacryloxypropyltris(propoxy)silan, 3-Methacryloxypropyltris(butoxy)silan.

Weitere als Netzwerkbildner geeignete Stoffe sind Titanate, Zirkonate oder Aluminate, wie sie beispielsweise von der Firma Kenrich Petrochemicals hergestellt und unter dem Handelsnamen Ken-React angeboten werden.

Bei weiteren Ausführungsformen der vorliegenden Erfindung beträgt das Verhältnis der Stoffmengen in Mol von Netzwerkbildner zu der Zahl der Monomere des organischen Polymers der anorganische/organischen Hybridschicht mindestens 1:10, mehr bevorzugt mindestens 1,2:10, noch mehr bevorzugt mindestens 1,4:10.

Ferner ist es bei weiteren Ausführungsformen bevorzugt, dass das Verhältnis der Stoffmengen in Mol von Netzwerkbildner zu der Zahl der Monomere des organischen Polymers der anorganische/organischen Hybridschicht höchstens 5:10, mehr bevorzugt höchstens 4:10, noch mehr bevorzugt höchstens 3,5:10 beträgt.

Insbesondere ist es bei weiteren Ausführungsformen der vorliegenden Erfindung bevorzugt, dass das Verhältnis der Stoffmengen in Mol von Netzwerkbildner zu der Zahl der Monomere des organischen Polymers der anorganische/organischen Hybridschicht im Bereich von 1:10 bis 5:10, mehr bevorzugt im Bereich von 1,2:10 bis 4:10, noch mehr bevorzugt im Bereich von 1,4:10 bis 3,5:10, liegt.

Weitere Varianten der vorliegenden Erfindung sind ferner dadurch gekennzeichnet, dass das organische Polymer vorzugsweise im Wesentlichen aus Monomeren besteht, die mindestens eine Funktionalität aufweisen oder vor der Polymerisation aufgewiesen haben, die ausgewählt wird aus der Gruppe bestehend aus Aminogruppen, Thiolgruppen, Epoxygruppen, Acrylatgruppen, Methacrylatgruppen, Vinylgruppen, Allylgruppen, Alkenylgruppen, Alkinylgruppen, Carboxygruppen, Carboxylanhydridgruppen, Isocyanatgruppen, Cyanatgruppen, Ureidogruppen und Carbamatgruppen, mehr bevorzugt aus der Gruppe bestehend aus Acrylatgruppen, Methacrylatgruppen, Hydroxygruppen, Carboxygruppen und Carboxylanhydridgruppen, noch mehr bevorzugt aus der Gruppe bestehend aus Acrylatgruppen und Methacrylatgruppen. Vorzugsweise besteht das organische Polymer zu mindestens 99 Gew.-%, mehr bevorzugt vollständig aus solchen Monomeren. Insbesondere ist es bevorzugt, dass die vorgenannten reaktiven Gruppen im Wesentlichen während der Polymerisation abreagiert sind.

Bei weiteren Ausführungsformen wird das organische Polymer vorzugsweise ausgewählt aus der Gruppe bestehend aus Polyacrylaten, Polymethacrylaten, Polyethern, Polyestern, Polyaminen, Polyamiden, Polyolen, Polyurethanen und Polyolefinen, wobei die Polyolefine kein Polyethylen umfassen, mehr bevorzugt aus der Gruppe bestehend aus Polyacrylaten, Polymethacrylaten, Polyethern und Polyestern, noch mehr bevorzugt aus der Gruppe bestehend aus Polyacrylaten und Polymethacrylaten.

Vorzugsweise ist das organische Polymer über die funktionellen Gruppe $R^{an1}$ eines oder mehrerer organischer Netzwerkbildner in der anorganisch/organischen Hybridschicht kovalent gebunden.

Bei weiteren Ausführungsformen ist es bevorzugt, dass das Verhältnis der Menge an organischem Polymer der anorganisch/organischen Hybridschicht zur spezifischen Oberfläche des unbeschichteten Metallpigments mindestens 4,6 mg/m$^2$, mehr bevorzugt mindestens 5,1 mg/m$^2$, beträgt.

Ferner ist es bei weiteren Ausführungsformen bevorzugt, dass das Verhältnis der Menge an organischem Polymer der anorganisch/organischen Hybridschicht zur spezifischen Oberfläche des unbeschichteten Metallpigment höchstens 9,7 mg/m$^2$, mehr bevorzugt höchstens 9,5 mg/m$^2$, beträgt.

Insbesondere ist es bei weiteren Ausführungsformen bevorzugt, dass das Verhältnis der Menge an organischem Polymer der anorganisch/organischen Hybridschicht zur spezifischen Oberfläche des unbeschichteten Metallpigment in einem Bereich von 4,6 mg/m$^2$ bis 9,7 mg/m$^2$, mehr bevorzugt in einem Bereich von 5,1 mg/m$^2$ bis 9,5 mg/m$^2$, liegt.

Ferner hat ich gezeigt, dass vorzugsweise ein bestimmtes Gewichtsverhältnis der Metalloxidmenge der Beschichtung zur organischen Polymermenge der anorganisch/organischen Hybridschicht vorliegt. Bei weiteren Ausführungsformen beträgt das Gewichtsverhältnis der Metalloxidmenge der Beschichtung zur organischen Polymermenge der anorganisch/organischen Hybridschicht mindestens 2,5 : 1, mehr bevorzugt mindestens 3,0 : 1, noch mehr bevorzugt mindestens 3,3 : 1.

Ferner ist es bei weiteren Ausführungsformen bevorzugt, dass das Gewichtsverhältnis der Metalloxidmenge der Beschichtung zur organischen Polymermenge der anorganisch/organischen Hybridschicht höchstens 9,0 : 1, mehr bevorzugt höchstens 8,1 : 1, noch mehr bevorzugt höchstens 6,7 : 1, beträgt.

Insbesondere ist es bei weiteren Ausführungsformen bevorzugt, dass das Gewichtsverhältnis der Metalloxidmenge der Beschichtung zur organischen Polymermenge der anorganisch/organischen Hybridschicht in einem Bereich von 2,5 : 1 bis 9,0 : 1, mehr bevorzugt in einem Bereich von 3,0 : 1 bis 8,1 : 1, noch mehr bevorzugt in einem Bereich von 3,3 : 1 bis 6,7 : 1, liegt.

Gemäß weiteren Varianten der vorliegenden Erfindung ist es bevorzugt, dass die Metalloxide der anorganisch/organischen Hybridschicht im Wesentlichen, mehr bevorzugt vollständig, ausgewählt werden aus der Gruppe bestehend aus Siliciumoxid, Aluminiumoxid, Boroxid, Zirkoniumoxid, Ceroxid, Eisenoxid, Titanoxid, Chromoxid, Zinnoxid, Molybdänoxid, Vanadiumoxid, Zinkoxid, Magnesiumoxid und Mischungen davon, mehr bevorzugt aus der Gruppe bestehend aus Siliciumoxid, Aluminiumoxid, Eisenoxid, und Mischungen davon, wobei die vorgenannten Metalloxide auch deren Hydroxide und Oxidhydrate umfassen. Besonders bevorzugt werden die erfindungsgemäß aufzubringenden Metalloxide im Wesentlichen, vorzugsweise vollständig, ausgewählt aus der Gruppe bestehend aus Siliciumoxid, Aluminiumoxid und Mischungen davon, wobei die vorgenannten Metalloxide auch deren Oxidhydrate und Hydroxide umfassen. Insbesondere hat sich die Verwendung von Siliciumoxiden wie Silciumdioxid, Siliciumhydroxid, Siliciumoxidhydrat und Mischungen hiervon als vorteilhaft erwiesen. Ferner ist es bei weiteren Ausführungsformen bevorzugt, dass mindestens 90 Gew.-%, mehr bevorzugt mindestens 97 Gew.-% der Metalloxide der anorganisch/organischen Hybridschicht aus den vorgenannten Metalloxiden ausgewählt werden.

Der Begriff "Metalloxidschicht" beziehungsweise "Metalloxid" im Sinne der vorliegenden Erfindung umfasst, soweit nicht anderweitig spezifiziert, auch oxidierte Formen der Metalle und Halbmetalle wie Hydroxide und Oxidhydrate.

Typischerweise hat es sich als vorteilhaft erwiesen, wenn der Gehalt an Metalloxid in der Beschichtung mindestens 50 Gew.-%, vorzugsweise mindestens 56 Gew.-%, noch mehr bevorzugt mindestens 61 Gew.-%, beträgt, jeweils bezogen auf das Gesamtgewicht der Beschichtung.

Ferner ist es bei weiteren Ausführungsformen bevorzugt, dass der Gehalt an Metalloxid in der Beschichtung höchstens 86 Gew.-%, vorzugsweise höchstens 82 Gew.-%, noch mehr bevorzugt höchstens 79 Gew.-%, beträgt, jeweils bezogen auf das Gesamtgewicht der Beschichtung.

Insbesondere ist es bei weiteren Ausführungsformen bevorzugt, dass der Gehalt an Metalloxid in der Beschichtung in einem Bereich von 50 Gew.-% bis 86 Gew.-%, vorzugsweise in einem Bereich von 56 Gew.-% bis 82 Gew.-%, noch mehr bevorzugt in einem Bereich von 61 Gew.-% bis 79 Gew.-%, liegt, jeweils bezogen auf das Gesamtgewicht der Beschichtung.

Bei weiteren Ausführungsformen ist es ferner bevorzugt, dass die spezifizierten Bestandteile der anorganisch/organischen Hybridschicht, das heißt das mindestens eine Metalloxid, das mindestens eine organische Polymer und der mindestens eine Netzwerkbildner, mindestens 80 Gew.-%, mehr bevorzugt mindestens 90 Gew.-%, noch mehr bevorzugt mindestens 97 Gew.-%, der anorganisch/organischen Hybridschicht darstellen, bezogen auf das Gesamtgewicht der anorganisch/organischen Hybridschicht.

Ferner ist es bei weiteren Ausführungsformen bevorzugt, dass die Gesamtmenge sowohl des mindestens einen Metalloxids als auch die Gesamtmenge des mindestens einen organischen Polymers in der anorganisch/organischen Hybridschicht jeweils mindestens 10 Gew.-%, mehr bevorzugt mindestens 20 Gew.-%, beträgt, jeweils bezogen auf das Gesamtgewicht der anorganisch/organischen Hybridschicht. So zeigte sich bei zu geringen Mengen an Metalloxid oder organischen Polymer die vorteilhaften Eigenschaften der anorganisch/organischen Hybridschicht deutlich schwächer ausgeprägt waren.

Es zeigte sich weiterhin, dass zu geringe Mengen an Netzwerkbildner in einer merklichen Verschlechterung der Schutzwirkung der anorganisch/organischen Hybridschicht resultieren. Bei weiteren Ausführungsformen beträgt die Gesamtmenge der Netzwerkbildner in der anorganisch/organischen Hybridschicht vorzugsweise mindestens 1,5 Gew.-%, mehr bevorzugt mindestens 1,8 Gew.-%, noch mehr bevorzugt höchstens 2,3 Gew.-%, jeweils bezogen auf das Gesamtgewicht der anorganisch/organischen Hybridschicht.

Weiterhin zeigte sich, dass zu große Mengen an Netzwerkbildner entweder keine Verbesserung mehr erzielten oder sogar in einer Verschlechterung der optischen Eigenschaften resultieren können. Bei weiteren Ausführungsformen beträgt die Gesamtmenge der Netzwerkbildner in der anorganisch/organischen Hybridschicht vorzugsweise höchstens 9,2 Gew.-%, mehr bevorzugt höchstens 8,5 Gew.-%, noch mehr bevorzugt höchstens 7,9 Gew.-%, jeweils bezogen auf das Gesamtgewicht der anorganisch/organischen Hybridschicht.

Insbesondere ist es bei weiteren Ausführungsformen bevorzugt, dass die Gesamtmenge der Netzwerkbildner in der anorganisch/organischen Hybridschicht im Bereich von 1,5 Gew.-% bis 9,2 Gew.-%, mehr bevorzugt im Bereich von 1,8 Gew.-% bis 8,5 Gew.-%, noch mehr bevorzugt im Bereich von 2,3 Gew.-% bis 7,9 Gew.-%, liegt, jeweils bezogen auf das Gesamtgewicht der anorganisch/organischen Hybridschicht.

Ferner können die erfindungsgemäßen Pigmente bei weiteren Varianten der vorliegenden Erfindung neben der anorganisch/organischen Hybridschicht weitere Beschichtungsschichten umfassen. Dies ermöglicht die weitere Anpassung der Produkteigenschaften. Hiermit können die Eigenschaften weiter beeinflusst werden. Zur Vereinfachung des Produktionsprozesses ist es bei weiteren Ausführungsformen hingegen bevorzugt, dass die erfindungsgemäße Beschichtung lediglich die anorganisch/organische Hybridschicht sowie optional im Wesentlichen reine Metalloxidbeschichtungen und Nachbeschichtungen umfasst. Zur Nachbeschichtung werden vorzugsweise monomere und polymere Silane eingesetzt, insbesonders bevorzugt vorkondensierte Heteropolysiloxane. Beispiele für als Nachbeschichtung einsetzbare Silane sind Dynasylan 9116, Dynasylan OCTEO, Dynasylan AMMO und Dynasylan DAMO.

Bei weiteren Ausführungsformen ist auf der anorganisch/organischen Hybridschicht mindestens eine diskrete Beschichtungsschicht angeordnet, die eine im Wesentlichen rein anorganische Schicht darstellt oder im Wesentlichen aus organischem Polymer besteht.

Zur weiteren Modifizierung der Pigmente können beispielsweise weitere Beschichtungsschichten aufgebracht werden, welche im Wesentlichen aus Metalloxid bestehen. Im Rahmen dieser Erfindung wird unter dem Begriff "im Wesentlichen bestehend aus Metalloxid" oder beispielsweise unter "im Wesentlichen bestehend aus Siliciumoxid, Siliciumhydroxid, Siliciumoxidhydrat oder deren Mischungen" verstanden, dass die Schicht überwiegend, vorzugsweise zu mindestens 90 Gew.-%, mehr bevorzugt zu mindestens 95 Gew.-%, noch mehr bevorzugt zu mindestens 99 Gew.-%, aus Metalloxid beziehungsweise aus Siliciumoxid, Siliciumhydroxid, Siliciumoxidhydrat oder deren Mischungen besteht. Beispielsweise können betreffende Schichten, welche mittels des Sol-Gel-Verfahrens hergestellt und nicht calciniert wurden, noch Alkoxygruppen enthalten. Vorzugsweise handelt es sich bei der erfindungsgemäß aufzubringenden mindestens einen Beschichtungsschicht, umfassend mindestens ein Metalloxid, um (eine) nichtcalcinierte Beschichtung(en).

"Nichtcalciniert" im Sinne der vorliegenden Erfindung bedeutet, dass keine Erhitzung erfolgte, um eine im Wesentlichen vollständige Entfernung des in der durch Sol-Gel-Verfahren aufgebrachten Schicht vorhandenen Wassers zu bewirken. Dies kann beispielsweise durch ein Erhitzen über 400 °C erfolgen. Hiermit können beispielsweise Pigmente erhalten werden, deren "Wassergehalt" in der betreffenden Schicht weniger als 3 Gew.-% beträgt.

Bei weiteren Ausführungsformen weist das erfindungsgemäße Pigment mindestens eine Beschichtungsschicht auf, welche im Wesentlichen aus Metalloxid besteht, wobei das Metalloxid vorzugsweise ausgewählt wird aus der Gruppe bestehend aus Siliciumoxid, Titanoxid, Aluminiumoxid, Zirkoniumoxid, Eisenoxid, Kupferoxid, Zinnoxid, Kobaltoxid, Chromoxid, Ceroxid, Zinkoxid, Antimonoxid, Manganoxid, Nickeloxid, Yttriumoxid, Molybdänoxid, Vanadiumoxid, Tantaloxid, Wolframoxid und Mischungen davon, mehr bevorzugt aus der Gruppe bestehend aus Siliciumoxid, Titanoxid, Aluminiumoxid, Eisenoxid, Kupferoxid, Ceroxid und Mischungen davon, wobei die vorgenannten Metalloxide auch Hydroxide und Oxidhydrate umfassen. Vorzugsweise stellt die vorgenannte mindestens eine Beschichtungsschicht bestehend im Wesentlichen aus Metalloxid kein Oxidationsprodukt des metallischen Substrats dar.

Bei Pigmenten, bei denen der optische Effekt nicht durch Interferenzeffekte infolge der - Beschichtung beeinflusst werden soll, hat sich die Verwendung niedrigbrechender Metalloxide als vorteilhaft erwiesen. Dies betrifft vorzugsweise die metalloxidhaltigen Beschichtungsschichten, welche von der anorganisch/organischen Hybridschicht verschieden sind. Der Begriff "niedrigbrechend" im Sinne der vorliegenden Erfindung bedeutet, dass deren Brechungsindex höchstens 1,7 beträgt. Hierbei bezieht sich die Angabe auf den Brechungsindex des betreffenden Metalloxids in makroskopischer Form. Dieser kann mittels geläufiger Verfahren bestimmt werden, beispielsweise mittels eines Refraktometers. Bei weiteren Ausführungsformen werden daher die Metalloxide der Beschichtung zu mindestens 95 Gew.-%, vorzugsweise zu mindestens 99 Gew.-%, aus der Gruppe der niedrigbrechenden Metalloxide ausgewählt, jeweils bezogen auf das Gesamtgewicht der Beschichtung. Bei weiteren Ausführungsformen ist es bevorzugt, dass weniger als 10 Gew.-%, mehr bevorzugt weniger als 3 Gew.-%, noch mehr bevorzugt weniger als 1 Gew.-%, der in der Beschichtung enthaltenen Metalloxide keine niedrigbrechenden Metalloxide sind, bezogen auf das Gesamtgewicht der Metalloxide der Beschichtung. Ein besonders bevorzugtes niedrigbrechendes Metalloxid ist Siliciumoxid, umfassend insbesondere Siliciumdioxid, Siliciumhydroxid, Siliciumoxidhydrat und Mischungen hiervon.

Bei weiteren Ausführungsformen ist es ferner bevorzugt, dass sämtliche von der anorganisch/organischen Hybridschicht verschiedenen Beschichtungsschichten, welche im Wesentlichen aus Metalloxid bestehen und nicht aus einer Oxidation des Metalls des metallischen Substrats bzw. des Metallkerns entstanden sind, im Wesentlichen aus Siliciumoxid, Siliciumhydroxid, Siliciumoxidhydrat oder deren Mischungen bestehen.

Ferner ist es bei weiteren Ausführungsformen bevorzugt, dass das erfindungsgemäße Pigment mindestens eine Beschichtungsschicht bestehend im Wesentlichen aus organischen Polymer umfasst, wobei das Polymer dieser Beschichtungsschicht vorzugsweise im Wesentlichen oder vollständig ausgewählt wird aus der Gruppe bestehend aus Poly(meth)acrylat, Polyether, Polyester, Polyamin, Polyamid, Polyol, Polyurethan, Polyphenolformaldehyd, Polyolefin und Mischungen davon. Bei weiteren Ausführungsformen werden die Polymere aller Beschichtungsschichten bestehend im Wesentlichen aus organischen Polymer aus den vorgenannten Polymeren ausgewählt.

Bei weiteren Ausführungsformen ist es besonders bevorzugt, dass das erfindungsgemäße Pigment mindestens eines Beschichtungsschicht bestehend im Wesentlichen aus organischen Polymer umfasst, wobei das Polymer ein Poly(meth)acrylat ist. Der Begriff "Poly(meth)acrylat" bzw. "(Meth)acrylat" im Sinne der vorliegenden Erfindung bedeutet, dass es sich um Acrylate, Methacrylate oder Mischungen davon handelt.

Gemäß einer weiteren Variante der vorliegenden Erfindung werden spezifische metallische Substrate genutzt. So zeigte sich beispielsweise dass durch Kombination der erfindungsgemäßen Beschichtungen mit spezifischen metallischen Substraten besonders vorteilhafte Eigenschaften für gebräuchliche Anwendungen insbesondere hinsichtlich der Optik oder der Schutzeigenschaften erhalten werden können.

Bei weiteren Ausführungsformen ist es bevorzugt, dass die erfindungsgemäßen Pigmente, insbesondere die Metalleffektpigmente, einen mittleren Pigmentdurchmesser ($D_{50}$-Wert) in einem Bereich von 2 bis 66 $\mu$m, vorzugsweise in einem Bereich von 4 bis 50 $\mu$m, noch mehr bevorzugt in einem Bereich von 8 bis 47 $\mu$m, aufweisen.

Der D-Wert ist eine dem Fachmann gebräuchliche Größe zur Charakterisierung von derartigen metallischen Substraten. Die jeweilig angegebene Zahl gibt hierbei an, wieviel % der Partikel in einer volumengemittelten Partikelgrößenverteilung unterhalb einer angegebenen Größe liegen. Beispielsweise gibt der $D_{50}$-Wert an, unter welcher Größe 50% der Partikel liegen. Die Messungen erfolgen beispielsweise mittels Lasergranulometrie unter Einsatz eines Partikelgrößenanalysator der Fa. Quantachrome (Gerät: Cilas 1064). Die Messung erfolgt hierbei gemäß Herstellerangaben. Hierzu werden 1,5 g des pulverförmigen Beschichtungsmaterials oder Paste mit einem Feststoffgehalt von 1,5 g in ca. 100 ml Ethanol dispergiert, 300 Sekunden in einem Ultraschallbad (Gerät: Sonorex IK 52, Fa. Bandelin) behandelt und anschließend mittels einer Pasteurpipette in die Probenvorbereitungszelle des Messgerätes gegeben und mehrmals vermessen. Aus den einzelnen Messergebnissen werden die resultierenden Mittelwerte gebildet. Die Auswertung der Streulichtsignale erfolgt dabei nach der Fraunhofer Methode.

Ferner liegt der $D_{90}$-Wert vorzugsweise in einem Bereich von 10 bis 81 $\mu$m, mehr bevorzugt in einem Bereich von 16 bis 80 $\mu$m und noch mehr bevorzugt in einem Bereich von 21 bis 79 $\mu$m.

Zudem ist es mehr bevorzugt, dass der $D_{10}$-Wert in einem Bereich von 0,5 $\mu$m bis 34 $\mu$m, mehr bevorzugt in einem Bereich von 1 $\mu$m bis 29 $\mu$m und noch mehr bevorzugt in einem Bereich von 2 $\mu$m bis 27 $\mu$m liegt.

Insbesondere ist es bei Ausführungsformen der Erfindung bevorzugt, dass der $D_{50}$-Wert in einem Bereich von 2 $\mu$m bis 66 $\mu$m, der $D_{90}$-Wert in einem Bereich von 10 $\mu$m bis 81 $\mu$m und der $D_{10}$-Wert in einem Bereich von 0,5 $\mu$m

bis 34 μm liegt. Vorzugsweise liegt der $D_{50}$-Wert in einem Bereich von 4 μm bis 50 μm, der $D_{90}$-Wert in einem Bereich von 16 μm bis 80 μm und der $D_{10}$-Wert in einem Bereich von 1 μm bis 29 μm·

Ein weiteres Merkmal zur Charakterisierung der erfindungsgemäßen Pigmente, vorzugsweise Metalleffektpigmente, ist der Span $\Delta D$, der folgendermaßen definiert ist

$$\Delta D = (D_{90} - D_{10})/ D_{50}.$$

Vorzugsweise weisen die erfindungsgemäßen Pigmente, insbesondere die Metalleffektpigmente, einen Span in einem Bereich von 0,6 bis 2,1, vorzugsweise in einem Bereich von 0,7 bis 1,9 und noch mehr bevorzugt in einem Bereich von 0,75 bis 1,7, auf.

Ferner zeichnen sich die erfindungsgemäßen beschichteten Metalleffektpigmente, vorzugsweise Metalleffektpigmente, vorzugsweise durch eine bestimmte mittlere Dicke ($h_{50}$-Wert) aus. Die mittlere Dicke gibt den Wert an, bei dem 50% der Metallpigmente in einer Summenhäufigkeitsverteilung bezeichnet wird, die angegebene Dicke oder weniger haben, wobei mindestens 100 Pigmente, beispielsweise 100 Pigmente, vermessen wurden.

Die Präparation und die Vermessung der Pigmente zur Bestimmung der Dickenverteilung erfolgt nach der in der US 2007/0199478 A1 ([0129 - [0131]) beschriebenen Methode. Es werden dabei nur Pigmente mit einem azimuthalen Winkel von unter 10° gezählt. Die Bestimmung einschlägiger Kennwerte der Summenhäufigkeitsverteilung kann beispielsweise mittels eines Standardprogramms wie Excel (Quantil-Funktion) erfolgen.

Sollte das vorgenannte Verfahren zur Präparation der Pigmente nicht anwendbar sein, so kann alternativ beispielsweise eine Präparation im Lack erfolgen. Hierbei ist auf eine möglichst gute Orientierung der Plättchen im Anwendungsmedium zu achten. Anschließend wird der ausgehärtete Lack angeschliffen und der Querschliff im REM betrachtet. Für die Zählung werden nur Teilchen ausgewählt, die eine gute Orientierung aufweisen.

So ist es bei den erfindungsgemäßen beschichteten Metalleffektpigmenten bevorzugt, dass der $h_{50}$-Wert in einem Bereich von 15 nm bis 2 um, vorzugsweise in einem Bereich von 20 nm bis 1,5 μm, liegt. Insbesondere ist es bei weiteren Ausführungsformen bevorzugt, dass die erfindungsgemäßen beschichteten Metalleffektpigmente einen $h_{50}$-Wert in einem Bereich von 20 nm bis 370 nm, mehr bevorzugt in einem Bereich von 20 nm bis 240 nm, und besonders bevorzugt in einem Bereich von 15 bis 80 nm und ganz besonders bevorzugt in einem Bereich von 20 bis 50 nm aufweisen.

Zur Charakterisierung der erfindungsgemäß besonders bevorzugten Metalleffektpigmente kann beispielsweise das Aspektverhältnis dienen. Dieses ergibt sich gemäß folgender Formel

$$\text{Aspektverhältnis} = \frac{D_{50}}{h_{50}}.$$

So sind die erfindungsgemäßen beschichteten Metalleffektpigmente bei bevorzugten Ausführungsformen durch ein Aspektverhältnis in einem Bereich von 1500:1 bis 10:1, vorzugsweise in einem Bereich von 1200:1 bis 15:1, mehr bevorzugt in einem Bereich von 950:1 bis 25:1, gekennzeichnet.

Unter dem Begriff "metallische Substrate" werden im Sinne der vorliegenden Erfindung unbeschichtete metallhaltige Pigmente verstanden, welche höchstens eine dünne Oxidationsschicht aufweisen. Die metallischen Substrate sind vorteilhafterweise Metalleffektpigmente. Vorzugsweise handelt es sich hierbei um Metallpigmente, welche im Wesentlichen, vorzugsweise vollständig, aus mindestens einem Metall oder mindestens einer Metalllegierungen bestehen. Insbesondere handelt es sich bei den metallischen Substraten nicht um Nanopartikel oder Nanopartikelagglomerate. Unter dem Begriff "Nanopartikel" im Sinne der vorliegenden Erfindung werden Partikel mit einer mittleren Partikelgröße von weniger als 400 nm verstanden. Vorzugsweise handelt es sich bei den metallischen Substraten nicht um Partikel mit einer mittleren Partikelgröße von weniger als 500 nm oder deren Agglomerate. Die Bestimmung von derart kleinen Partikeln erfolgt beispielsweise mittels eines DelsaNano C Gerätes der Firma Beckman Coulter gemäß Herstellerangaben.

Bei Anwendungen, bei denen hohe metallische Glanzwerte ohne gravierende Farbänderungen durch Oxidation des metallischen Substrats gewünscht sind, ist es bevorzugt, dass das Metall der erfindungsgemäßen verwendeten metallischen Substrats zum Großteil in elementarer Metallform, mithin in nichtoxidierter Form, vorliegt. Bei weiteren Ausführungsformen beträgt daher der Sauerstoffgehalt des Metalls des metallischen Substrats höchstens 15 Gew.-%, vorzugsweise höchstens 12 Gew.-%, mehr bevorzugt höchstens 8 Gew.-%, noch mehr bevorzugt höchstens 5 Gew.-% und am meisten bevorzugt höchstens 3 Gew.-%, jeweils bezogen auf das Gewicht des Metalls des metallischen Substrats.

Sofern jedoch auf spezifische Farbtöne erwünscht sind, können diese ohne zusätzliche Farbpigmente oder in Ergänzung zu vorhandenen Farbpigmenten mittels Einfärbung durch eine gezielte Oxidation der Metallpigmente unter Erzeugung einer farbgebenden Oxidschicht erzielt werden.

Bei weiteren Ausführungsformen ist es bevorzugt, dass das Metall des metallischen Substrats weitgehend aus einem Metall besteht, das ausgewählt wird aus der Gruppe bestehend aus Aluminium, Kupfer, Eisen, Zink, Zinn, Titan, Edelstahl, Mischungen hiervon und Legierungen hiervon, mehr bevorzugt aus der Gruppe bestehend aus Aluminium, Eisen, Kupfer und Messing. Bestehend "weitgehend" aus einem Metall X oder einer Mischung der Metalle X bis Z bedeutet im Sinne der vorliegenden Erfindung, dass das Metall X oder die Mischung der Metalle X bis Z mindestens 60 Gew.-% darstellen, bezogen auf das Gewicht des Metalls des metallischen Substrats ohne Sauerstoff bzw. des Metallkerns ohne Sauerstoff. Vorzugsweise besteht das Metall des metallischen Substrats bzw. der Metallkern zu mindestens 95 Gew.-%, mehr bevorzugt zu mindestens 99 Gew.-% aus dem spezifizierten Metall oder den spezifizierten Metallen, jeweils bezogen auf das Gewicht des Metalls des metallischen Substrats ohne Sauerstoff bzw. des Metallkerns ohne Sauerstoff.

Bei weiteren Ausführungsformen ist es insbesondere bevorzugt, dass es sich bei dem metallischen Substrat um einen Metallkern handelt. Dieser Metallkern besteht vorzugsweise weitgehend aus einem Metall, das ausgewählt wird aus der Gruppe bestehend aus Aluminium, Kupfer, Eisen, Zink, Zinn, Titan, Chrom, Kobalt, Silber, Edelstahl, Nickel, Antimon, Magnesium, Zirkonium, Silicium und Bor und Mischungen sowie Legierungen davon. Im Sinne der vorliegenden Erfindung umfasst der Begriff "Metall" auch die Halbmetalle Silicium und Bor, insbesondere Silicium, welche vorzugsweise als Legierungsbestandteil eingesetzt werden. Vorzugsweise stellen die vorgenannten Metalle mindestens 95 Gew.-% des Metallkerns dar, bezogen auf das Gewicht des Metallkerns ohne Sauerstoff. Aufgrund einer in den meisten Fällen schwer vermeidbaren oberflächlichen Oxidation wird der Gehalt an Sauerstoff bei der Berechnung der Anteile der vorgenannten Metalle nicht berücksichtigt.

Bei weiteren Ausführungsformen ist es bevorzugt, dass sich die vorgenannten sowie nachfolgenden Angaben der vorliegenden Erfindung nicht auf das Metall des metallischen Substrats und das Gewicht des Metalls des metallischen Substrats beziehen, sondern auf den Metallkern und das Gewicht des Metallkerns.

Bei weiteren Ausführungsformen ist es bevorzugt, dass das Metall des metallischen Substrats zu mindestens 95 Gew.-% aus einem Metall besteht das ausgewählt wird aus der Gruppe bestehend aus Aluminium, Eisen, Zink, Zinn, Silber, Kupfer, Chrom, Titan und Mischungen davon, bezogen auf das Gewicht des Metalls des metallischen Substrats ohne Sauerstoff.

Bevorzugte Mischungen der Metalle sind Messing (Goldbronze), Zink-Magnesium-Legierungen und Stahl.

Besonders bevorzugt sind Metallkerne, die zu mindestens 95 Gew.-% aus Aluminium, Eisen, Zink, Stahl, Kupfer oder Messing, mehr bevorzugt Aluminium, Kupfer, Eisen oder Messing, bestehen, bezogen auf das Gewicht des Metallkerns ohne Sauerstoff.

Eine besonders bevorzugte Gruppe von metallischen Substraten sind mit Aluminium beschichtete nichtmetallische Substrate und Metallkerne ausgewählt aus Aluminiumpigmenten. Insbesondere bevorzugt sind Metallkerne ausgewählt aus Aluminiumpigmenten. Vorzugsweise besteht bei weiteren Ausführungsformen das Metall des metallischen Substrats zu mindestens 95 Gew.-% aus Aluminium, bezogen auf das Gewicht des Metalls des metallischen Substrats ohne Sauerstoff. Ferner ist es bei weiteren Ausführungsformen bevorzugt, dass der Anteil an anderen Metallen in den metallischen Substraten weniger als 1 Gew.-%, mehr bevorzugt weniger als 0,1 Gew.-% beträgt, bezogen auf das Gewicht des Metalls des metallischen Substrats ohne Sauerstoff.

Eine weitere bevorzugte Gruppe von metallischen Substraten sind kupferhaltige metallische Substrate. Diese weisen einen Gehalt an elementarem Kupfer von wenigstens 50 Gew.-%, mehr bevorzugt von wenigstens 70 Gew.-% auf, jeweils bezogen auf das Gewicht des Metalls des metallischen Substrats ohne Sauerstoff. Insbesondere ist es bevorzugt, dass kupferhaltige Metallkerne eingesetzt werden. Im Sinne der Erfindung wird unter dem vorgenannten Gehalt an elementarem Kupfer auch der in einer Legierung enthaltene Anteil an Kupfer verstanden. Besonders bevorzugt ist der Einsatz von plättchenförmigen kupferhaltigen Pigmenten, nachfolgend auch Kupfereffektpigmente genannt.

Gemäß weiteren Ausführungsformen werden Kupferpigmente, insbesondere Kupfereffektpigmente, als metallische Substrate eingesetzt. "Kupferpigmente" im Sinne der vorliegenden Erfindung weisen vorzugsweise einen Gehalt an elementarem Kupfer von 98 bis 100 Gew.-%, mehr bevorzugt von 99 bis 99,999 Gew.-%, auf, jeweils bezogen auf das Gewicht des Metalls des metallischen Substrats. Insbesondere ist es bei weiteren Ausführungsformen bevorzugt, dass die Kupferpigmente Metallkerne sind. Es muss verstanden werden, dass der Fachmann bei der Angabe 100 Gew.-% Kupfer auch gegebenenfalls in Spurenmengen enthaltene übliche Fremdmetalle mitliest. Der Begriff "Spurenmenge" im Sinne der vorliegenden Erfindung bezeichnet Mengen von vorzugsweise höchstens 0,01 Gew.-%, bezogen auf das Gesamtgewicht des Metalls.

Gemäß weiteren Ausführungsformen werden in der vorliegenden Erfindung Messingpigmente, insbesondere plättchenförmige Messingpigmente, nachfolgend auch Messingeffektpigmente genannt, als metallische Substrate eingesetzt. Der Begriff "Messingpigment" bezeichnet im Sinne der vorliegenden Erfindung metallische Pigmente, bei denen das Metall ausgewählt wird aus einer Legierung bestehend zumindest weitgehend aus Zink und Kupfer. Derartige Pigmente werden auch als Goldbronzepigmente bezeichnet. Vorzugsweise weisen die erfindungsgemäß eingesetzten Messingpigmente, insbesondere Messingeffektpigmente, einen Kupfergehalt von 70 bis weniger als 98 Gew.-%, mehr bevorzugt von 75 bis 90 Gew.-%, auf, jeweils bezogen auf das Gewicht des Metalls des metallischen Substrats ohne Sauerstoff. Insbesondere ist es bei weiteren Ausführungsfomen bevorzugt, dass die Messingpigmente Metallkerne sind.

Zink bildet neben Kupfer einen weiteren Hauptbestandteil des Metalls der Messingpigmente, wobei die Gesamtmenge an Kupfer und Zink bei weiteren Ausführungsformen vorzugsweise mindestens 95 Gew.-%, mehr bevorzugt mindestens 99 Gew.-%, noch mehr bevorzugt mindestens 99,9 Gew.-% darstellt, jeweils bezogen auf das Gewicht des Metalls des kupferhaltigen metallischen Substrats ohne Sauerstoff.

Generell können plättchenförmige Metallpigmente, insbesondere die oben explizit aufgeführten, mittels verschiedener dem Fachmann geläufiger Verfahren gewonnen werden. Beispiele hierfür sind die Vermahlung von Metallgrieß, insbesondere Aluminiumgrieß, kupferhaltiger Grieß und Eisengrieß, oder die Gasphasenabscheidung von Metall, insbesondere von Aluminium, im PVD-Verfahren. Die beiden vorgenannten Herstellverfahren unterscheiden sich nicht allein in der typischerweise erreichten oder erreichbaren Qualität der Pigmente, sondern auch in den Anforderungen hinsichtlich der weiteren Prozessierung, deren Handhabung und deren spezifischen Eigenschaften.

Ein weit verbreitetes Verfahren zur Gewinnung eines breiten Spektrums an Metalleffektpigmenten mit sehr unterschiedlichen Eigenschaften ist die Vermahlung von Metallgrieß. Hierbei wird typischerweise flüssiges Metall verdüst zur Gewinnung eines feinen Metallgrießes. In der Metallschmelze können auch verschiedene Metalle miteinander legiert werden. Ein Beispiel ist die Herstellung von Messinggrieß. Der gewonnene Grieß wird nachfolgend gegebenenfalls klassiert oder nachbehandelt, bevor er vermahlen wird.

Die Vermahlung kann nass oder trocken erfolgen. Die Auswahl der entsprechenden Verfahrensvariante erfolgt basierend auf unter anderem den gewünschten Rahmenbedingungen, den gewünschten Produkten und den eingesetzten Edukten. So erwies sich beispielsweise die Nassvermahlung als sicherheitstechnisch vorteilhaft und erbrachte eine gleichmäßigere und schonendere Verformung auch bei weniger optimierten Verfahrensparametern. Beispielsweise bei der Vermahlung von Aluminium wird typischerweise die Nassvermahlung bevorzugt. Die Trockenvermahlung bietet hingegen eine vereinfachte Prozessierung, da beispielsweise eine nachfolgende Umnetzung auf ein anderes Lösungsmittel entfällt. Sie findet Anwendung beispielsweise bei der Vermahlung von Kupfer- bzw. Messinggrieß zu plättchenförmigen Kupfer bzw. Messingpigmenten. Der Begriff "Nassvermahlung" beschreibt eine Vermahlung der Pigmente in Gegenwart eines Lösungsmittels.

Bei der Vermahlung wird der Metallgrieß in Kugelmühlen in mehreren Mahlstufen bei unterschiedlichen Mahlbedingungen, wie beispielsweise Mühlengröße, -durchmesser, - drehgeschwindigkeit, Kugelgröße, Mahldauer unter Zugabe von Schmiermittel, wie beispielsweise Stearin- oder Ölsäure, zur Verhinderung einer Kaltverschweißung der Metallpartikel und mit Mahlkörpern, wie z. B. Stahlkugeln, vermahlen. Die plättchenförmigen Metallpigmente werden nach dem Vermahlen und optionalen Klassieren in verschiedenen Behältnissen gesammelt und anschließenden homogenisiert bzw. gemischt.

Weitere Informationen zu einem hierbei einsetzbaren Vermahlungsverfahren finden sich in der US 2011/0179971 A1, auf deren Offenbarung hiermit in ihrer Gesamtheit Bezug genommen wird.

Die vorgenannten mittels Vermahlung hergestellten Metalleffektpigmente können beispielsweise gemäß dem in der US 2007/0199478 A1 oder US 2010/0047199 A1 beschriebenen Verfahren hergestellt werden.

Gemäß einer weiteren Ausführungsform werden daher mittels Vermahlung erhaltener Pigmente mit einem $h_{50}$-Wert im Bereich von 20 bis 100 nm, einem Formfaktor von mindestens 200 und einer relativen Dickenverteilung $\Delta h$-Wert im Bereich von 30 bis 140 % als Metallkerne verwendet. Der $\Delta h$-Wert wird gemäß Formel VII berechnet:

$$\Delta h = (h_{90}\text{-}h_{10})/h_{50} \qquad (VII)$$

Die Herstellung derartiger Metallpigmente werden in den EP 1 613 702 B1, EP 2 102 294 B1 und der EP 2 128 203 A1 beschrieben.

PVD-Metalleffektpigmente weisen eine absolut plane Oberfläche und hervorragende optische Eigenschaften auf. Insbesondere ist der Aufbau von mittels physikalischer Dampfabscheidung hergestellter Pigmente nahezu ideal für optische Effekte. Die resultierenden herausragenden optischen Eigenschaften machen diese Pigmente für höchstqualitative Anwendungen besonders interessant.

Ein Problem der vorgenannten Pigmente, insbesondere der PVD-Pigmente, war bislang deren starke Agglomerationsneigung, welche ein Konzentrieren schwierig und ein Trocknen unmöglich machten, ohne die vorteilhaften Eigenschaften, insbesondere die optischen Eigenschaften, signifikant zu verschlechtern.

Unter Verwendung von mittels Vermahlung erhaltener Pigmente mit einem $h_{50}$-Wert im Bereich von 20 bis 100 nm, einem Formfaktor von mindestens 200 und einen $\Delta h$-Wert im Bereich von 30 bis 140 %, oder von PVD-Pigmenten als metallische Substrate für die erfindungsgemäßen Metalleffektpigmente, werden hochbrillante Pigmente zur Verwendung in Farben, Druckfarben, Lacken und Kosmetika erhalten. Sowohl bei durch Vermahlung erhaltenen Pigmenten als auch bei PVD-Pigmenten werden insbesondere von Aluminium-, Kupfer-, Messing- (Goldbronze) und Eisenpigmente, vorzugsweise Aluminiumpigmente, bevorzugt.

Bei weiteren Ausführungsformen ist es bevorzugt, dass sowohl die mittels PVD-Verfahren als auch die mittels Vermahlung erhaltenen Pigmente einen $h_{50}$-Wert im Bereich von 20 bis 100 nm, einen Formfaktor von mindestens 200 und einen $\Delta h$-Wert im Bereich von 20 bis unter 70 %, vorzugsweise von 25 bis 65 %, noch mehr bevorzugt von 30 bis 60 %, aufweisen. Vorzugsweise liegt der $h_{50}$-Wert im Bereich von 23 bis 50 nm, der Formfaktor bei mindestens 250 und der $\Delta h$-Wert im Bereich von 20 bis unter 70 %, vorzugsweise von 25 bis 65 %, noch mehr bevorzugt von 30 bis 60 %. Die Herstellung derartiger Aluminiumpigmente durch Nassmahlung ist beispielsweise in der US 2010/047199 (A1) beschrieben.

Beispielsweise machen die Verwendung der Polymerfolien oder auch die hohen Energiemengen, die zum Verdampfen der Metalle benötigt werden, die Herstellung von PVD-Pigmenten sehr kostspielig. Werden besonders hochwertige Pigmente mit herausragender Brillanz benötigt, so sind bei weiteren Ausführungsformen die mittels PVD-Verfahren hergestellten Metallpigmente bevorzugt. Werden hingegen sehr hochwertige Pigmente mit hervorragender Brillanz und geringen Kosten benötigt, so werden die mittels Vermahlung hergestellten Metallpigmente bevorzugt.

Überraschenderweise zeigte sich ferner, dass durch die Aufbringung eines vorkondensierten Heteropolysiloxans auf eine metalloxidhaltige Beschichtungsschicht, beispielsweise die erfindungsgemäße anorganisch/organische Hybridschicht, deutlich höhere Konzentrationen an Pigmentsuspensionen genutzt werden können, ohne dass merkliche Agglomerationen auftreten. Besonders überraschend war, dass aus besonders zur Agglomeration neigenden PVD-Pigmenten und besonders hochwertigen mittels Vermahlung erhaltenen Pigmenten mit einer zusätzlichen Beschichtung aus vorkondensierten Heteropolysiloxanen sogar ein Pulver ohne Zusatz von Bindemitteln erhalten werden konnte.

Bei weiteren Ausführungsformen wurde auf mindestens einer anorganisch/organischen Hybridschicht mindestens ein vorkondensiertes Heteropolysiloxan aufgebracht, wobei das mindestens eine vorkondensierte Heteropolysiloxan mindestens ein Aminosilan und mindestens ein Silan, das ausgewählt wird aus der Gruppe bestehend aus Alkylsilanen, Vinylsilanen und Arylsilanen, umfasst und das Heteropolysiloxan in vorkondensierter Form aufgebracht wurde.

Die Möglichkeit, qualitativ hochwertige, jedoch empfindliche Pigmente, wie insbesondere PVD-Pigmente, in stark konzentrierter Form ohne Gefahr von wesentlichen Qualitätseinbußen verarbeiten zu können, führt zu mehreren Vorteilen. Beispielsweise wird die Handhabung der Pigmente vereinfacht, und es werden die Anwendungsmöglichkeiten stark erweitert. Auch wird die Reinigung der Pigmente vereinfacht, und es wird eine gleichbleibend hohe Qualität bei dem Endanwender bei verringerten Anforderungen an Transport, Lagerung und Prozessierung gewährleistet.

Die Möglichkeit einer signifikanten Konzentrierung von PVD-Pigmenten und sogar deren Trocknung ermöglicht eine gänzlich neue Handhabung der Pigmente. Beispielsweise können entsprechende Pigmente mittels einfacher Verfahrensschritte ohne merkliche Qualitätsverluste vor weiteren Aufarbeitungsschritten abfiltriert werden. Beispielsweise können die Pigmente nach vollständiger Entfernung des Lösungsmittels direkt in lösemittelfreien Lackanwendungen eingesetzt werden. Beispielsweise wird der Transport und die Lagerung infolge der Abtrennungsmöglichkeit des Lösungsmittels sehr stark vereinfacht. Beispielsweise sind neue Modifizierungsmöglichkeiten, beispielsweise eine Modifizierung der Pigmente in einer Gasphasenreaktion, gegeben. Beispielsweise ermöglicht die vorliegende Erfindung die Verwendung der Pigmente in lösemittelfreien Systemen, wie Pulverlack oder Kunststoffen, oder in Systemen bei denen Lösemittel Probleme bereiten können, wie UV-Lacke und Druckfarben. Beispielsweise können Bestandteile leicht entfernt werden, welche im Produkt zur Migration neigen würden. Dies vereinfacht oder ermöglicht erst die Verwendung von Pigmenten im Lebensmittelbereich. Beispielsweise wird ein Umnetzen auf ein anderes Lösemittel, d.h. ein Austausch des Lösemittels, deutlich vereinfacht.

Die vorkondensierten Heteropolysiloxane weisen bevorzugt ein mittleres Molekulargewicht von mindestens 500 g/mol, besonders bevorzugt von mindestens 750 g/mol und ganz besonders bevorzugt von mindestens 1000 g/mol auf. Das mittlere Molekulargewicht kann beispielsweise mittels NMR-spektrokopischen Methoden wie $^{29}$Si-NMR gegebenenfalls in Verbindung mit $^{1}$H-NMR erfolgen. Eine Beschreibung derartiger Verfahren findet sich beispielsweise in Publikationen wie "Organofunctional alkoxysilanes in dilute aqueous solution: new accounts on the dynamic structural mutability", Journal of Organometallic Chemistry 625 (2001) 208-216.

Ferner zeigte sich überraschenderweise bei den erfindungsgemäßen Pigmenten eine gute Applizierbarkeit, sehr gute Deckung und Abriebbeständigkeit bei der Verwendung in einem Pulverlack. Insbesondere erwies sich die erfindungsgemäß aufzubringende Beschichtung als vorteilhaft bei der Beschichtung ferromagnetischer Pigmente, welche zudem während oder nach Applikation des Pulverlackes besonders gut ausgerichtet werden können.

Bei weiteren Ausführungsformen ist es bevorzugt, dass die erfindungsgemäßen Pigmente eine Beschichtungsschicht aufweisen, die durch Aufbringung eines vorkondensierten Heteropolysiloxans erzeugt wird. Insbesondere ist es bevorzugt, dass das vorkondensierte Heteropolysiloxan auf eine metalloxidhaltige Beschichtungsschicht, vorzugsweise die erfindungsgemäße anorganisch/organische Hybridschicht, aufgebracht wird.

Das vorkondensierte Heteropolysiloxan kann auf verschiedene Weisen aufgetragen werden. Besonders vorteilhaft hat sich die Zugabe des Polysiloxans, vorzugsweise in gelöster bzw. dispergierter Form, zu einer Suspension, umfassend die zu beschichtenden Metallpigmente, erwiesen. Hierbei kann für die Bereitstellung der Suspension, umfassend die zu beschichteten Metallpigmente, beispielsweise ein Reaktionsprodukt erhalten aus einem vorhergehenden Beschichtungsschritt eingesetzt werden.

Die Struktur der vorkondensierten Heteropolysiloxane kann insbesondere kettenförmig, cyclisch, vernetzt oder Mischungen davon sein.

Ferner ist es bei weiteren Ausführungsformen bevorzugt, dass das vorkondensierte Heteropolysiloxan mindestens zu 87 Gew-%, vorzugsweise mindestens zu 93 Gew-%, mehr bevorzugt mindestens zu 97 Gew-%, bezogen auf das Gesamtgewicht des vorkondensierten Heteropolysiloxans, aus Silanmonomerkomponenten aufgebaut ist, die ausgewählt werden aus der Gruppe bestehend aus Aminosilanen, Alkylsilanen, Vinylsilanen, Arylsilanen und Mischungen davon. Insbesondere ist es bevorzugt, dass das vorkondensierte Heteropolysiloxan aus den Aminosilankomponenten und Alkylsilankomponenten in den vorgenannten Mengen aufgebaut ist.

Die Silanmonomere werden beispielsweise als Alkoxide eingesetzt. Diese Alkoxidbindung wird zur Polymerisation gespalten, und infolge eines Kondensationsschrittes werden die Silanmonomere zu dem jeweiligen vorkondensierten Heteropolysiloxan umgesetzt bzw. vernetzt. Vorzugsweise werden als Alkoxide für die vorliegende Erfindung Methoxide und Ethoxide eingesetzt. Sofern es nicht anders spezifiziert ist, basieren die Gew.-% der Silanmonomerkomponenten in dem vorkondensierten Heteropolysiloxan im Sinne der vorliegenden Erfindung auf dem Gewicht der Silanmonomere ohne die Bestandteile, welche infolge der Kondensation zum vorkondensierten Heteropolysiloxan abgespalten werden, wie beispielsweise Alkoxygruppen. Die Herstellung derartiger Polysiloxane ist in der Literatur beschrieben. Beispielsweise finden sich entsprechende Herstellverfahren in der US 5,808,125 A, US 5,679,147 A und US 5,629,400 A.

Als besonders vorteilhafte Aminosilane, welche für den Aufbau der erfindungsgemäß verwendbaren vorkondensierten Heteropolysiloxane eingesetzt werden können, haben sich Aminosilane mit 1 oder 2 Aminogruppen pro Si-Atom erwiesen. Bei weiteren Ausführungsformen werden die im vorkondensierten Heteropolysiloxan enthaltenen Aminosilankomponenten zu mindestens 92 Gew.-%, vorzugsweise zu mindestens 97 Gew.-% ausgewählt aus Aminosilanen mit 1 oder 2 Aminogruppen, jeweils bezogen auf das Gesamtgewicht der in dem vorkondensierten Heteropolysiloxan enthaltenen Aminosilankomponenten.

Beispielsweise haben sich $(H_2N(CH_2)_3)Si(OCH_3)_3$ ((3-Aminopropyl)(trimethoxy)silan, AMMO),$(H_2N(CH_2)_3)Si(OC_2H_5)_3$ ((3-Aminopropyl)(triethoxy)silan, AMEO), $(H_2N(CH_2)_2NH(CH_2)_3)Si(OCH_3)_3$ ((N-(2-Aminoethyl)-3-aminopropyl)(trimethoxy)silan, (DAMO)),$(H_2N(CH_2)_2NH(CH_2)_3)Si(OC_2H_5)_3$ ((N-(2-Aminoethyl)-3-aminopropyl)(triethoxy)silan) und deren Mischungen als vorteilhaft erwiesen. Bei weiteren Ausführungsformen werden die im vorkondensierten Heteropolysiloxan enthaltenen Aminosilankomponenten zu mindestens 92 Gew.-%, vorzugsweise zu mindestens 97 Gew.-%, aus der vorgenannten Gruppe und deren Mischungen ausgewählt, jeweils bezogen auf das Gesamtgewicht der in dem vorkondensierten Heteropolysiloxan enthaltenen Aminosilankomponenten.

Bei weiteren Ausführungsformen ist es bevorzugt, dass das erfindungsgemäß verwendete vorkondensierte Heteropolysiloxan nur geringe oder gar keine Mengen an Epoxysilanen enthält. So zeigten Pigmente umfassend entsprechende vorkondensierte Heteropolysiloxane in gebräuchlichen Nasslacksystemen typischerweise eine bessere Anhaftung. Insbesondere ist es bei weiteren Ausführungsformen bevorzugt, dass das vorkondensierte Heteropolysiloxan höchstens 10 Gew.-%, vorzugsweise höchstens 6 Gew.-%, mehr bevorzugt höchstens 4 Gew.-% und noch mehr bevorzugt höchstens Spurenmengen von Epoxysilankomponenten umfasst, jeweils bezogen auf das Gesamtgewicht des vorkondensierten Heteropolysiloxans.

Ferner hat sich gezeigt, dass bereits geringe Mengen an vorkondensierten Heteropolysiloxan typischerweise ausreichend sind. Bei weiteren Ausführungsformen weist die Beschichtungsschicht, umfassend das mindestens eine, vorzugsweise eine, vorkondensierte Heteropolysiloxan, eine mittlere Dicke von höchstens 20 nm, mehr bevorzugt höchstens 10 nm auf. Insbesondere ist es hierbei bevorzugt, wenn das mindestens eine, vorzugsweise eine, vorkondensierte Heteropolysiloxan im Wesentlichen als Monolage vorliegt.

Als besonders vorteilhaft hat es sich erwiesen, wenn mindestens ein vorkondensiertes Heteropolysiloxan auf eine umhüllende Beschichtungsschicht umfassend Siliciumoxid aufgebracht wurde.

Die erfindungsgemäß eingesetzten vorkondensierten Heteropolysiloxane können durch Kondensation von beispielsweise Alkylsilanen und Aminosilanen hergestellt werden. Dem Fachmann ist jedoch bewusst, dass identische vorkondensierte Heteropolysiloxane auch auf anderem Wege, beispielsweise durch Reaktion mindestens eines Alkylsilans, mindestens eines Halogenalkylsilans und mindestens eines Amins hergestellt werden können. Derartige vorkondensierte Heteropolysiloxane, welche formal auch als Kondensationsprodukte entsprechender Alkylsilane und Aminosilane betrachtet werden könnten, sind vorliegend erfindungsgemäß umfasst. Der Fachmann kann in Kenntnis der vorliegenden Erfindung und im Hinblick auf das bekannte Fachwissen unter verschiedenen retrosynthetischen Routen auswählen.

Bei weiteren Ausführungsformen ist es ferner bevorzugt, dass höchstens 1 Gew.-% der Silanmonomerkomponenten fluorhaltige Silane sind, bezogen auf das Gesamtgewicht des vorkondensierten Heteropolysiloxans. Vorzugsweise sind fluorhaltige Silankomponenten lediglich in Spurenmengen oder mehr bevorzugt nicht in der aufgebrachten vorkondensierten Heteropolysiloxanschicht enthalten.

Der Begriff "Aminosilan" bedeutet im Sinne der vorliegenden Erfindung, dass das betreffende Silan mindestens eine Aminogruppe aufweist. Diese Aminogruppe muss nicht direkt an das Siliciumatom der Silylgruppe gebunden sein.

Beispiele von erfindungsgemäß verwendbaren Aminosilanen bei der Herstellung des vorkodensierten Heteropolysiloxans sind (6-Amino-n-hexyl)(triethoxy)silan, (6-Amino-n-hexyl)(trimethoxy)silan, (4-Amino-3,3-dimethylbutyl)(trimethoxy)silan, $H_2N(CH_2)_3Si(OCH_3)_3$ ((3-Aminopropyl)(trimethoxy)silan, AMMO), $H_2N(CH_2)_3Si(OC_2H_5)_3$ ((3-Aminopropyl)(triethoxy)silan, AMEO), (3-Aminoisobutyl)(trimethoxy)silan, (3-Aminoisobutyl)(triethoxy)silan, (2-Aminoethyl)(trimethoxy)silan, (2-Aminoethyl)(triethoxy)silan, (Aminomethyl)-(trimethoxy)silan, (Aminomethyl)(triethoxy)silan, (N-Cyclohexylaminomethyl)(triethoxy)silan (GENIOSIL XL 926), (N-Phenylaminomethyl)(trimethoxy)silan, (6-Amino-n-hexyl)(methyl)(dimethoxy)silan, (3-Aminopropyl)(methyl)(dimethoxy)silan, (3-Aminopropyl)(methyl)(diethoxy)silan, (2-Aminoethyl)(phenyl)(dimethoxy)silan, (2-Aminoethylamino)(ethyl)(triethoxy)silan (2-Aminoethyl)(methyl)(diethoxy)silan, (2-Aminoethyl)(methyl)(dimethoxy)silan, (1-Aminomethyl)(methyl)(diethoxy)silan, (N-Cyclohexyl-aminomethyl)(methyl)(dieethoxy)silan (GENIOSIL XL 924), (N-Ethylamino-i-butyl)(trimethoxy)silan, (N-n-Butyl-3-aminopropyl)(trimethoxy)silan, (N-n-Butyl-3-aminopropyl)(triethoxy)silan, (N-n-Butyl-1-aminomethyl)(triethoxy)silan, (N-n-Butyl-1-aminomethyl)(trimethoxy)silan, (Benzyl-3-aminopropyl)(trimethoxy)silan, (Benzyl-3-aminopropyl)(triethoxy)silan, (N-Phenylaminomethyl)(trimethoxy)silan (GENIOSIL XL 973), (N-Phenylaminopropyl)¬(trimethoxy)silan (N-Formyl-3-aminopropyl)(triethoxy)silan, (N-Formyl-3-aminopropyl)(trimethoxy)silan, (N-Formyl-1-aminomethyl)(methyl)(dimethoxy)silan, (N-Formyl-1-aminomethyl)(methyl)(diethoxy)silan, (N-n-Butyl-3-aminopropyl)(methyl)(diethoxy)silan, (N-n-Butyl-3-aminopropyl)(methyl)(dimethoxy)silan, (N-n-Butyl-1-aminomethyl)-(methyl)(dimethoxy)silan, (N-Butyl-1-aminomethyl)(methyl)(diethoxy)silan, (Diaminoethylen-3-propyl)(triethoxy)silan, (N-(2-Aminoethyl)aminoethyl)(trimethoxy)silan, (2-Aminoethylaminoethyl)(triethoxy)silan, (N-(1-Aminoethyl)aminomethyl)(trimethoxy)silan, (N-(1-Aminoethyl)aminomethyl)(triethoxy)silan, $H_2N(CH_2)_2NH(CH_2)_3Si(OCH_3)_3$ ((N-(2-Aminoethyl)-3-aminopropyl)(trimethoxy)silan, (DAMO)), (2-Aminoethylaminopropyl)(triethoxy)silan (Silquest A-1130), (2-Aminoethylaminoethyl)-(trimethoxy)silan, (2-Aminoethylaminoethyl)(triethoxy)silan, (1-Aminoethylaminopropyl)(trimethoxy)silan, (1-Aminoethylaminopropyl)(triethoxy)silan, (1-Aminoethylaminomethyl)(trimethoxy)silan, (1-Aminoethylaminomethyl)(triethoxy)silan, (N-Cyclohexyl-3-aminopropyl)-(trimethoxy)silan, (N-(N-Benzylaminoethyl)aminopropyl)(trimethoxy)silan, (3-Ureidopropyl)(trimethoxy)silan, (3-Ureidopropyl)(triethoxy)silan, (N-(2-Aminoethyl)-3-aminopropyl)(methyl)(dimethoxy)silan, (N-(2-Aminoethyl)-3-aminopropyl)(methyl)(diethoxy)silan, $H_2N(CH_2)_2NH(CH_2)_2NH(CH_2)_3Si(OCH_3)_3$ ((Triaminodiethylen-3-propyl)(trimethoxy)silan, TRIAMO), (Triaminodiethylen-3-propyl)(triethoxy)silan, (Triaminodiethylen-3-propyl)(trimethoxy)silan, (Triaminodiethylen-3-propyl) (triethoxy)silan, (((Aminoethyl)aminoethyl)amino-propyl)(trimethoxy)silan, (((Aminoethyl)aminoethyl)aminopropyl)(triethoxy)silan, Bis-(trimethoxysilan)amin, Bis(triethoxysilan)amin, Bis(trimethoxysilylethyl)amin, Bis-(triethoxysilylmethyl)amin, Bis(triethoxysilylethyl)amin, Bis(tri-methoxysilylpropyl)amin, Bis(triethoxysilylpropyl)amin, Bis(trimethoxysilylisopropyl)amin, Bis(triethoxysilylisopropyl)amin, (3-Trimethoxy)silylmethyl-O-methylcarbamat, N-Dimethoxy-(methyl)silylmethyl-O-methyl-carbamat, Triethoxysilylpropyl)-t-butylcarbamate, Triethoxysilyl-propylethylcarbamate, Tris(trimethoxysilylmethyl)amine, Tris(trimethoxysilylethyl)amin, Tris(trimethoxysilyl-n-propyl)amin, Tris(trimethoxysilyl-i-propyl)amin, $N[CH_2)_3Si(OC_2H_5)_3]_3$ (Tris(triethoxysilylmethyl)amin, Tris-AMEO), Tris(triethoxysilylmethyl)amin, Tris(triethoxysilylethyl)amin, Tris(triethoxysilyl-n-propyl)amin, Tris(triethoxysilyl-i-propyl)amin, $N[CH_2)_3Si(OCH_3)_3]_3$ (Tris-AMMO), $(H_5C_2O)_3Si(CH_2)_3NH(CH_2)_3Si(OC_2H_5)_3$, (Bis(triethoxysilylpropyl)amin, Bis-AMEO), $(H_3CO)_3Si(CH_2)_3NH(CH_2)_3Si(OCH_3)_3$ (Bis(trimethoxysilylpropyl)amin. Bis-AMMO), $(H_3CO)_3Si(CH_2)_3NH(CH_2)_2NH(CH_2)_3Si(OCH_3)_3$ (Bis-DAMO), $(H_3CO)_3Si(CH_2)_3NH(CH_2)_2NH(CH_2)_2NH(CH_2)_3Si(OCH_3)_3$ ( Bis-TRIAMO), $(H_3CO)_2(CH_3)Si(CH_2)_3NH(CH_2)_2NH(CH_2)_3Si(OCH_3)_2(CH_3)$, $(H_3CO)_3(CH_3)Si(CH_2)SiNH(CH_2)_2NH(CH_2)_3Si(OCH_3)_2(CH_3)$, $(H_3CO)_3Si(CH_2)_3NH(CH_2)_2NH(CH_2)_2NH(CH_2)_3Si(OCH_3)_3$ (Bis-DAMO), $(H_3CO)_3Si(CH_2)_3NH(CH_2)_2NH(CH_2)_2NH(CH_2)_2NH(CH_2)_3Si(OCH_3)_3$ (BiS-TRIAMO), (3-(Trimethoxysilyl)methyl)(O-methyl)carbamat, (N-(Dimethoxy)(methyl)silyl)methyl)(O-methyl)carbamat, (3-(Triethoxysilyl)propyl)(t-butyl)carbamat, ((Triethoxysilyl)propyl)(ethyl)carbamat. Bei weiteren Ausführungsformen wird vorzugsweise mindestens ein Aminosilan, mehr bevorzugt mindestens 95 Gew.-% der Aminosilane, bezogen auf das Gesamtgewicht der in dem vorkondensierten Heteropolysiloxans enthaltenen Aminosilankomponenten, noch mehr bevorzugt sämtliche Aminosilane, aus den vorgenannten Beispielen ausgewählt.

Insbesondere ist es bevorzugt, dass das mindestens eine Aminosilan ausgewählt wird aus der Gruppe der Aminosilane gemäß Formel (I):

$$R^{a1}_{x1}R^{b1}_{y1}R^{c1}_{(4-x1-y1)}Si \qquad (I)$$

, wobei die $R^{a1}$ unabhängig voneinander ausgewählt werden aus mit mindestens einer Stickstoffgruppe substituierten funktionellen Gruppen, wobei die funktionelle Gruppe ausgewählt wird aus der Gruppe bestehend aus C1-C16 Alkylgruppen, C2-C8 Alkenylgruppen, C2-C8 Alkinylgruppen und Phenylgruppen, C7-C12 Alkylarylgruppen und C7-C12 Arylalkylgruppen,
die $R^{b1}$ unabhängig voneinander ausgewählt werden aus der Gruppe bestehend aus C1-C16 Alkylgruppen, C2-C8 Alkenylgruppen, C2-C8 Alkinylgruppen, Phenylgruppen, C7-C12 Arylalkylgruppen und C7-C12 Alkylarylgruppen, wobei die vorgenannten Gruppen unsubstituiert sind,
die $R^{c1}$ unabhängig voneinander ausgewählt werden aus der Gruppe der Alkoxygruppen,
x1 = 1, 2 oder 3 ist, und
y1 aus der Gruppe der ganzen Zahlen von 0 bis (3-x1) ausgewählt wird.

Vorzugsweise werden mindestens 95 Gew.-% der Aminosilane, bezogen auf das Gesamtgewicht der in dem vorkondensierten Heteropolysiloxan enthaltenen Aminosilankomponenten, mehr bevorzugt sämtliche Aminosilane, aus den vorgenannten Silanen ausgewählt. Ferner ist es bevorzugt, dass x1 = 1 oder 2 ist und y1 aus der Gruppe der ganzen Zahlen von 0 bis (2-x1) ausgewählt wird.

Wird in den Ausführungsformen der vorliegenden Erfindung eine Variable aus einem Bereich ganzer Zahlen ausgewählt, so sind die genannten Endpunkte des Zahlenbereichs mit eingeschlossen.

Soweit es in der vorliegenden Anmeldung nicht anderweitig spezifiziert ist, können die hierin genannten Gruppen wie Alkylgruppen, Alkenylgruppen, Alkinylgruppen und Alkoxygruppen in dieser oder in den anderen Ausführungsformen der vorliegenden Anmeldung verzweigt oder unverzweigt vorliegen.

Bei weiteren Ausführungsformen werden die $R^{a1}$ unabhängig voneinander ausgewählt aus mit mindestens einer Stickstoffgruppe substituierten funktionellen Gruppen, wobei die funktionelle Gruppe ausgewählt wird aus der Gruppe bestehend aus C1-C5 Alkylgruppen, C2-C5 Alkenylgruppen und C2-C5 Alkinylgruppen und Mischungen davon, die $R^{b1}$ unabhängig voneinander ausgewählt werden aus der Gruppe bestehend aus C1-C4 Alkylgruppen, C2-C4 Alkenylgruppen, C2-C4 Alkinylgruppen, Phenylgruppen und Mischungen davon, wobei die vorgenannten Gruppen von $R^{b1}$ unsubstituiert sind, und die $R^{c1}$ unabhängig voneinander ausgewählt werden aus der Gruppe der C1-C4 Alkoxygruppen.

Insbesondere ist es bevorzugt, dass die $R^{c1}$ ausgewählt werden aus der Gruppe bestehend aus Methoxy und Ethoxy.

Bei weiteren Ausführungsformen ist es bevorzugt, dass die mindestens eine Stickstoffgruppe von $R^{a1}$ ausgewählt wird aus der Gruppe bestehend aus $-NH_{(2-r1)}R^{d1}_{r1}$ und $-(NH_{(3-s1)}R^{d1}_{s1})^+$, wobei r1 aus den ganzen Zahlen von 0 bis 2 und s1 aus den ganzen Zahlen von 0 bis 3 ausgewählt wird und die $R^{d1}$ unabhängig voneinander ausgewählt werden aus der Gruppe bestehend aus C1-C16 Alkylgruppen, C2-C8 Alkenylgruppen, C2-C8 Alkinylgruppen, Phenylringen, C7-C12 Alkylarylgruppen, C7-C12 Alkoxyalkylgruppen, Dialkylendiamingruppen und Trialkylentriamingruppen, sowie gesättigten und ungesättigten Alkylenen und Heteroalkylenen wie $-(CH_2)_3-$, $-(CH_3)_4-$, $-(CH_2)_5-$, $-CH=CH-CH=CH-$ oder $-CH=N-(CH_2)_2-$ falls r1 oder s1 $\geq$ 2 ist, wobei die Heteroatome in den Heteroalkylenen aus N und O ausgewählt werden. Vorzugsweise sind die Heteroatome der Heteroalkylene Stickstoffatome. Gegebenenfalls vorhandenen Substituenten der vorgenannten Gruppen werden vorzugsweise ausgewählt werden aus stickstoffhaltigen Substituenten wie $-NH_{(2-t1)}R^{e1}_{t1}$ und $-(NH_{(2-u1)}R^{e1}_{u1})^+$, wobei t1 aus den ganzen Zahlen von 0 bis 2 ausgewählt wird, u1 aus den ganzen Zahlen von 0 bis 3 ausgewählt wird, und die $R^{e1}$ ausgewählt werden aus der Gruppe bestehend aus C1-C4 Alkylgruppen. Insbesondere ist es bevorzugt, dass die vorgenannten $R^{d1}$ unsubstituiert sind.

Bei weiteren Ausführungsformen werden die $R^{d1}$ ausgewählt aus der Gruppe bestehend aus C1-C4 Alkylgruppen, C2-C4 Alkenylgruppen, C2-C4 Alkinylgruppen, Phenylringen, C7-C8 Alkylarylgruppen, C7-C8 Alkoxyalkylgruppen, Dialkylendiamingruppen und Trialkylentriamingruppen, sowie gesättigten und ungesättigten C4-C7 Alkylenen und C3-C6 Heteroalkylenen wie $-(CH_2)_3-$, $-(CH_3)_4-$, $-(CH_2)_5-$, $-CH=CH-CH=CH-$ oder $-CH=N-(CH_2)_2-$ falls r1 oder s1 $\geq$ 2 ist, wobei die Heteroatome in den Heteroalkylenen aus N und O ausgewählt werden.

Ferner ist es bei weiteren Ausführungsformen bevorzugt, dass die mindestens eine Stickstoffgruppe von $R^{a1}$ ausgewählt wird aus der Gruppe bestehend aus $-NH_{(2-r1)}R^{d1}_{r1}$ und $-(NH_{(3-s1)}R^{d1}_{s1})^+$, wobei r1 aus den ganzen Zahlen von 0 bis 2 und s1 aus den ganzen Zahlen von 0 bis 3 ausgewählt wird und die $R^{d1}$ unabhängig voneinander ausgewählt werden aus der Gruppe bestehend aus unsubstituierten und substituierten C1-C8 Alkylgruppen, vorzugsweise C1-C4 Alkylgruppen, wobei die Substituenten ausgewählt werden aus der Gruppe bestehend aus $-NH_{(2-t1)}R^{e1}_{t1}$ und $-(NH_{(3-u1)}R^{e1}_{u1})^+$, wobei t1 aus den ganzen Zahlen von 0 bis 2 und u1 aus den ganzen Zahlen von 0 bis 3 ausgewählt wird und die $R^{e1}$ unabhängig voneinander ausgewählt werden aus der Gruppe bestehend aus unsubstituierten C1-C4 Alkylgruppen und C1-C4 Aminoalkylgruppen.

Bei weiteren Ausführungsformen ist es bevorzugt, dass mindestens ein Aminosilan ausgewählt wird aus der Gruppe bestehend aus Aminoalkyltrialkoxysilanen, Bis(aminoalkyl)dialkoxysilanen, (Alkyl)(aminoalkyl)(dialkoxy)silan, ((Aminoalkyl)aminoalkyl)(trialkoxy)silanen, Bis(trialkoxysilylalkyl)aminen, Tris(trialkoxylalkyl)aminen, Bis-N,N'-(trialkoxysilylalkyl)alkylendiaminen, Bis- N,N'-(trialkoxysilylalkyl)dialkylentriaminen, wobei die Alkylgruppen unabhängig voneinander ausgewählt werden aus der Gruppe bestehend aus Methylgruppe, Ethylgruppe, Propylgruppen und Butylgruppen, und die Alkoxygruppen unabhängig voneinander ausgewählt werden aus der Gruppe bestehend aus Methoxygruppe und Ethoxygruppe. Besonders bevorzugt wird mindestens ein Aminosilan ausgewählt aus der Gruppe bestehend aus Aminoalkyltrialkoxysilanen, ((Aminoalkyl)aminoalkyl)(trialkoxy)silanen und Bis(trialkoxysilylalkyl)aminen.

Gemäß der vorliegenden Erfindung wird zwischen Alkylsilanen und den in verschiedenster Weise funktionalisierten Silanen wie Aminosilanen unterschieden. Der Begriff "Alkylsilan" im Sinne der vorliegenden Erfindung umfasst keine funktionalisierten Silane wie Aminosilane, auch wenn diese beispielsweise zusätzlich zu einer Aminoalkylgruppe eine unsubstituierte Alkylgruppe aufweisen können. Beispiele des mindestens einen Alkylsilans sind (Methyl)(trialkoxy)silan, (Ethyl)(trialkoxy)silan, (n-Propyl)(trialkoxy)silan, (i-Propyl)(trialkoxy)silan, (n-Butyl)(trialkoxy)silan, (i- Butyl)(trialkoxy)silan, (n-Octyl)(trialkoxy)silan, (i-Octyl)(trialkoxy)silan, (Decyl)(trialkoxy)silan, (Dodecyl)(trialkoxy)silan, (Hexadecyl)(trialkoxy)silan, (Dimethyl)(dialkoxy)silan, wobei alkoxy für methoxy, ethoxy und Mischungen davon steht. Vorzugsweise wird mindestens ein Alkylsilan, vorzugsweise mindestens 95 Gew.-% der Alkylsilane, bezogen auf das Gesamtgewicht der in dem vorkondensierten Heteropolysiloxan enthaltenen Alkylsilankomponenten, mehr bevorzugt sämtliche Alkylsilane, aus den vorgenannten Beispielen ausgewählt.

Bei weiteren Ausführungsformen ist es bevorzugt, dass das mindestens eine Alkylsilan eine Struktur gemäß Formel (II) aufweist

$$R^{a2}_{x2}R^{b2}_{(4-x2)}Si \qquad (II)$$

wobei die $R^{a2}$ unabhängig voneinander ausgewählt werden aus der Gruppe der unsubstituierten C1-C16 Alkylgruppen, die $R^{b2}$ unabhängig voneinander ausgewählt werden aus der Gruppe der Alkoxygruppen und x2 aus 1 und 2 ausgewählt wird. Vorzugsweise werden mindestens 95 Gew.-% der Alkylsilane, bezogen auf das Gesamtgewicht der in dem Heteroppolysiloxan enthaltenen Alkylsilankomponenten, mehr bevorzugt sämtliche Alkylsilane, aus den vorgenannten Silanen ausgewählt.

Bei weiteren Ausführungsformen ist es bevorzugt, dass die $R^{a2}$ unabhängig voneinander ausgewählt werden aus der Gruppe der unsubstituierten C1-C8 Alkylgruppen, und die $R^{b2}$ unabhängig voneinander ausgewählt werden aus der Gruppe der C1-C4 Alkoxygruppen.

Bei weiteren Ausführungsformen ist es bevorzugt, dass mindestens ein $R^{a2}$ des mindestens einem Alkylsilans gemäß Formel (II) ausgewählt wird aus der Gruppe der unsubstituierten C1-C3 Alkylgruppen.

Insbesondere ist es bevorzugt, dass die $R^{c2}$ ausgewählt werden aus der Gruppe bestehend aus Methoxy und Ethoxy.

Bei weiteren Ausführungsformen ist es bevorzugt, dass die $R^{a2}$ ausgewählt werden aus der Gruppe der C1-C8 Alkylgruppen, mehr bevorzugt der C1-C6 Alkylgruppen und noch mehr bevorzugt der C1-C4 Alkylgruppen, wobei die vorgenannten Gruppen unsubstituiert sind. Beispiele solcher Alkylketten sind Methyl, Ethyl, i-Propyl, n-Propyl, n-Butyl, i-Butyl, t-Butyl, Hexyl und Octyl.

Bei weiteren Ausführungsformen ist es bevorzugt, dass die mindestens eine Vinylsilankomponente ausgewählt wird aus der Gruppe bestehend aus (Vinyl)(trialkoxy)silan,(Vinyl)(methyl)(dialkoxy)silan, (Vinyl)(tris(methoxyethyl))silan, (Vinyl)tris(2-methoxyethoxy)silan, (Vinyl)(triacetoxy)silan, (((Vinylbenzylamino)ethylamino)propyl)(trialkoxy)silan, (Allyl)(,trialkoxy)silan und (Allyl)(triethoxy)silan, wobei alkoxy für methoxy, ethoxy und Mischungen davon steht, vorzugsweise für methoxy. Vorzugsweise wird mindestens ein Vinylsilan, vorzugsweise mindestens 95 Gew.-% der Vinylsilane, bezogen auf das Gesamtgewicht der in dem vorkondensierten Heteropolysiloxan enthaltenen Vinylsilankomponenten, mehr bevorzugtmehr bevorzugt sämtliche Vinylsilane, aus den vorgenannten Beispielen ausgewählt.

Bei weiteren Ausführungsformen ist es bevorzugt, dass das mindestens eine Vinylsilan eine Struktur gemäß Formel (III) aufweist

$$R^{a3}_{x3}R^{b3}_{y3}R^{c3}_{(4-x3-y3)}Si \qquad (III),$$

wobei die $R^{a3}$ unabhängig voneinander ausgewählt werden aus der Gruppe der unsubstituierten C2-C16 Vinylgruppen,

die $R^{b3}$ unabhängig voneinander ausgewählt werden aus der Gruppe der unsubstituierten C1-C16 Alkylgruppen, die $R^{c3}$ unabhängig voneinander ausgewählt werden aus der Gruppe der Alkoxygruppen,

x3 ausgewählt wird aus 1 und 2, und

y3 aus der Gruppe der ganzen Zahlen von 0 bis (2-x3) ausgewählt wird.

Vorzugsweise werden mindestens 95 Gew.-% der Vinylsilane, bezogen auf das Gesamtgewicht der in dem vorkondensierten Heteropolysiloxan enthaltenen Vinylsilankomponenten, mehr bevorzugtmehr bevorzugt sämtliche Vinylsilane, aus den vorgenannten Silanen ausgewählt.

Bei weiteren Ausführungsformen ist es bevorzugt, dass die $R^{a3}$ unabhängig voneinander ausgewählt werden aus der Gruppe der unsubstituierten C2-C7 Vinylgruppen, die $R^{b3}$ unabhängig voneinander ausgewählt werden aus der Gruppe der unsubstituierten C1-C8 Alkylgruppen, und die $R^{c3}$ unabhängig voneinander ausgewählt werden aus der Gruppe der C1-C4 Alkoxygruppen.

Insbesondere ist es bevorzugt, dass die $R^{b3}$ ausgewählt werden aus der Gruppe bestehend aus Methoxy und Ethoxy.

Bei weiteren Ausführungsformen ist es bevorzugt, dass das mindestens ein Arylsilan ausgewählt wird aus der Gruppe bestehend aus (Phenyl)(trialkoxy)silan, (Phenyl)(methyl)(dialkoxy)silan, (Diphenyl)(dialkoxy)silan, (Phenyl)(methyl)(dialkoxy)silan und (Benzyl-2-aminoethyl-3-aminopropyl)(trialkoxy)silan, wobei alkoxy für methoxy, ethoxy und Mischungen davon, vorzugsweise für methoxy, steht.

Bei weiteren Ausführungsformen ist es bevorzugt, dass das mindestens eine Arylsilan eine Struktur gemäß Formel (IV) aufweist

$$R^{a4}{}_{x4}R^{b4}{}_{y4}R^{c4}{}_{(4-x4-y4)}Si \qquad (IV),$$

wobei die $R^{a4}$ unabhängig voneinander ausgewählt werden aus der Gruppe bestehend aus Phenylgruppen, unsubstituierten C7-C12 Alkylarylgruppen und unsubstituierten C7-C12 Arylalkylgruppen,
die $R^{b4}$ unabhängig voneinander ausgewählt werden aus der Gruppe der unsubstituierten C1-C16 Alkylgruppen,
die $R^{c4}$ unabhängig voneinander ausgewählt werden aus der Gruppe der Alkoxygruppen,
x4 ausgewählt wird aus 1 und 2, und
y4 aus der Gruppe der ganzen Zahlen von 0 bis (2-x4) ausgewählt wird.

Vorzugsweise werden mindestens 95 Gew.-% der Arylsilane, bezogen auf das Gesamtgewicht der in dem vorkondensierten Heteropolysiloxan enthaltenen Arylsilankomponenten, mehr bevorzugt sämtliche Arylsilane, aus den vorgenannten Silanen ausgewählt.

Bei weiteren Ausführungsformen ist es bevorzugt, dass die $R^{a4}$ ausgewählt werden aus der Gruppe bestehend aus Phenylgruppen, C7-C10 unsubstituierten Alkylarylgruppen und unsubstituierten C7-C10 Arylalkylgruppen, die $R^{b4}$ ausgewählt werden aus der Gruppe der unsubstituierten C1-C8 Alkylgruppen, und die $R^{c4}$ unabhängig voneinander ausgewählt werden aus der Gruppe der C1-C4 Alkoxygruppen.

Insbesondere ist es bevorzugt, dass die $R^{c4}$ ausgewählt werden aus der Gruppe bestehend aus Methoxy und Ethoxy.

Beispiele von Acrylsilanen sind (Methacryloxymethyl)(methyl)(dialkoxy)silan, (Methacryloxymethyl)(trialkoxy)silan, (3-Methacryloxypropyl)(trialkoxy)silan, (3-Methacryloxyisobutyl)(trialkoxy)silan, (3-Methacryloxypropyl)(methyl)(dialkoxy)silan, (1-Methacryloxymethyl)(trialkoxy)silan, (3-Acryloxypropyl)(trialkoxy)silan und (Acryloxymethyl)(trialkoxy)silan, wobei alkoxy für methoxy oder ethoxy steht. Ein besonders bevorzugtes Beispiel ist (Methacryloxypropyl)(trimethoxy)silan (MEMO).

Bei weiteren Ausführungsformen ist es bevorzugt, dass mindestens eine Acrylsilan eine Struktur gemäß Formel (V) aufweist

$$R^{a5}{}_{x5}R^{b5}{}_{y5}R^{c5}{}_{(4-x5-y5)}Si \qquad (V),$$

wobei die $R^{a5}$ ausgewählt werden aus der Gruppe bestehend aus unsubstituierten C3-C10 Acrylgruppen und unsubstituierten ((C3-C7-Acryloxy)C1-C5-alkyl)trialkoxy,
die $R^{b5}$ ausgewählt werden aus der Gruppe der unsubstituierten C1-C16 Alkylgruppen, die $R^{c5}$ ausgewählt werden aus der Gruppe der Alkoxygruppen,
x5 ausgewählt wird aus 1 und 2, und
y5 aus der Gruppe der ganzen Zahlen von 0 bis (2-x5) ausgewählt wird.

Vorzugsweise werden mindestens 95 Gew.-% der Acrylsilane, bezogen auf das Gesamtgewicht der in dem vorkondensierten Heteropolysiloxan enthaltenen Acrylsilankomponenten, mehr bevorzugt sämtliche Acrylsilane, aus den vorgenannten Silanen ausgewählt.

Bei weiteren Ausführungsformen werden die $R^{a5}$ ausgewählt aus der Gruppe der unsubstituierten C3-C7 Acrylgruppen, die $R^{b5}$ werden ausgewählt aus der Gruppe der unsubstituierten C1-C8 Alkylgruppen, und die $R^{c5}$ werden ausgewählt aus der Gruppe der unsubstituierten C1-C4 Alkoxygruppen.

Insbesondere ist es bevorzugt, dass die $R^{c5}$ ausgewählt werden aus der Gruppe bestehend aus Methoxy und Ethoxy.

Beispiele von Epoxysilanen sind 3-Glycidoxypropyltrialkoxysilan, 3-Glycidoxypropyltrialkoxysilan, Glycidoxypropyl-methyldialkoxysilan und (Beta-(3,4-Epoxycyclohexyl)ethyl)(trialkoxy)silan, wobei alkoxy für methoxy, ethoxy oder propoxy steht.

Es zeigte sich, dass die Verwendung größerer Mengen an Epoxysilankomponenten, beispielsweise in typischerweise genutzten Nasslacksystemen wie Melaminsystemen, nachteilig ist. Hingegen erwies es sich in beispielsweise Pulverlacksystemen nicht als merklich nachteilig oder gar als vorteilhaft. Insbesondere im Zusammenhang mit ferromagnetischen Pigmenten ist der Einsatz eines vorkondensierten Heteropolysiloxans umfassend merkliche Mengen an Epoxysilanen typischerweise nicht problematisch oder sogar wünschenswert. Bei weiteren Ausführungsformen ist es jedoch bevorzugt, dass das vorkondensierte Heteropolysiloxan weniger als 10 Gew.-%, mehr bevorzugtmehr bevorzugt weniger als 3 Gew.-%, noch mehr bevorzugt weniger als 1 Gew.-% an Epoxysilankomponenten umfasst. Letzteres gilt insbesondere dann, wenn ferromagnetische Pigmente, welche primär für den Einsatz in Pulverlacksystemen gedacht sind, für ein breiteres Anwendungsfeld bereitgestellt werden sollen.

Bei weiteren Ausführungsformen ist es bevorzugt, dass das vorkondensierte Heteropolysiloxan mindestens ein weiteres Monomer umfasst, das ausgewählt wird aus der Gruppe bestehend aus Tetramethoxysilan, Tetraethoxysilan, Tetrapropoxysilan und Mischungen davon.

Bei weiteren Ausführungsformen umfasst das vorkondensierte Heteropolysiloxan vorzugsweise mindestens ein Silan gemäß Formel (VI)

$$R^{a6}_4Si \qquad (VI),$$

wobei die $R^{a6}$ unabhängig voneinander ausgewählt werden aus der Gruppe der Alkoxygruppen. Vorzugsweise werden die $R^a$ unabhängig voneinander ausgewählt aus der Gruppe der C1-C4 Alkoxygruppen.

Bei weiteren Ausführungsformen ist es bevorzugt, dass mindestens 32 Gew.-% der Monomerkomponenten, vorzugsweise mindestens 36 Gew.-% der Monomerkomponenten, noch mehr bevorzugt mindestens 41 Gew.-% der Monomerkomponenten, die das vorkondensierte Heteropolysiloxan aufbauen, aus den Aminosilanen ausgewählt werden, jeweils bezogen auf das Gesamtgewicht des vorkondensierten Heteropolysiloxans.

Zudem ist es bei weiteren Ausführungsformen bevorzugt, dass der Anteil an Aminosilankomponenten im vorkondensierten Heteropolysiloxan höchstens 95 Gew.-%, mehr bevorzugt höchstens 89 Gew.-%, noch mehr bevorzugt höchstens 86 Gew.-%, beträgt, jeweils bezogen auf das Gesamtgewicht des vorkondensierten Heteropolysiloxans.

Insbesondere ist es bei weiteren Ausführungsformen bevorzugt, dass der Anteil an Aminosilankomponenten im vorkondensierten Heteropolysiloxan im Bereich von 32 Gw.-% bis 95 Gew.-%, mehr bevorzugt im Bereich von 36 Gew.-% bis 89 Gew.-%, noch mehr bevorzugt im Bereich von 41 Gew.-% bis 86 Gew.-%, liegt, jeweils bezogen auf das Gesamtgewicht des vorkondensierten Heteropolysiloxans.

Bei weiteren Ausführungsformen ist es bevorzugt, dass mindestens 12 Gew.-% der Monomerkomponenten, mehr bevorzugt mindestens 17 Gew.-% der Monomerkomponenten, noch mehr bevorzugt mindestens 23 Gew.-% der Monomerkomponenten des vorkondensierten Heteropolysiloxans aus den Alkylsilanen ausgewählt werden, jeweils bezogen auf das Gesamtgewicht des vorkondensierten Heteropolysiloxans.

Ferner ist es bei weiteren Ausführungsformen bevorzugt, dass höchstens 76 Gew.-% der Monomerkompontens, mehr bevorzugt höchstens 72 Gew.-% der Monomerkomponenten, noch mehr bevorzugt höchstens 69 Gew.-% der Monomerkomponenten des vorkondensierten Heteropolysiloxans aus Alkylsilanen ausgewählt werden, jeweils bezogen auf das Gesamtgewicht des vorkondensierten Heteropolysiloxans.

Insbesondere ist es bei weiteren Ausführungsformen bevorzugt, dass der Anteil an Alkylsilankomponenten im vorkondensierten Heteropolysiloxan im Bereich von 12 Gew.-% bis 76 Gew.-%, mehr bevorzugt im Bereich von 17 Gew.-% bis 72 Gew.-%, noch mehr bevorzugt im Bereich von 23 Gew.-% bis 69 Gew.-%, liegt, jeweils bezogen auf das Gesamtgewicht des vorkondensierten Heteropolysiloxans.

Bei weiteren Ausführungsformen ist es bevorzugt, dass das vorkondensierte Heteropolysiloxan zu mindestens 87 Gew.-%, mehr bevorzugt zu mindestens 93 Gew.-%, noch mehr bevorzugt zu mindestens 97 Gew.-% aus Monomerkomponenten besteht, die ausgewählt werden aus Aminosilanen und Alkylsilanen.

Bei weiteren bevorzugten Ausführungsformen beziehen sich die vorgenannten Mengenangaben bezüglich der Alkylsilankomponenten und Aminosilankomponenten auf die spezifischen Gruppen an Alkylsilanen und Aminosilanen, wie voranstehend aufgeführt. Dies gilt beispielsweise, wenn mindestens 95 Gew.-% der Alkylsilane eine Struktur gemäß Formel (II) aufweisen

$$R^{a2}_{x2}R^{b2}_{(4-x2)}Si \qquad (II),$$

wobei die $R^{a2}$ unabhängig voneinander ausgewählt werden aus der Gruppe der unsubstituierten C1-C16 Alkylgruppen,
die $R^{b2}$ unabhängig voneinander ausgewählt werden aus der Gruppe der Alkoxygruppen und
x2 aus 1 und 2 ausgewählt wird. Vorzugsweise werden mindestens 95 Gew.-% der Alkylsilane, bezogen auf das Gesamtgewicht der in dem vorkondensierten Heteropolysiloxan enthaltenen Alkylsilankomponenten, mehr bevorzugt sämtliche Alkylsilane, aus den vorgenannten Silanen ausgewählt.

Die mit dem vorkondensierten Heteropolysiloxan beschichteten Pigmente zeigen überraschenderweise eine verbesserte Orientierung in Nasslacken, wodurch bessere Helligkeitsflops erzielt werden.

Das gegebenenfalls eingesetzte vorkondensierte Heteropolysiloxan umfasst mindestens eine Aminosilankomponente und mindestens eine Silankomponente, die ausgewählt wird aus der Gruppe bestehend aus Alkylsilanen, Vinylsilanen und Arylsilanen. Die Kondensation entsprechender Monomere, Oligomere und/oder Polymere zur Herstellung der eingesetzten vorkondensierten Heteropolysiloxane erfolgt mithilfe dem Fachmann bekannter Methoden, wie sie beispielsweise in US 5808125 A, US 5679147 A und US 5629400 A offenbart sind. Ferner sind verschiedene vorkondensierte Heteropolysiloxane auch käuflich erhältlich. Bevorzugte vorkondensierte Heteropolysiloxane sind beispielsweise von der Firma Evonik Industries AG, 45128 Essen, Deutschland, unter den Handelsnamen Dynasylan Hydrosil 2627, Dynasylan Hydrosil 2776, Dynasylan Hydrosil 2909, Dynasylan 1146 und Dynasylan Hydrosil 2907 erhältlich. Besonders bevorzugte wasserbasierende vorkondensierte Heteropolysiloxane sind Dynasylan Hydrosil 2627, Dynasylan Hydrosil 2776, Dynasylan Hydrosil 2907 und Dynasylan Hydrosil 2909.

Gemäß einer bevorzugten Variante der Erfindung wird das vorkondensierte Heteropolysiloxan aus der Gruppe, die aus Dynasylan Hydrosil 2627, Dynasylan Hydrosil 2776, Dynasylan Hydrosil 2909, Dynasylan 1146, Dynasylan Hydrosil 2907 und Mischungen davon besteht, ausgewählt.

Vorzugsweise wird das vorkondensierte Heteropolysiloxan in der Form einer wässrigen Formulierung eingesetzt. Insbesondere weisen Formulierungen vorzugsweise eine Konzentration an vorkondensiertem Heteropolysiloxan im Bereich von 5 bis 50 Gew.-% auf, bezogen auf das Gesamtgewicht der Formulierung.

Die Aufbringung des vorkondensierten Heteropolysiloxans kann mithilfe dem Fachmann bekannter Verfahren erfolgen. Ein bevorzugtes Verfahren, da insbesondere mit sehr geringem Aufwand und hervorragenden Ergebnissen verbunden, ist das Einwirken lassen einer wässrigen Lösung des vorkondensierten Heteropolysiloxans auf das zu beschichtende Pigment. Hierbei sollte die Einwirkzeit zwischen 15 Minuten und 240 Minuten liegen.

Bei weiteren Ausführungsformen ist es insbesondere bevorzugt, dass der Aufbringung der Heteropolysiloxanschicht kein Schritt der Härtung, insbesondere thermische Härtung, der Siloxanschicht folgt.

Bei weiteren Ausführungsformen weist das vorkondensierte Heteropolysiloxan weniger als 0,5 Gew.-% an Alkoholen auf, die während der Herstellung des vorkondensierten Heteropolysiloxans infolge Hydrolyse und/oder Kondensation freigesetzt werden.

Überraschenderweise zeigte sich ferner, dass vorzugsweise das vorkondensierte Heteropolysiloxan im Wesentlichen vollständig, vorzugsweise vollständig hydrolysiert ist. Trotz der verringerten Möglichkeit einer weiteren Reaktion oder Vernetzung erzielen derartige vorkondensierte Heteropolysiloxane überwiegend bessere Resultate.

Vorzugsweise weisen die erfindungsgemäß eingesetzten vorkondensierten Heteropolysiloxane einen Gehalt an leichtflüchtigen organischen Lösungsmitteln von weniger als 0,3 Gew.-% auf, bezogen auf das Gesamtgewicht des vorkondensierten Heteropolysiloxans.

Vorzugsweise wird das vorkondensierte Heteropolysiloxan in Form einer wässrigen Formulierung eingesetzt. Typischerweise ist es bei den meisten Anwendungen bevorzugt, dass die wässrige Formulierung umfassend das vorkondensierte Heteropolysiloxan möglichst geringe Mengen an VOCs (volatile organic compounds = flüchtige organische Verbindungen) wie des normalerweise bei der Umsetzung der Silanmonomere entstehenden Alkohols enthält. Bei weiteren Ausführungsformen ist es bevorzugt, dass die wässrigen Formulierungen weniger als 5 Gew.-%, vorzugsweise weniger als 1 Gew.-%, mehr bevorzugt weniger als 0,5 Gew.-%, noch mehr bevorzugt weniger als 0,1 Gew.-%, an VOCs enthält, jeweils bezogen auf das Gewicht der wässrigen Formulierung. Idealerweise werden wässrige Formulierungen eingesetzt, bei denen keine VOCs enthalten sind.

Bei weiteren Ausführungsformen wird das Metalloxid der Beschichtungsschicht, auf die das vorkondensierte Heteropolysiloxan aufgebracht wird im Wesentlichen, vorzugsweise vollständig, ausgewählt aus der Gruppe bestehend aus Siliciumoxid, Aluminiumoxid, Boroxid, Zirkoniumoxid, Ceroxid, Eisenoxid, Titanoxid, Chromoxid, Zinnoxid, Molybdänoxid, deren Oxidhydraten, deren Hydroxiden und Mischungen davon. Besonders bevorzugt werden die erfindungsgemäßen Metalloxide aus der Gruppe bestehend aus Siliciumoxid, Aluminiumoxid und Mischungen davon, ausgewählt, wobei der Begriff Metalloxid auch Oxidhydrate und Hydroxide umfasst. Insbesondere hat sich die Verwendung von Siliciumoxiden wie Siliciumdioxid, Siliciumhydroxid und/oder Siliciumoxidhydrat als vorteilhaft erwiesen.

Besonders hochwertige Eigenschaften, beispielsweise hinsichtlich der Stabilität, werden durch die Kombination der anorganisch/organischen Hybridschicht mit dem vorkondensierten Heteropolysiloxan erzielt. Bei weiteren Ausführungsformen ist es bevorzugt, dass das vorkondensierte Heteropolysiloxan auf die erfindungsgemäße anorganisch/organische Hybridschicht aufgebracht wird. Insbesondere ist es bevorzugt, dass die vorgenannte anorganisch/organische Hybridschicht Siliciumoxid, Siliciumhydroxid, Siliciumoxidhydrat oder deren Mischungen umfasst. Vorzugsweise besteht das in der anorganisch/organischen Hybridschicht enthaltene Metalloxid im Wesentlichen aus Siliciumoxid, Siliciumhydroxid, Siliciumoxidhydrat oder deren Mischungen.

Ferner erwies es sich als besonders vorteilhaft, wenn das vorkondensierte Heteropolysiloxan als äußerste Schicht des erfindungsgemäßen Pigments aufgebracht wurde. So zeigten Pigmente, bei denen das vorkondensierte Heteropolysiloxan Kontakt zum umgebenden Medium hatte eine besonders große Verträglichkeit in einer Vielzahl von Beschichtungsmitteln. Vorzugsweise wird das vorkondensierte Heteropolysiloxan hierbei als äußerste Schicht auf eine erfindungsgemäße anorganisch/organische Hybridschicht aufgebracht.

Gemäß weiteren Varianten der vorliegenden Erfindung ist es ferner bevorzugt die Beschichtung als Ganzes zu optimieren, um verbesserte Ergebnisse beispielsweise für spezifische Einsatzzwecke bereitzustellen.

Bei weiteren Ausführungsformen weist die Beschichtung der erfindungsgemäßen Pigmente, insbesondere der Metalleffektpigmente, vorzugsweise eine mittlere Dicke in einem Bereich von 20 nm bis 160 nm, mehr bevorzugt in einem Bereich von 25 nm bis 135 nm, auf, bestimmt unter Einsatz von Rasterelektronenmikroskopie.

Der Schichtdicken der Beschichtung, sowie der einzelnen Beschichtungsschichten werden beispielsweise mittels REM-Aufnahmen an geeigneten Querschliffen ermittelt. Hierbei werden die Pigmente in einem Lack appliziert und dieser ausgehärtet. Hierbei ist auf eine möglichst gute Orientierung der Plättchen im Anwendungsmedium zu achten. Anschließend wird der ausgehärtete Lack angeschliffen und nach üblicher Probenpräparation der Querschliff im REM betrachtet. Für die Zählung werden nur Teilchen ausgewählt, die eine gute planparallele Orientierung aufweisen. Schlecht orientierte Plättchen ergeben bei dieser Methode einen hohen Fehler aufgrund des unbekannten Betrachtungswinkels. Die Beschichtungen weisen einen sehr guten Kontrast zum Metallkern auf. Sollte man die Schichtdicken der Metalloxid- und der Kunststoffschicht nicht gut unterscheiden können, so kann man örtlich aufgelöste EDX-Analysen verwenden, bevor die Schichtdicken vermessen werden. Der Begriff "mittlere Schichtdicke" im Sinne der Erfindung bezeichnet das arithmetische Mittel der Schichtdicken der Schichten von mindestens 30 Metallpigmenten, vorzugsweise 40 Metallpigmenten. Sofern die Beschichtung ungleichmäßig ist, so wird das arithmetische Mittel der dünnsten und der dicksten Stelle der Beschichtung des jeweiligen Partikels gebildet. Einzelne gravierende Abweichungen, die beispielsweise auf dem Einschluss bereits beschichteter Feinteilpigmente in die Beschichtung beruhen, werden nicht bei der Bestimmung der mittleren Schichtdicke berücksichtigt.

Bei weiteren Ausführungsformen ist es bevorzugt, dass die Dicke der anorganisch/organischen Hybridschicht mindestens 40 %, mehr bevorzugt mindestens 60 %, noch mehr bevorzugt mindestens 85 %, der Gesamtdicke der Beschichtung beträgt. Die Bestimmung der Gesamtdicke der Beschichtung erfolgt vorzugsweise mittels REM, wobei die gesamte Beschichtung über dem metallischen Substrat betrachtet wird. Eventuell leicht anhaftende Nachbeschichtungen oder Bestandteile beispielsweise einer Lackzusammensetzung, in der die Pigmente formuliert waren, werden naturgemäß nicht miteinbezogen. Derartige Reste werden vor der Messung durch Waschen der Pigmente in dem Fachmann bekannten Lösungsmitteln entfernt.

Die erfindungsgemäßen Pigmente können als Trockenpräparat, Paste oder Suspension vorliegen. Trockenpräparate im Sinne der vorliegenden Erfindung sind beispielsweise Pulver und Granulate. Derartige Formen zeigten beispielsweise den Vorteil, das keine zusätzlichen Lösungsmittel transportiert und gelagert werden müssen, welche gleichzeitig eine Unverträglichkeit in späteren Anwendungen hervorrufen können. Pasten und Suspensionen hingegen zeigen zwar entsprechende Nachteile, können jedoch einfacher gehandhabt werden, da beispielsweise die Staubbildung bei der Entnahme und beim Umfüllen reduziert oder verhindert wird.

Ferner betrifft die vorliegende Erfindung ein Verfahren zur Herstellung beschichteter Pigmente, wobei das Verfahren die folgenden Schritte umfasst:

i) Umsetzen mindestens eines Metalloxidedukts, mindestens einem Edukt eines organischen Polymers und mindestens eines Netzwerkbildners in einer Flüssigphase unter Ausbildung einer Beschichtungszusammensetzung,
ii) Aufbringen der Beschichtungszusammensetzung auf metallische Substrate unter Ausbildung einer anorganisch/organischen Hybridschicht,

wobei die anorganisch/organische Hybridschicht mindestens ein anorganisches Netzwerk umfassend mindestens ein Metalloxid und mindestens ein organisches Polymer aufweisen und das anorganische Netzwerk und das organische Polymer kovalent miteinander verbunden sind,
wobei das mindestens eine Metalloxid ausgewählt wird aus Oxiden, Hydroxiden und Oxidhydraten der Metalle und Halbmetalle und kein Oxidationsprodukt des metallischen Substrats darstellt,
der Netzwerkbildner zumindest teilweise mit dem Metalloxid und dem organischen Polymer verbunden ist und
das Verhältnis der Menge an Metalloxid der Beschichtung zur spezifischen Oberfläche des unbeschichteten Metallpigments in einem Bereich von 16,1 mg/m$^2$ bis 25 mg/m$^2$ liegt, und
wobei das Verhältnis der Menge organischem Polymer der anorganisch/organischen Hybridschicht zur spezifischen Oberfläche des unbeschichteten Metallpigments in einem Bereich von 3,9 mg/m$^2$ bis 10,1 mg/m$^2$. Die vorgenannte Metalloxidmenge der Beschichtung bezieht sich auf die Beschichtung ohne gegebenenfalls aufgebrachte Nachbeschichtungen. Vorzugsweise handelt es sich bei den metallischen Substraten um plättchenförmige metallische Substrate. Die Schritte i) und ii) können hierbei zeitlich getrennt, gleichzeitig oder teilweise gleichzeitig ablaufen.

Naturgemäß muss die Umsetzung der Edukte der anorganisch/organischen Hybridschicht zumindest teilweise gleichzeitig erfolgen, um die erfindungsgemäße Hybridschicht auszubilden. Hierbei können der Netzwerkbildner, das Edukt des Metalloxids und das Edukt des organischen Polymers in beliebiger Reihenfolge miteinander vermischt werden. Es muss jedoch gewährleistet werden, dass die kovalenten Bindungen zwischen den einzelnen Bestandteilen ausgebildet werden können. Vorzugsweise werden die Edukte zunächst miteinander vermischt, bevor die Umsetzung beispielsweise durch Zugabe einer Säure, Base oder eines spezifischen Katalysators gestartet wird.

Die metallischen Substrate können hierbei bereits vorgelegt worden sein oder erst während oder nach der Reaktion der Beschichtungsbestandteile zugegeben werden. Hierbei können die Beschichtungsbestandteile auch über einen definierten Zeitraum in veränderlicher Menge zugegeben werden, um bei bestimmten Ausführungsformen einen größeren Anteil von Metalloxid nahe der Oberfläche des metallischen Substrats und/oder nahe der Oberfläche der anorganisch/organischen Hybridschicht zu erhalten. Ferner liegt bei weiteren Ausführungsformen ein erhöhter Anteil an organischem Polymer nahe der Oberfläche des metallischen Substrats und/oder nahe der Oberfläche der anorganisch/organischen Hybridschicht vor. Erfindungsgemäß wird nicht unter einer anorganisch/organischen Mischschicht verstanden, dass lediglich eine kovalente Verbindung zwischen diskreten Schichten aus Metalloxid und organischen Polymer erzeugt wird. Beispielsweise durch Abscheidung von Metalloxid als Beschichtung auf dem metallischen Substrat in einem ersten Schritt, gefolgt von der Zugabe von Netzwerkbildner und dem Edukt des organischen Polymers oder umgekehrt.

Bei weiteren Ausführungsformen ist es bevorzugt, dass das Gewichtsverhältnis der Metalloxidmenge der Beschichtung zur organischen Polymermenge der anorganisch/organischen Hybridschicht der beschichteten Metallpigmente in einem Bereich von 2,5 : 1 bis 9,0 : 1, mehr bevorzugt in einem Bereich von 3,0 : 1 bis 8,1 : 1, noch mehr bevorzugt in einem Bereich von 3,3 : 1 bis 6,7 : 1, liegt.

Ferner ist es gemäß weiteren Ausführungsformen bevorzugt, dass das Verhältnis der Stoffmengen in Mol von Netzwerkbildner zu organischem Polymer der anorganische/organischen Hybridschicht der beschichteten Metallpigmente im Bereich von 1:10 bis 5:10, mehr bevorzugt im Bereich von 1,2:10 bis 4:10, noch mehr bevorzugt im Bereich von 1,4:10 bis 3,5:10, liegt.

Bei weiteren Ausführungsformen wird die anorganisch/organische Hybridschicht auf das unbeschichtete metallische Substrat aufgebracht. Hierbei wird unter unbeschichtet verstanden, dass das metallische Substrat keine umhüllende Beschichtung aufweist, sondern beispielsweise lediglich Reste von Vermahlungshilfsmitteln bei plättchenförmigen metallischen Substraten oder Additiven zur Verringerung oder Verhinderung von Agglomeration.

Ferner hat sich die Verwendung von vorkondensierten Heteropolysiloxanen bei den erfindungsgemäßen Pigmenten als vorteilhaft erwiesen, wobei diese auf eine metalloxidhaltige Beschichtungsschicht aufgebracht werden. Vorzugsweise erfolgt die Zugabe der vorkondensierten Heteropolysiloxane nach dem Aufbau mindestens einer anorganisch/organischen Hybridschicht.

Insbesondere ist es bei weiteren Ausführungsformen bevorzugt, dass die Zugabe der vorkondensierten Heteropolysiloxane dem Schritt des Aufbaus der anorganisch/organischen Hybridschicht folgt. Hierbei ist es vorteilhaft, wenn die Umsetzung der Edukte der anorganisch/organischen Mischschicht zumindest weitgehend beendet, bevor das vorkondensierte Heteropolysiloxan aufgebracht wird. Unter der "weitgehend beendeten" Herstellung der anorganisch/organischen Beschichtungsschicht wird im Sinne der vorliegenden Erfindung verstanden, dass höchstens 30 Gew.-% der Edukte der Beschichtung noch reaktiv sind. Vorzugsweise sind weniger als 15 Gew.-%, mehr bevorzugt weniger als 5 Gew.-% der Edukte noch reaktiv. Die Bestimmung der Menge erfolgt anhand der noch in Lösung befindlichen, reaktiven Eduktmengen. Dies erwies sich überraschenderweise als äußerst vorteilhaft, da hiermit ein sehr dünner Beschichtungsaufbau realisiert werden kann. Hierbei wurde keine merkliche Verminderung der vorteilhaften Eigenschaften hinsichtlich beispielsweise der Stabilität beobachtet werden, verglichen beispielsweise mit einem Aufbau mit dazwischen liegender Metalloxidschicht. Gleichzeitig resultiert ein derartiger Aufbau in verbesserten optischen Eigenschaften.

Bei weiteren Ausführungsformen liegt die Reaktionstemperatur bei den Schritten i) und/oder ii) vorzugsweise in einem Bereich von 0°C bis 180°C, mehr bevorzugt von 40°C bis 120°C, noch mehr bevorzugt in einem Bereich von 60°C bis 100°C.

Ferner ist es bei weiteren Ausführungsformen bevorzugt, dass die Umsetzung der Edukte der anorganisch/organischen Hybridschicht entweder

a) zunächst bei einem pH-Wert von höchstens 6,8 und später bei einem pH-Wert von mindestens 7,2 oder
b) zunächst bei einem pH-Wert von mindestens 7,2 und später bei einem pH-Wert von höchstens 6,8 durchgeführt wird.

So zeigte überraschenderweise, dass der Wechsel des pH-Wertes während der Reaktion der Bestandteile der anorganisch/organischen Beschichtung beispielsweise in einer verbesserten Stabilität der Beschichtung resultierte.

Bei weiteren Ausführungsformen ist es ferner bevorzugt, dass der pH-Wert bei a) zunächst oder bei b) später im Bereich von bei 2,5 bis 6,8, mehr bevorzugt bei a) zunächst oder bei b) später im Bereich von 3,5 bis 6,5, noch mehr bevorzugt im Bereich von a) zunächst oder bei b) später bei 4 bis 6, liegt.

Ferner ist es bei weiteren Ausführungsformen bevorzugt, dass der pH-Wert bei a) später oder bei b) zunächst im Bereich von bei 7,2 bis 11,5, mehr bevorzugt bei a) später oder bei b) zunächst im Bereich von 7,5 bis 11, noch mehr bevorzugt bei a) später oder bei b) zunächst im Bereich von 8 bis 10,2, liegt.

Die hierbei eingesetzten Säuren und Basen dienen insbesondere als Katalysator für die Hydrolyse und Kondensation des Metalloxids.

Es wurde ferner beobachtet, dass sich die Verwendung spezifischer Säuren zur Einstellung des pH-Wertes als besonders vorteilhaft erwies. Bei weiteren Ausführungsformen werden Säuren eingesetzt, die ausgewählt werden aus der Gruppe der organischen Säuren, vorzugsweise aus der Gruppe bestehend aus Ameisensäure, Essigsäure, Propionsäure, Oxalsäure, Malonsäure, Maleinsäure, Bernsteinsäure, Anhydriden der genannten Säuren und Mischungen hiervon, mehr bevorzugt aus der Gruppe bestehend aus Ameisensäure, Essigsäure, Oxalsäure und Mischungen hiervon. Hiermit konnten überraschenderweise besonders homogene Beschichtungen aufgebaut werden.

Sofern die Herstellung der Beschichtungen hingegen möglichst kostengünstig erfolgen soll, haben sich anorganische Säuren, insbesondere Mineralsäuren wie Salzsäure, Schwefelsäure, Salpetersäure, Phosphorsäure und Mischungen hiervon, als vorteilhaft erwiesen.

Ferner wurde beobachtet, dass ein verbesserter Schichtaufbau typischerweise durch Einsatz einer Base erzielt werden kann, die ausgewählt wird aus der Gruppe bestehend aus organischen Basen, Ammoniak, Hydrazin und Mischungen hiervon erzielt werden kann. Vorzugsweise werden die organischen Basen ausgewählt aus Aminen wie primären, sekundären und tertiären Aminen. Beispiele sind Dimethylethanolamin (DMEA), Monoethanolamin, Diethanolamin, Triethanolamin, Ethylendiamin (EDA), t-Butylamin, Monomethylamin, Dimethylamin, Trimethylamin, Monoethylamin, Diethylamin, Triethylamin, Pyridin, Pyridinderivate, Anilin, Anilinderivat, Cholin, Cholinderivate, Harnstoff, Harnstoffderivate, Hydrazinderivate und Mischungen davon.

Bei weiteren Ausführungsformen ist es hingegen bevorzugt, dass mindestens eine Base eingesetzt wird, die ausgewählt wird aus den anorganischen Basen, vorzugsweise aus anorganischen Basen die ausgewählt werden aus der Gruppe bestehend aus Ammoniak, Hydrazin, Natriumhydroxid, Kaliumhydroxid, Ammoniumcarbonat, Ammoniumhydrogencarbonat, Natriumcarbonat, Natriumhydrogencarbonat, Kaliumcarbonat, Kaliumhydrogencarbonat und Mischungen davon besteht.

Insbesondere ist es bei weiteren Ausführungsformen bevorzugt, dass die Umsetzung der Edukte der anorganisch/organischen Hybridschicht zunächst bei einem pH-Wert weniger als 7 und später bei einem pH-Wert von mehr als 7 erfolgt.

Bei weiteren Ausführungsformen wird zunächst die Umsetzung der Edukte der anorganisch/organischen Hybridschicht gestartet, bevor die metallischen Substrate zugegeben werden. Dies erwies in einigen Fällen als überraschenderweise als vorteilhaft, da hierbei die trotz der bereits teilweise abgeschlossenen Reaktion der Edukte der anorganisch/organischen Hybridschicht noch immer sehr zuverlässige Beschichtungen erhalten werden konnten. So ist diese Verfahrensvariante besonders geeignet zur Beschichtung empfindlicher metallischer Substrate und zur Umsetzung reaktionsträger Edukte der anorganisch/organischen Hybridschicht, da die metallischen Substrate nicht von Beginn der Umsetzung der Edukte der anorganisch/organischen Hybridschicht vorhanden sein müssen.

Hingegen erwies sich für die meisten Fälle als vorteilhaft die Umsetzung der Edukte der anorganisch/organischen Hybridschicht in Gegenwart der metallischen Substrate durchzuführen. Insbesondere scheinen hiermit noch gleichmäßigere und stabilere Beschichtungen erhalten zu werden.

Bei weiteren Ausführungsformen umfasst das Verfahren die Aufbringung von mindestens einer weiteren Beschichtungsschicht umfassend mindestens ein Metalloxid.

Bei weiteren Ausführungsformen umfasst das Verfahren die Aufbringung mindestens einer weiteren Beschichtungsschicht, die im Wesentlichen aus mindestens einem organischen Polymer besteht. Die betreffende Schicht ist vorzugsweise zwischen der anorganisch/organischen Hybridschicht und dem metallischen Substrat angeordnet.

Ferner ist es bei weiteren Ausführungsformen bevorzugt, dass im Wesentlichen Monomere für das mindestens eine organische Polymer eingesetzt werden, die mit mindestens einer Funktionalität ausgestattet waren, die ausgewählt wird aus der Gruppe bestehend aus Aminogruppen, Thiolgruppen, Epoxygruppen, Acrylatgruppen, Methacrylatgruppen, Vinylgruppen, Allylgruppen, Alkenylgruppen, Alkinylgruppen, Carboxygruppen, Carboxylanhydridgruppen, Isocyanatgruppen, Cyanatgruppen, Ureidogruppen und Carbamatgruppen, mehr bevorzugt aus der Gruppe bestehend aus Acrylatgruppen, Methacrylatgruppen, Hydroxygruppen, Carboxygruppen und Carboxylanhydridgruppen, noch mehr bevorzugt aus der Gruppe bestehend aus Acrylatgruppen und Methacrylatgruppen. Vorzugsweise werden mindestens 95 Gew.-%, mehr bevorzugt mindestens 99 Gew.-%, der im organischen Polymer enthaltenen Monomerbestandteile aus solchen Monomeren ausgewählt, jeweils bezogen auf das Gesamtgewicht der Monomerbestandteile.

Bei weiteren Ausführungsformen werden die spezifizierten Beschichtungsschichten in einer Ein-Topf-Reaktion aufgebracht.

Bei weiteren Ausführungsformen wird mindestens ein Tetraalkoxysilan oder das Oligomer von mindestens einem Tetraalkoxysilan bei der Herstellung der anorganisch/organischen Hybridschicht eingesetzt. Die Alkoxygruppen des Tetraalkoxysilans können unabhängig voneinander ausgewählt werden, beispielsweise aus der Gruppe der C1-C4 Alkoxygruppen. Jedoch stellen Tetraalkoxygruppen mit mindestens 3, vorzugsweise mindestens 4, identischen Alkoxygruppen besonders bevorzugte Tetraalkoxysilane dar. Vorzugsweise werden die Tetraalkoxysilane ausgewählt aus der Gruppe bestehend aus Tetraethoxysilan und Oligomeren von Tetraethoxysilan.

Bei weiteren Ausführungsformen wird ein organofunktionelles Silan bei der Herstellung mindestens einer anorganisch/organischer Hybridschicht eingesetzt.

Bei weiteren Ausführungsformen wird als Flüssigphase ein Lösungsmittel ausgewählt aus der Gruppe bestehend aus Wasser, Alkoholen, Glykolethern, Ketonen, Acetatestern, Testbenzin und Mischungen davon.

Gemäß einer weiteren Variante betrifft die vorliegende Erfindung ein beschichtetes Pigment, insbesondere ein beschichtetes Metalleffektpigment mit Metallkern, welche gemäß dem erfindungsgemäßen Verfahren oder einer seiner Ausführungsformen hergestellt wurde. Insbesondere ist es hierbei bevorzugt, dass es gemäß einem der Verfahren wie in den Ansprüchen 14 bis 16 und den Aspekten 23 bis 32 beschrieben hergestellt wurde.

Ferner betrifft die Erfindung Beschichtungsmittel, welche erfindungsgemäß beschichtete Pigmente enthalten. Insbesondere solche, wie sie in den Ansprüchen 1 bis 13 oder den Aspekten 1 bis 22 spezifiziert werden oder die mittels des Verfahrens gemäß einem der Ansprüche 14 bis 16 oder einem der Aspekte 23 bis 32 hergestellt wurden.

Bei weiteren Ausführungsformen wird das Beschichtungsmittel ausgewählt aus der Gruppe bestehend aus Wasserlacken, lösemittelhaltigen Lacken und Pulverlacken. Ein besonders bevorzugtes Beschichtungsmittel für die erfindungsgemäß beschichteten ferromagnetischen Pigmente sind Pulverlacke.

Ferner betrifft die Erfindung einen Gegenstand der Metallpigmente mit Beschichtung, auch als beschichtete Metallpigmente bezeichnet, gemäß einem der Ansprüche 1 bis 13 oder Aspekte 1 bis 22 aufweist.

Ferner betrifft die vorliegende Erfindung die Verwendung der erfindungsgemäßen Pigmente in einem Kosmetikum, einem Kunststoff oder einem Beschichtungsmittel. Beispiele der Beschichtungsmittel sind Lacke, wie Nasslacke und Pulverlacke, und Farben, wie Druckfarben und Bautenanstrichen.

Insbesondere können die erfindungsgemäßen Pigmente in Beschichtungsmittel eingearbeitet werden, welche beispielsweise ausgewählt werden aus der Gruppe bestehend aus Nasslacken wie wasserbasierenden Lacken und lösemittelhaltigen Lacken, Pulverlacken, Coil Coating Formulierungen und Druckfarben. Ferner sind die erfindungsgemäßen Pigmente hervorragend geeignet zur Verwendung in Kunststoffen oder Kosmetika. Eine besonders bevorzugte Gruppe von Beschichtungsmitteln stellen Farben wie Druckfarben und Tinten, Nasslacke und Pulverlacke dar. Ein besonderes Anwendungsfeld der Druckfarben und Tinten ist das Gebiet der Sicherheitsdruckfarben.

Ferner betrifft die vorliegende Erfindung die Verwendung der erfindungsgemäßen Pigmente in wasserbasierenden sowie lösemittelbasierenden Lacken. Derartige Lacke werden beispielsweise in Automobilsektor und Industriesektor eingesetzt. Bei der Anwendung in wasserbasierenden Systemen kann das erfindungsgemäße Pigment in Wasser oder üblichen Colösern, wie zum Beispiel Butylglycol, angeschlämmt werden. Zudem kann das erfindungsgemäße Pigment auch direkt in das wasserbasierende Anwendungsmedium eingearbeitet werden. Analoges gilt für den Einsatz in lösemittelbasierenden Lacken. Die erfindungsgemäßen Pigmente zeichnen sich hierbei durch ein hervorragendes Dispergierverhalten aus.

Des Weiteren betrifft die Erfindung die Verwendung der erfindungsgemäßen Pigmente in Pulverlacken. Pulverlacke werden beispielsweise in der industriellen Serienfertigung zur Beschichtung von elektrisch leitfähigen und temperaturbeständigen Werkstoffen verwendet. Der zu applizierende Pulverlack liegt hierbei als festes und lösungsmittelfreies Pulver vor. Ferner sind die als Grundierung oder Einschichtdecklack eingesetzten Pulverlacke fast vollständig recyclebar. Die umweltfreundlichen und vielseitig einsetzbaren Pulverlacke enthalten Bindemittel, Pigmente, Füllstoffe und Vernetzer sowie optional Additive. Unter einem Bindemittel wird die dem Fachmann geläufige Definition verstanden. D.h. das Bindemittel umfasst sowohl den Filmbildner wie auch nicht flüchtige Hilfsstoffe wie beispielsweise Weichmacher, Füllstoffe und/oder Härter. Eine Auftragung der feinpulvrigen Pulverlacke erfolgt in der Regel elektrostatisch, bevor sie durch Einbrennen oder durch Strahlungsenergie gehärtet werden.

Zur Pigmentierung der Pulverlacke können unter anderem plättchenförmige Pigmente eingesetzt werden. Bei mittels Mischverfahren hergestellten Pulverlacken kann es sich jedoch als problematisch erweisen, dass durch die beim Extrusions- und Vermahlungsprozess auf die Pigmentplättchen einwirkenden Scherkräfte eine Beschädigung oder Zerstörung der Pigmentplättchen auftreten kann. Hierdurch kann insbesondere der Glanz und somit auch die Optik derart pigmentierter Anwendungen negativ beeinträchtigt werden.

Aus diesem Grund werden beispielsweise im Dry-blend-Verfahren die Metalleffektpigmente dem Basispulverlack erst nach der Vermahlung zugemischt. Dies hat jedoch den Nachteil, dass während der Lackapplikation eine mögliche Separation von Pigment und Pulverlack durch das unterschiedliche Aufladeverhalten der einzelnen Lackbestandteile auftritt. Hieraus folgt ein unregelmäßiger optischer Effekt in Folge einer Ab- oder Anreicherung von Pigment im Pulverlack während der Pulverlackapplikation. Ferner führt die Separierung von Pigment und Bindemittel zu einer veränderten Zusammensetzung des "Oversprays", d.h. des Anteils an Pulverlack, der an dem zu beschichtenden Gegenstand vorbeigesprüht wird und aus Kostengründen recycelt werden sollte. Alternativ wird das sogenannte Bonding-Verfahren eingesetzt, bei dem das Pigment unter Erwärmen an den Partikeln des Basislackes fixiert wird. Die Herstellung derartiger Bonding-Pulverlacke ist jedoch verhältnismäßig kostenaufwendig. Die Herstellung der zurzeit kostengünstigsten Pulverlacke erfolgt mittels Mischverfahren. Hierbei werden die Pigmente zusammen mit allen anderen Rohstoffen vermischt, extrudiert und vermahlen.

Ferner können die erfindungsgemäßen Pigmente im Coil-Coating-Verfahren eingesetzt werden. Dieses Verfahren zeichnet sich durch seine große Umweltfreundlichkeit aus. Beschichtung und Trocknung finden hierbei kontinuierlich in einem geschlossenen System statt, wobei im no-rinse Verfahren zudem das Abspülen von Chemikalienresten entfällt. Ferner kann durch eine optimierte Prozessführung ein Auftragswirkungsgrad von nahezu 100 % erreicht werden, während ansonsten bei den meisten Lackierverfahren beispielsweise größere Verluste durch das over spraying vorhanden sind. Da jedoch der Lack beim Coil-Coating bei Temperaturen von 240 bis 280°C eingebrannt wird, können für dieses Verfahren nur besonders stabile Pigmente eingesetzt werden.

Ferner können die erfindungsgemäßen Pigmente in Druckfarben eingesetzt werden. Beispiele solcher Druckfarben sind Tiefdruck-, Sieb oder Flexodruckfarben. Die erfindungsgemäßen Pigmente sind auch besonders geeignet für wasserbasierende Lacke (Wasserlacke) und Außenanwendungen.

Tief-, Flexo- oder Siebdruckfarben enthalten Lösemittel bzw. Lösemittelgemische. Diese dienen unter Anderem zum Lösen der Bindemittel, aber auch zur Einstellung wichtiger Anwendungseigenschaften der Druckfarben, wie beispielsweise der Viskosität oder der Trocknungsgeschwindigkeit. Typischerweise werden niedrig siedende Lösemittel eingesetzt, während höher siedende in kleineren Mengen zur Einstellung der Trocknungsgeschwindigkeit dienen.

Neben Lösemittel können verschiedene weitere Bestandteile in einer Farbe enthalten sein, wie beispielsweise Reaktivverdünner und Fotoinitiatoren bei strahlungshärtbaren Druckfarben; Bindemittel wie beispielsweise Nitrocellulose, Ethylcellulose, Hydroxyethylcellulose, Acrylate, Polyvinylbutyrale, aliphatische und aromatische Polyurethane und Polyharnstoffe; Füllstoffe wie Calciumcarbonat, Aluminiumoxidhydrat, Aluminiumsilikat und Magnesiumsilikat, Wachse wie Polyethylenwachse, oxidierte Polyethylenwachse, Petroleumwachse und Ceresinwachse; Fettsäureamide, Weichmacher; Dispergierhilfsmittel, Fettsäuren und Antiabsetzmittel.

Ferner betrifft die vorliegende Erfindung die Verwendung der erfindungsgemäßen Pigmente in Polymeren. Neben dem zusätzlichen Schutz infolge der erfindungsgemäßen Beschichtung lassen sich die Pigmente gut in die Polymere, beispielsweise thermoplastische Polymere, einarbeiten und dispergieren.

Beispiele thermoplastischer Polymere sind Polyoxyalkylene, Polycarbonate (PC), Polyester wie Polybutylenterephthalat (PBT) oder Polyethylenterephthalat (PET), Polyolefine wie Polyethylen (PE) oder Polypropylen (PP), Poly(meth)acrylate, Polyamide, vinylaromatische (Co)polymere wie Polystyrol, schlagzähmodifiziertes Polystyrol wie HIPS, oder ASA-, ABS- oder AES-Polymerisate, Polyarylenether wie Polyphenylenether (PPE), Polysulfone, Polyurethane, Polylactide, halogenhaltige Polymerisate, imidgruppenhaltige Polymere, Celluloseester, Silicon-Polymere oder thermoplastische Elastomere. Es können auch Mischungen unterschiedlicher Thermoplaste eingesetzt werden.

Bei weiteren Ausführungsformen wird das Kosmetikum ausgewählt aus der Gruppe bestehend aus Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semi-permanente Haarfarben, temporäre Haarfarben, Hautpflegekompositionen wie Lotions, Gele, Emulsionen und Nagellackkompositionen.

Ferner betrifft die vorliegende Erfindung die Verwendung der erfindungsgemäßen anorganisch/organischen Hybridschicht zur Erzeugung von Schutzschichten auf Gegenständen mit metallischer Oberfläche, insbesondere Pigmenten.

Zudem betrifft die vorliegende Erfindung die Verwendung eines vorkondensierten Heteropolysiloxans wie vorgenannt spezifiziert zur Beschichtung von metalloxidhaltigen Oberflächen.

Gemäß einem Aspekt 1 betrifft die vorliegende Erfindung ein Pigment umfassend ein metallisches Substrat und mindestens eine anorganisch/organische Hybridschicht, wobei die anorganisch/organische Hybridschicht mindestens ein Metalloxid, mindestens einen Netzwerkbildner und mindestens ein organisches Polymer umfasst, wobei das mindestens eine Metalloxid kein Oxidationsprodukt des metallischen Substrats darstellt und der Begriff Metalloxid Oxide, Hydroxide und Oxidhydrate der Metalle und Halbmetalle umfasst, der Netzwerkbildner mindestens teilweise kovalent mit dem Metalloxid und dem organischen Polymer verbunden ist, und wobei das Verhältnis der Menge an Metalloxid der Beschichtung zur spezifischen Oberfläche des unbeschichteten Metallpigments in einem Bereich von 16,1 mg/m$^2$ bis 25 mg/m$^2$, vorzugsweise in einem Bereich von 17,2 mg/m$^2$ bis 23 mg/m$^2$, mehr bevorzugt in einem Bereich von 17,9 mg/m$^2$ bis 22,3 mg/m$^2$, liegt und das Verhältnis der Menge organischem Polymer der anorganisch/organischen Hybridschicht zur spezifischen Oberfläche des unbeschichteten Metallpigments in einem Bereich von 3,9 mg/m$^2$ bis 10,1 mg/m$^2$, vorzugsweise in einem Bereich von 4,6 mg/m$^2$ bis 9,7 mg/m$^2$, mehr bevorzugt in einem Bereich von 5,1 mg/m$^2$ bis 9,5 mg/m$^2$, liegt.

Gemäß einem Aspekt 2 betrifft die vorliegende Erfindung ein Pigment gemäß Aspekt 1, wobei das metallische Substrat plättchenförmig ist.

Gemäß einem Aspekt 3 betrifft die vorliegende Erfindung ein Pigment gemäß einem der Aspekte 1 bis 2, wobei das Gewichtsverhältnis der Metalloxidmenge der Beschichtung zur organischen Polymermenge der anorganisch/organischen Hybridschicht in einem Bereich von 2,5 : 1 bis 9,0 : 1, mehr bevorzugt in einem Bereich von 3,0 : 1 bis 8,1 : 1, noch mehr bevorzugt in einem Bereich von 3,3 : 1 bis 6,7 : 1, liegt.

Gemäß einem Aspekt 4 betrifft die vorliegende Erfindung ein Pigment gemäß einem der Aspekte 1 bis 3, wobei das Verhältnis der Stoffmengen in Mol von Netzwerkbildner zu organischem Polymer der anorganische/organischen Hybridschicht im Bereich von 1:10 bis 5:10, mehr bevorzugt im Bereich von 1,2:10 bis 4:10, noch mehr bevorzugt im Bereich von 1,4:10 bis 3,5:10, liegt.

Gemäß einem Aspekt 5 betrifft die vorliegende Erfindung ein Pigment gemäß einem der Aspekte 1 bis 4, wobei das organische Polymer kein Polyethylen ist.

Gemäß einem Aspekt 6 betrifft die vorliegende Erfindung ein Pigment gemäß einem der Aspekte 1 bis 5, wobei die Dicke der anorganisch/organischen Hybridschicht mindestens 40 %, mehr bevorzugt mindestens 60 %, noch mehr bevorzugt mindestens 85 %, der Gesamtdicke der Beschichtung beträgt.

Gemäß einem Aspekt 7 betrifft die vorliegende Erfindung ein Pigment gemäß einem der Aspekte 1 bis 6, wobei die Menge der anorganisch/organischen Hybridschicht Gemäß einem Aspekt 13 betrifft die vorliegende Erfindung ein Pigment gemäß einem der Aspekte 1 bis 12, wobei mindestens 87 Gew-%, vorzugsweise mindestens 93 Gew-%, mehr bevorzugt mindestens 97 Gew-%, der Silanmonomerkomponenten des vorkondensierten Heteropolysiloxans ausgewählt werden aus der Gruppe bestehend aus Aminosilanen, Alkylsilanen und Mischungen davon, jeweils bezogen auf das Gesamtgewicht des vorkondensierten Heteropolysiloxans.

Gemäß einem Aspekt 14 betrifft die vorliegende Erfindung ein Pigment gemäß einem der Aspekte 1 bis 13, wobei das Metall des metallischen Substrats des beschichteten Metallpigments gemäß einem der Aspekte 1 bis 25 weitgehend aus einem Metall besteht, das ausgewählt wird aus der Gruppe bestehend aus Aluminium, Kupfer, Eisen, Zink, Zinn, Titan, Chrom, Kobalt, Silber, Edelstahl, Nickel, Antimon, Magnesium, Zirkonium, Silicium, Bor, Mischungen hiervon und Legierungen hiervon.

Gemäß einem Aspekt 15 betrifft die vorliegende Erfindung ein Pigment gemäß einem der Aspekte 1 bis 14, wobei die mindestens eine Aminosilankomponente des vorkondensierten Heteropolysiloxans zu mindestens 92 Gew.-%, bezogen auf das Gesamtgewicht der in dem vorkondensierten Heteropolysiloxan enthaltenen Aminosilankomponenten, vorzugsweise vollständig, ausgewählt wird aus der Gruppe bestehend aus $(H_2N(CH_2)_3)Si(OCH_3)_3$ ((3-Aminopropyl)(trimethoxy)silan, AMMO), $(H_2N(CH_2)_3Si(OC_2H_5)_3$ ((3-Aminopropyl)(triethoxy)silan, AMEO), $(H_2N(CH_2)_2)NH(CH_2)_3)Si(OCH_3)_3$ ((N-(2-Aminoethyl)-3-aminopropyl)(trimethoxy)silan, (DAMO)), (N-(2-Aminoethyl)-3-aminopropyl)(triethoxy)silan und Mischungen hiervon.

Gemäß einem Aspekt 16 betrifft die vorliegende Erfindung ein Pigment gemäß einem der Aspekte 1 bis 15, wobei das mindestens eine Aminosilan des vorkondensierten Heteropolysiloxans ausgewählt wird aus der Gruppe der Aminosilane gemäß Formel (I):

$$R^{a1}{}_{x1}R^{b1}{}_{y1}R^{c1}{}_{(4-x1-y1)}Si \qquad (I),$$

wobei die $R^{a1}$ unabhängig voneinander ausgewählt werden aus mit mindestens einer Stickstoffgruppe substituierten funktionellen Gruppen, wobei die funktionelle Gruppe ausgewählt wird aus der Gruppe bestehend aus C1-C16 Alkylgruppen, C2-C8 Alkenylgruppen, C2-C8 Alkinylgruppen und Phenylgruppen, C7-C12 Alkylarylgruppen und C7-C12 Arylalkylgruppen,
die $R^{b1}$ unabhängig voneinander ausgewählt werden aus der Gruppe bestehend aus C1-C16 Alkylgruppen, C2-C8 Alkenylgruppen, C2-C8 Alkinylgruppen, Phenylgruppen, C7-C12 Arylalkylgruppen und C7-C12 Alkylarylgruppen, wobei die vorgenannten mindestens 5 Gew.-%, mehr bevorzugt mindestens 6 Gew.-%, noch mehr bevorzugt mindestens 8 Gew.-%, beträgt, jeweils bezogen auf das Gesamtgewicht des Pigments.

Gemäß einem Aspekt 8 betrifft die vorliegende Erfindung ein Pigment gemäß einem der Aspekte 1 bis 7, wobei die Menge der anorganisch/organischen Hybridschicht höchstens 25 Gew.-%, mehr bevorzugt höchstens 21 Gew.-%, noch mehr bevorzugt höchstens 18 Gew.-%, beträgt, bezogen auf das Gesamtgewicht des Pigments.

Gemäß einem Aspekt 9 betrifft die vorliegende Erfindung ein Pigment gemäß einem der Aspekte 1 bis 8, wobei das mindestens eine Metalloxid der anorganisch/organischen Hybridschicht ausgewählt wird aus der Gruppe bestehend aus Siliciumoxid, Aluminiumoxid, Boroxid, Zirkoniumoxid, Ceroxid, Eisenoxid, Titanoxid, Chromoxid, Zinnoxid, Molybdänoxid, Vanadiumoxid, Zinkoxid, Magnesiumoxid und Mischungen davon, mehr bevorzugt aus der Gruppe bestehend aus Siliciumoxid, Aluminiumoxid, Eisenoxid und Mischungen davon, noch mehr bevorzugt aus Siliciumoxid, Aluminiumoxid und Mischungen davon, wobei der Begriff Metalloxid auch Oxidhydrate und Hydroxide umfasst.

Gemäß einem Aspekt 10 betrifft die vorliegende Erfindung ein Pigment gemäß einem der Aspekte 1 bis 9, wobei auf mindestens einer anorganisch/organischen Hybridschicht mindestens ein vorkondensiertes Heteropolysiloxan aufgebracht wurde, wobei das mindestens eine vorkondensierte Heteropolysiloxan mindestens ein Aminosilan und mindestens ein Silan, das ausgewählt wird aus der Gruppe bestehend aus Alkylsilanen, Vinylsilanen und Arylsilanen, umfasst und das Heteropolysiloxan in vorkondensierter Form aufgebracht wurde.

Gemäß einem Aspekt 11 betrifft die vorliegende Erfindung ein Pigment gemäß einem der Aspekte 1 bis 10, wobei das vorkondensierte Heteropolysiloxan als äußerste Schicht auf eine umhüllende anorganisch/organische Hybridschicht aufgebracht.

Gemäß einem Aspekt 12 betrifft die vorliegende Erfindung ein Pigment gemäß einem der Aspekte 1 bis 11, wobei der Gehalt an Metalloxid in der Beschichtung in einem Bereich von 50 Gew.-% bis 86 Gew.-%, vorzugsweise in einem Bereich von 56 Gew.-%, bis 82 Gew.-%, noch mehr bevorzugt in einem Bereich von 61 Gew.-%, bis 79 Gew.-%, liegt, jeweils bezogen auf das Gesamtgewicht der Beschichtung.

Gruppen unsubstituiert sind,
die $R^{c1}$ unabhängig voneinander ausgewählt werden aus der Gruppe der Alkoxygruppen,
x1 1, 2 oder 3 ist, und
y1 aus der Gruppe der ganzen Zahlen von 0 bis (3-x1) ausgewählt wird.

Gemäß einem Aspekt 17 betrifft die vorliegende Erfindung ein Pigment gemäß einem der Aspekte 1 bis 16, wobei mindestens 95 Gew.-% des Metalls des metallischen Substrats ausgewählt werden aus der Gruppe bestehend aus Aluminium, Eisen, Kupfer und Messing, bezogen auf das Gewicht des Metalls des metallischen Substrats ohne Sauerstoff.

Gemäß einem Aspekt 18 betrifft die vorliegende Erfindung ein Pigment gemäß einem der Aspekte 1 bis 17, wobei die Pigmente ein Aspektverhältnis ($D_{50}/h_{50}$) in einem Bereich von 1500:1 bis 10:1, vorzugsweise von 1200:1 bis 15:1, mehr bevorzugt von 950:1 bis 25:1, auf.

Gemäß einem Aspekt 19 betrifft die vorliegende Erfindung ein Pigment gemäß einem der Aspekte 1 bis 18, wobei mindestens ein Netzwerkbildner ausgewählt wird aus den Verbindungen gemäß Formel (NI) oder (NII),

$$R^{an1}_{xn1}R^{bni}_{yn1}SiX_{(4-xn1-yn1)} \qquad (NI)$$

$$(R^{an1}O)_{xn2}(R^{bn1}O)_{yn2}MX_{(zn2-xn2-yn2)} \qquad (N\ II),$$

wobei die X unabhängig voneinander ausgewählt werden aus hydrolysierbaren Gruppen, nach deren Hydrolyse eine kovalente Bindung von organischem Netzwerkbildner mit dem anorganischen Netzwerk ausgebildet werden kann,
die $R^{an1}$ unabhängig voneinander ausgewählt werden aus reaktiven organischen Gruppen, die mit dem organischen Polymer kovalent verbindbar sind,
die $R^{bn1}$ unabhängig voneinander ausgewählt werden aus organischen Gruppen, die mit dem organischen Polymer kovalent verbindbar sein können,
M ausgewählt wird aus der Gruppe bestehend aus Al, Zr und Ti,
xn1 eine ganze Zahl von 1 bis 3 ist, yn1 eine ganze Zahl von 0 bis (3-xn1) ist,
zn2 die formale Oxidationszahl von M ist, xn2 eine ganze Zahl von 1 bis (zn2-1) ist,
yn2 eine ganze Zahl von 0 bis (zn2-2) ist, und
xn2+yn2 ≤ zn2-1 ist.

Gemäß einem Aspekt 20 betrifft die vorliegende Erfindung ein Pigment gemäß einem der Aspekte 1 bis 19, wobei mindestens ein Netzwerkbildner ausgewählt wird aus den Verbindungen gemäß Formel (NI) oder (NII),

$$R^{an1}_{xn1}\ R^{bn1}_{yn1}SiX_{(4-xnl-yn1)} \qquad (NI)$$

$$(R^{an1}O)_{xn2}(R^{bn1}O)_{yn2}MX_{(zn2-xn2-yn2)} \qquad (NII)$$

wobei die X unabhängig voneinander ausgewählt werden aus der Gruppe bestehend aus Halogengruppen, der Hydroxygruppe und C1-C4 Alkoxygruppen ohne Heteroatome in der Kohlenstoffkette,
die $R^{an1}$ unabhängig voneinander ausgewählt werden aus der Gruppe bestehend aus C1-C26 Alkylgruppen, C2-C26 Alkenylgruppen, C2-C26 Alkinylgruppen, C6-C30-Arylgruppen, C7-C31 Alkylarylgruppen, C7-C31 Arylalkylgruppen, C8-C32 Alkenylarylgruppen, C5-C20 Cycloalkylgruppen, C6-C21 Alkylcycloalkylgruppen und C6-C21 Cycloalkylalkylgruppen, wobei gegebenenfalls vorhandene Substituenten ausgewählt werden aus der Gruppe bestehend aus Aminogruppen, Hydroxygruppe, Thiolgruppen, Epoxygruppen, Acrylatgruppen, Methacrylatgruppen, Vinylgruppen, Allylgruppen, Carboxygruppen, Carboxylanhydridgruppen, Isocyanatgruppen, Cyanatgruppen, Ureidogruppen, Carbamatgruppen und Mischungen hiervon,
die $R^{bn1}$ unabhängig voneinander ausgewählt werden aus der Gruppe bestehend aus C1-C24 Alkylgruppen, C2-C24 Alkenylgruppen, C2-C24 Alkinylgruppen, C6-C24 Arylgruppen, fluorierte C6-C24 Arylgruppen, teilfluorierte C6-C24 Arylgruppen, C7-C30 Alkylarylgruppen, C7-C30 Arylalkylgruppen, C8-C30 Alkenylarylgruppen, C8-C30 Arylalkenylgruppen, C8-C30 Arylalkinylgruppen, C8-C30 Alkinylarylgruppen, C5-C20 Cycloalkylgruppen und C6-C25 Alkylcycloalkylgruppen, noch mehr bevorzugt aus der Gruppe bestehend aus C1-C24 Alkylgruppen, C2-C24 Alkenylgruppen, C6-C18 Arylgruppen, C7-C24 Alkylarylgruppen, C7-C24 Arylalkylgruppen, C5-C16 Cycloalkylgruppen und C6-C20 Alkylcycloalkylgruppen, wobei die vorgenannten Gruppen unsubstituiert sind und in den Kohlenstoffketten und Kohlenstoffringsystemen gegebenenfalls enthaltene Heteroatome ausgewählt werden aus der Gruppe bestehend aus O, N und S,

M ausgewählt wird aus der Gruppe bestehend aus Al, Zr und Ti,

xn1 eine ganze Zahl von 1 bis 3 ist, yn1 eine ganze Zahl von 0 bis (3-xn1) ist,

zn2 die formale Oxidationszahl von M ist, xn2 eine ganze Zahl von 1 bis (zn2-1) ist,

yn2 eine ganze Zahl von 0 bis (zn2-2) ist, und

xn2+yn2 ≤ zn2-1 ist.

Gemäß einem Aspekt 21 betrifft die vorliegende Erfindung ein Pigment gemäß einem der Aspekte 1 bis 20, wobei die anorganisch/organische Hybridschicht mindestens ein Netzwerkbildner gemäß Formel (NI) umfasst.

Gemäß einem Aspekt 22 betrifft die vorliegende Erfindung ein Pigment gemäß einem der Aspekte 1 bis 21, wobei das organische Polymer ausgewählt wird aus der Gruppe bestehend aus Polyacrylaten, Polymethacrylaten, Polyethern, Polyestern, Polyaminen, Polyamiden, Polyolen, Polyurethanen und Polyolefinen, wobei die Polyolefine kein Polyethylen umfassen, mehr bevorzugt aus der Gruppe bestehend aus Polyacrylaten, Polymethacrylaten, Polyethern und Polyestern, noch mehr bevorzugt aus der Gruppe bestehend aus Polyacrylaten und Polymethacrylaten.

Gemäß einem Aspekt 23 betrifft die vorliegende Erfindung ein Verfahren zur Herstellung von Metallpigmenten mit metallischem Substrat und Beschichtung, umfassend folgende Schritte:

i) Umsetzen mindestens eines Metalloxidedukts, mindestens einem Edukt eines organischen Polymers und mindestens eines Netzwerkbildners in einer Flüssigphase unter Ausbildung einer Beschichtungszusammensetzung,

ii) Aufbringen der Beschichtungszusammensetzung auf metallische Substrate unter Ausbildung einer anorganisch/organischen Hybridschicht,

wobei die anorganisch/organische Hybridschicht mindestens ein anorganisches Netzwerk umfassend mindestens ein Metalloxid und mindestens ein organisches Polymer aufweisen und das anorganische Netzwerk und das organische Polymer kovalent miteinander verbunden sind,

wobei das mindestens eine Metalloxid kein Oxidationsprodukt des metallischen Substrats darstellt und der Begriff Metalloxid Oxide, Hydroxide und Oxidhydrate der Metalle und Halbmetalle umfasst,

der Netzwerkbildner zumindest teilweise mit dem Metalloxid und dem organischen Polymer verbunden ist und

das Verhältnis der Menge an Metalloxid der Beschichtung zur spezifischen Oberfläche des unbeschichteten Metallpigments in einem Bereich von 16,1 $mg/m^2$ bis 25 $mg/m^2$, vorzugsweise in einem Bereich von 17,2 $mg/m^2$ bis 23 $mg/m^2$, mehr bevorzugt in einem Bereich von 17,9 $mg/m^2$ bis 22,3 $mg/m^2$, liegt und

das Verhältnis der Menge organischem Polymer der anorganisch/organischen Hybridschicht zur spezifischen Oberfläche des unbeschichteten Metallpigments in einem Bereich von 3,9 $mg/m^2$ bis 10,1 $mg/m^2$, vorzugsweise in einem Bereich von 4,6 $mg/m^2$ bis 9,7 $mg/m^2$, mehr bevorzugt in einem Bereich von 5,1 $mg/m^2$ bis 9,5 $mg/m^2$, liegt.

Gemäß einem Aspekt 24 betrifft die vorliegende Erfindung ein Verfahren zur Herstellung von Metallpigmenten mit metallischem Substrat und Beschichtung gemäß Aspekt 23, wobei das metallische Substrat plättchenförmig ist.

Gemäß einem Aspekt 25 betrifft die vorliegende Erfindung ein Verfahren zur Herstellung von Metallpigmenten mit metallischem Substrat und Beschichtung gemäß einem der Aspekte 23 bis 24, wobei das Gewichtsverhältnis der Metalloxidmenge der Beschichtung zur organischen Polymermenge der anorganisch/organischen Hybridschicht der beschichteten Metallpigmente in einem Bereich von 2,5 : 1 bis 9,0 : 1, mehr bevorzugt in einem Bereich von 3,0 : 1 bis 8,1 : 1, noch mehr bevorzugt in einem Bereich von 3,3 : 1 bis 6,7 : 1, liegt.

Gemäß einem Aspekt 26 betrifft die vorliegende Erfindung ein Verfahren zur Herstellung von Metallpigmenten mit metallischem Substrat und Beschichtung gemäß einem der Aspekte 23 bis 25, wobei das Verhältnis der Stoffmengen in Mol von Netzwerkbildner zu organischem Polymer der anorganische/organischen Hybridschicht der beschichteten Metallpigmente im Bereich von 1:10 bis 5:10, mehr bevorzugt im Bereich von 1,2:10 bis 4:10, noch mehr bevorzugt im Bereich von 1,4:10 bis 3,5:10, liegt.

Gemäß einem Aspekt 27 betrifft die vorliegende Erfindung ein Verfahren zur Herstellung von Metallpigmenten mit metallischem Substrat und Beschichtung gemäß einem der Aspekte 23 bis 26, wobei die Menge der anorganisch/organischen Hybridschicht mindestens 5 Gew.-%, mehr bevorzugt mindestens 6 gew.-%, noch mehr bevorzugt mindestens 8 Gew.-%, beträgt, jeweils bezogen auf das Gesamtgewicht des Pigments.

Gemäß einem Aspekt 28 betrifft die vorliegende Erfindung ein Verfahren zur Herstellung von Metallpigmenten mit metallischem Substrat und Beschichtung gemäß einem der Aspekte 23 bis 27, wobei der Gehalt an Metalloxid in der Beschichtung in einem Bereich von 50 Gew.-% bis 86 Gew.-%, vorzugsweise in einem Bereich von 56 Gew.-%, bis 82 Gew.-%, noch mehr bevorzugt in einem Bereich von 61 Gew.-%, bis 79 Gew.-%, liegt, jeweils bezogen auf das Gesamtgewicht der Beschichtung.

Gemäß einem Aspekt 29 betrifft die vorliegende Erfindung ein Verfahren zur Herstellung von Metallpigmenten mit metallischem Substrat und Beschichtung gemäß einem der Aspekte 23 bis 28, wobei auf mindestens eine anorganisch/organische Hybridschicht mindestens einer Beschichtungsschicht umfassend mindestens ein vorkondensiertes Heteropolysiloxan aufgebracht wird,

wobei der Begriff Metalloxid auch Oxidhydrate und Hydroxide umfasst und das mindestens eine vorkondensierte Heteropolysiloxan mindestens ein Aminosilan und mindestens ein Silan umfasst, dass ausgewählt wird aus der Gruppe der Alkylsilane, Vinylsilane und Arylsilane.

Gemäß einem Aspekt 30 betrifft die vorliegende Erfindung ein Verfahren zur Herstellung von Metallpigmenten mit metallischem Substrat und Beschichtung gemäß einem der Aspekte 23 bis 29, wobei die Umsetzung der Edukte der anorganisch/organischen Hybridschicht entweder

a) zunächst bei einem pH-Wert von höchstens 6,8 und später bei einem pH-Wert von mindestens 7,2 oder
b) zunächst bei einem pH-Wert von mindestens 7,2 und später bei einem pH-Wert von höchstens 6,8 durchgeführt wird.

Gemäß einem Aspekt 31 betrifft die vorliegende Erfindung ein Verfahren zur Herstellung von Metallpigmenten mit metallischem Substrat und Beschichtung gemäß einem der Aspekte 23 bis 30, wobei die Umsetzung der Edukte der anorganisch/organischen Hybridschicht entweder

a) zunächst in einem pH-Wertbereich von 2,5 bis 6,8 und später in einem pH-Wertbereich von 7,2 bis 11,5, mehr bevorzugt zunächst in einem pH-Wertbereich von 3,5 bis 6,5 und später in einem pH-Wertbereich von 7,5 bis 11, noch mehr bevorzugt zunächst in einem pH-Wertbereich von 4 bis 6 und später in einem pH-Wertbereich von 8 bis 10,2 oder
b) zunächst in einem pH-Wertbereich von 7,2 bis 11,5 und später in einem pH-Wertbereich von 2,5 bis 6,8, mehr bevorzugt zunächst in einem pH-Wertbereich von 7,5 bis 11 und später in einem pH-Wertbereich von 3,5 bis 6,5, noch mehr bevorzugt zunächst in einem pH-Wertbereich von 8 bis 10,2 und später in einem pH-Wertbereich von 4 bis 6,

durchgeführt wird.

Gemäß einem Aspekt 32 betrifft die vorliegende Erfindung ein Verfahren zur Herstellung von Metallpigmenten mit metallischem Substrat und Beschichtung gemäß einem der Aspekte 23 bis 31, wobei die Umsetzung der Edukte der anorganisch/organischen Hybridschicht zunächst bei einem pH-Wert von höchstens 6,8, mehr bevorzugt zunächst bei einem pH-Wert von höchstens 6,5, noch mehr bevorzugt zunächst bei einem pH-Wert von höchstens 6, durchgeführt wird.

Gemäß einem Aspekt 33 betrifft die vorliegende Erfindung ein Pigment hergestellt mittels eines Verfahrens gemäß einem der Aspekte 23 bis 32.

Gemäß einem Aspekt 34 betrifft die vorliegende Erfindung die Verwendung der Pigmente gemäß einem der Aspekte 1 bis 22 in einem Kosmetikum, einem Kunststoff oder einem Beschichtungsmittel.

Gemäß einem Aspekt 35 betrifft die vorliegende Erfindung die Verwendung gemäß Aspekt 34, wobei das Beschichtungsmittel ausgewählt wird aus der Gruppe bestehend aus Nasslacken, Pulverlacken und Farben wie Druckfarben und Tinten.

Gemäß einem Aspekt 36 betrifft die vorliegende Erfindung ein Beschichtungsmittel beinhaltend ein Pigment gemäß einem der Aspekte 1 bis 22.

Gemäß einem Aspekt 37 betrifft die vorliegende Erfindung einen Gegenstand, wobei der Gegenstand Pigmente gemäß einem der Aspekte 1 bis 22 oder ein Beschichtungsmittel gemäß Aspekt 36 aufweist.

Beispiele

Beispiel 1:

Zu 100 g Aluminiumpigment in Form eines Pulvers oder in Form einer Paste wird Isopropanol gegeben, bis jeweils 250 g Suspension vorliegen. Die Suspension wird auf 70 °C erhitzt, bevor die Bestandteile der anorganisch/organischen Hybridschicht (Tetraethylorthosilikat (TEOS), Dynasylan MEMO, TMPTMA, Azo-bis-(isobutyronitril) (AIBN), sowie gebenenenfalls Allylmethacrylat oder Laurylmethacrylat) und 3 g Essigsäure in 30 g destilliertes Wasser zugegeben werden. Nach 3 Stunden werden 2,5 g Ethylendiamin in 50 g Isopropanol zugegeben. Nach weiteren 2 Stunden werden Dynasylan OCTEO und Dynasylan DAMO zugegeben und für 1 h gerührt, bevor das Reaktionsgemisch abgekühlt wird. Der Feststoff wird abfiltriert und als Paste gesammelt.

| | Pigment | BET [m²/g] | Menge Pigment [g] | TEOS [g] | MEMO [g] | TMPTMA [g] | Laurylmethacrylat [g] | Allylmethacrylat [g] | AIBN [g] | OCTEO [g] | DAMO [g] |
|---|---|---|---|---|---|---|---|---|---|---|---|
| B 1-1 | Mex 2192 | 4,0 | 100,00 | 35,0 | 0,4 | 1,8 | 0,4 | 0,0 | 0,1 | 0,9 | 1,0 |
| B 1-2 | Mex 3580 | 6,1 | 100,00 | 45,0 | 0,6 | 2,4 | 0,5 | 0,0 | 0,2 | 1,3 | 1,6 |
| B 1-3 | Mex 3580 | 6,1 | 100,00 | 40,1 | 0,8 | 3,6 | 0,7 | 0,0 | 0,3 | 1,3 | 0,7 |
| B 1-4 | Metallic 707 | 5,8 | 100,00 | 46,4 | 0,6 | 2,6 | 0,5 | 0,0 | 0,2 | 1,4 | 1,9 |
| B 1-5 | Mex 3580 | 6,1 | 100,00 | 42,1 | 0,5 | 2,2 | 0,8 | 0,0 | 0,2 | 1,3 | 1,6 |
| B 1-6 | Mex 3540 | 2,6 | 100,00 | 23,5 | 0,3 | 1,2 | 0,2 | 0,0 | 0,1 | 0,6 | 0,7 |
| B 1-7 | Mex 9160 | 6,5 | 100,00 | 52,4 | 0,7 | 3,1 | 0,6 | 0,0 | 0,3 | 1,4 | 1,6 |
| B 1-8 | Mex 3580 | 6,1 | 100,00 | 42,2 | 0,5 | 2,2 | 0,4 | 0,0 | 0,2 | 2,5 | 1,1 |
| VB 1-9 | Mex 3580 | 6,1 | 100,00 | 42,2 | 0,0 | 2,2 | 0,4 | 0,0 | 0,2 | 2,5 | 1,1 |
| B 1-10 | Mex 2192 | 4,0 | 100,00 | 45,0 | 0,5 | 2,1 | 0,0 | 0,0 | 0,2 | 1,3 | 1,2 |
| B 1-11 | Mex 3580 | 6,1 | 100,00 | 41,2 | 0,7 | 2,7 | 0,0 | 0,0 | 0,2 | 1,5 | 1,0 |
| B 1-12 | Mex 3540 | 2,6 | 100,00 | 44,1 | 0,7 | 2,6 | 0,0 | 0,0 | 0,1 | 1,0 | 0,9 |
| B 1-13 | Mex 9160 | 6,5 | 100,00 | 35,7 | 0,6 | 3,0 | 0,0 | 0,0 | 0,3 | 0,9 | 1,3 |
| B 1-14 | Mex 2192 | 4,0 | 100,00 | 42,5 | 0,5 | 2,9 | 0,0 | 0,6 | 0,1 | 1,2 | 1,5 |
| B 1-15 | Mex 3580 | 6,1 | 100,00 | 44,2 | 0,4 | 3,1 | 0,0 | 0,7 | 0,2 | 1,2 | 1,7 |
| B 1-16 | Mex 3540 | 2,6 | 100,00 | 41,7 | 0,7 | 2,2 | 0,0 | 0,5 | 0,2 | 1,5 | 1,2 |

B: Beispiel, VB: Vergleichsbeispiel

Beispiel 2:

Das Beispiel 2-1 erfolgte analog der Vorschrift von Beispiel 1.
Das Vergleichsbeispiel 2-2 erfolgte gemäß folgender Vorschrift:
Zu 100 g Aluminiumpigment in Form eines Pulvers oder in Form einer Paste wird Isopropanol gegeben, bis jeweils 250 g Suspension vorliegen. Die Suspension wird auf 70 °C erhitzt, bevor die das Tetraethylorthosilicat (TEOS) und 3 g Essigsäure in 30 g destilliertes Wasser zugegeben werden. Nach 3 Stunden folgen die Zugaben von MEMO, TMPTMA, Laurylmethacrylat und AIBN. Nach weiteren 3 Stunden erfolgt die Zugabe von 2,5 g Ethylendiamin in 50 g Isopropanol. Nach weiteren 2 Stunden werden Dynasylan OCTEO und Dynasylan DAMO zugegeben und für 1 h gerührt, bevor das Reaktionsgemisch abgekühlt wird. Der Feststoff wird abfiltriert und als Paste gesammelt.

| | Pigment | BET [m²/g] | Menge Pigment [g] | TEOS [g] | MEMO [g] | TMPTMA [g] | Laurylmethacrylat [g] | AIBN [g] | OCTEO [g] | DAMO [g] |
|---|---|---|---|---|---|---|---|---|---|---|
| B 2-1 | Mex 2156 | 3,4 | 200,00 | 45,9 | 0,6 | 2,4 | 0,5 | 0,2 | 1,0 | 1,3 |
| VB 2-2 | Mex 2156 | 3,4 | 200,00 | 45,9 | 0,6 | 2,4 | 0,5 | 0,2 | 1,0 | 1,3 |
| B: Beispiel, VB: Vergleichsbeispiel | | | | | | | | | | |

Beispiel 3:

Zu 100 g Aluminiumpigment in Form eines Pulvers oder in Form einer Paste wird Isopropanol gegeben, bis jeweils 250 g Suspension vorliegen. Die Suspension wird auf 70 °C erhitzt, bevor die Bestandteile der anorganisch/organischen Hybridschicht und 3 g Essigsäure in 30 g destilliertes Wasser zugegeben werden. Nach 3 Stunden werden 2,5 g Ethylendiamin in 50 g Isopropanol zugegeben. Nach weiteren 2 Stunden werden Dynasylan OCTEO und Dynasylan DAMO zugegeben und für 1 h gerührt, bevor das Reaktionsgemisch abgekühlt wird. Der Feststoff wird abfiltriert und als Paste gesammelt.

|  | Pigment | BET [m²/g] | Menge Pigment [g] | TEOS [g] | MEMO [g] | TMPTMA [g] | Laurylmethacrylat [g] | AIBN [g] | OCTEO [g] | DAMO [g] |
|---|---|---|---|---|---|---|---|---|---|---|
| VB 3-1 | Mex 2153 | 1,7 | 100,0 | 19,3 | 0,2 | 0,9 | 0,2 | 0,1 | 0,2 | 0,3 |
| VB 3-2 | Mex 2156 | 3,4 | 100,0 | 20,7 | 0,5 | 2,2 | 0,4 | 0,2 | 0,7 | 0,9 |
| B 3-3 | Mex 2156 | 3,4 | 100,0 | 23,1 | 0,6 | 2,7 | 0,5 | 0,2 | 1,0 | 0,4 |
| B 3-4 | Mex 3580 | 6,1 | 100,0 | 50,7 | 0,6 | 2,7 | 0,5 | 0,2 | 1,3 | 1,6 |
| VB 3-5 | Mex 3580 | 6,1 | 100,0 | 32,2 | 0,5 | 2,2 | 0,4 | 0,2 | 1,3 | 1,6 |
| B 3-6 | Mex 3580 | 6,1 | 100,0 | 42,2 | 0,5 | 2,2 | 0,4 | 0,2 | 1,3 | 0,7 |
| B 3-7 | Mex 2192 | 4,0 | 100,0 | 35,0 | 0,4 | 1,8 | 0,4 | 0,1 | 0,5 | 0,5 |
| B 3-8 | Metallic 707 | 5,8 | 100,0 | 38,0 | 1,0 | 4,3 | 0,8 | 0,3 | 1,5 | 0,6 |
| VB 3-9 | Metallux 9157 | 5,2 | 100,0 | 41,1 | 1,1 | 4,6 | 0,9 | 0,4 | 1,6 | 0,6 |
| B 3-10 | Metallux 9157 | 5,2 | 100,0 | 39,3 | 0,6 | 2,3 | 0,5 | 0,2 | 0,7 | 0,8 |
| VB 3-11 | Mex 2156 | 3,4 | 100,0 | 23,1 | 0,6 | 2,7 | 0,5 | 0,2 | 1,0 | 0,4 |
| B 3-12 | Mex 3580 | 6,1 | 100,0 | 53,9 | 0,5 | 2,2 | 0,4 | 0,2 | 1,3 | 0,5 |
| B 3-13 | Metallic 707 | 5,8 | 100,0 | 50,2 | 0,7 | 2,8 | 0,5 | 0,2 | 1,4 | 1,8 |
| B 3-14 | Metallux 9157 | 5,2 | 100,0 | 50,2 | 0,6 | 2,6 | 0,5 | 0,2 | 0,7 | 0,8 |
| VB 3-15 | Mex 2192 | 4,0 | 100,0 | 39,4 | 0,5 | 2,0 | 0,4 | 0,2 | 0,9 | 1,0 |

B: Beispiel, VB: Vergleichsbeispiel

Anwendungsbeispiel 1

Für einen erschwerten Gasungstest wurden 15 g Metallpigmentpaste mit einem Feststoffgehalt von 55 Gew.-% wird in 11,0 g Butylglykol mit einer Rührzeit von 5 min suspendiert. Zu dieser Suspension wurden 14,4 g Bindemittel farblos (ZK26-6826-402, Hersteller BASF Coatings) und 0,6 g 10 %-ige Dimethylethanolaminlösung (Lösungsmittel: Wasser) hinzugegeben und 5 min gerührt.

21,96 g der Suspension wurden in ein Gemisch aus 195,0 g Mischlack für Effektstoffprüfung milchig/farblos (ZW42-6008-0101, Hersteller BASF Coatings), 75,6 g Wasser-Basislack-Tönpaste Rot (ZU560-329-0001, Hersteller BASF Coatings, enthaltend Eisenoxid rot, $Fe_2O_3$) und 6,0 g Wasser-Basislack-Tönpaste Schwarz (ZU42-5943-0001, Hersteller BASF Coatings, enthaltend Eisenoxid schwarz, $Fe_2O_3 * FeO$) eingerührt. Nachfolgend wurde der pH-Wert der Suspension mit 10 %-iger Dimethylethanolaminlösung (Lösungsmittel Wasser) auf 9,0 eingestellt.

265 g der voranstehenden Zusammensetzung wurden in eine Gasungsflasche eingefüllt und diese mit einem Doppelkammergasblasenzähler verschlossen. Die Gaswaschflasche wurde in einem Wasserbad bei 40 °C für 1 Stunde temperiert, gasdicht verschlossen und der Test über maximal 41 Tage durchgeführt. Das entstandene Gasvolumen wurde anhand des verdrängten Wasservolumens in der oberen Kammer des Gasblasenzählers abgelesen. Bei einer Entwicklung von maximal 10 ml Wasserstoff nach 41 Tagen galt der Test als bestanden. Sofern die Probe nicht über den kompletten Zeitraum stabil war, ist die Zeitdauer bis zum Ausgasen der Probe vermerkt.

| | Metalloxid zur Oberfläche [mg/m$^2$] | Organisches Polymer zur Oberfläche [mg/m$^2$] | Ergebnis |
|---|---|---|---|
| B 1-1 | 22,6 | 5,3 | bestanden |
| B 1-2 | 20,3 | 5,0 | bestanden |
| B 1-3 | 17,9 | 7,1 | bestanden |
| B 1-4 | 20,7 | 5,1 | bestanden |
| B 1-5 | 17,7 | 4,8 | bestanden |
| B 1-6 | 23,0 | 5,5 | bestanden |
| B 1-7 | 21,1 | 5,6 | bestanden |
| B 1-8 | 17,7 | 4,2 | bestanden |
| VB 1-9 | 17,6 | 3,9 | 2 Tage |
| B 2-1 | 17,5 | 4,2 | bestanden |
| VB 2-2 | 17,5 | 4,2 | 14 Tage |
| VB 3-1 | 26,8 | 5,4 | 6 |
| VB 3-2 | 12,8 | 6,2 | 2 |
| B 3-4 | 20,2 | 4,8 | bestanden |
| VB 3-5 | 13,9 | 4,3 | 20 |
| B 3-6 | 18,0 | 4,3 | bestanden |
| B 3-7 | 22,9 | 5,4 | bestanden |
| B 3-8 | 17,3 | 8,7 | bestanden |
| B 3-10 | 20,5 | 5,4 | bestanden |
| B 3-12 | 24,1 | 4,5 | bestanden |
| B 3-13 | 22,5 | 5,7 | bestanden |
| B: Beispiel, VB: Vergleichsbeispiel | | | |

Anwendungsbeispiel 2:

Die Pigmentproben wurden in ein typisches Testlacksystem (ZW42-1100, Fa. BASF, 0,35 Gew.-% Metallanteil) eingearbeitet und die Prüfapplikationen durch Spritzlackierung auf einem geprimerten Stahlblech hergestellt. Die Schichtdicke betrug hierbei 6 μm. Der Basislack wurde mit einem handelsüblichen 1 K-Klarlack überlackiert und anschließend eingebrannt. Die Messungen wurden mit einem Gerät der Sorte BYK mac (Fa. Byk-Gardner) durchgeführt.

Der Flopindex ist nach Alman in der einschlägigen Literatur folgendermaßen definiert:

$$\text{Flopindex} = 2{,}69 \cdot (L_{E1} - L_{E3})^{1{,}11} / L_{E2}^{0{,}86}$$

wobei $L_{E1}$ für die Helligkeit des glanznahen Messwinkels (E1 = 15° zum Glanzwinkel), $L_{E2}$ für die Helligkeit des Messwinkels zwischen glanznahem und glanzfernen Winkel (E2 = 45° zum Glanzwinkel) und $L_{E3}$ für die Helligkeit des glanzfernen Messwinkels (E3 = 110° zum Glanzwinkel) steht. Nachfolgend werden die Veränderungen im Flop zwischen dem jeweiligen erfindungsgemäßen Beispiel und den entsprechenden Vergleichsbeispielen aufgeführt.

Die Glanzmessung erfolgte in Anlehnung an die DIN EN ISO 2813.

Je niedriger der Zahlenwert des Flopindexes ist, desto schwächer kommt der gewünschte Hell-/ Dunkelflop zum Ausdruck.

| | Metalloxid zur Oberfläche [mg/m²] | Organisches Polymer zur Oberfläche [mg/m²] | ΔFlop |
|---|---|---|---|
| VB 3-11 | 20,4 | 10,5 | -1,4 |
| B 3-53 | 19,7 | 10,1 | 0 |
| B: Beispiel, VB: Vergleichsbeispiel | | | |

| | Metalloxid zur Oberfläche [mg/m²] | Organisches Polymer zur Oberfläche [mg/m²] | ΔFlop |
|---|---|---|---|
| B 3-7 | 22,9 | 5,4 | 0 |
| VB 3-15 | 37,6 | 5,6 | -0,7 |
| B: Beispiel, VB: Vergleichsbeispiel | | | |

| | Metalloxid zur Oberfläche [mg/m²] | Organisches Polymer zur Oberfläche [mg/m²] | ΔFlop |
|---|---|---|---|
| VB 3-9 | 22,5 | 11,3 | -0,7 |
| B 3-10 | 20,5 | 5,4 | 0 |
| VB 3-14 | 25,6 | 6,1 | -0,3 |
| B: Beispiel, VB: Vergleichsbeispiel | | | |

**Patentansprüche**

1. Pigment umfassend ein metallisches Substrat und mindestens eine anorganisch/organische Hybridschicht,
   wobei die anorganisch/organische Hybridschicht mindestens ein Metalloxid, mindestens einen Netzwerkbildner und mindestens ein organisches Polymer umfasst,
   wobei das mindestens eine Metalloxid kein Oxidationsprodukt des metallischen Substrats darstellt und der Begriff Metalloxid Oxide, Hydroxide und Oxidhydrate der Metalle und Halbmetalle umfasst,
   der Netzwerkbildner mindestens teilweise kovalent mit dem Metalloxid und dem organischen Polymer verbunden ist,
   wobei das Verhältnis der Menge an Metalloxid der anorganisch/organischen Hybridschicht zur spezifischen Oberfläche des unbeschichteten Metallpigments in einem Bereich von 16,1 mg/m² bis 25 mg/m² liegt und
   das Verhältnis der Menge organischem Polymer der anorganisch/organischen Hybridschicht zur spezifischen Oberfläche des unbeschichteten Metallpigments in einem Bereich von 3,9 mg/m² bis 10,1 mg/m² liegt.

2. Pigment gemäß Anspruch 1, wobei das metallische Substrat plättchenförmig ist.

3. Pigment gemäß einem der Ansprüche 1 bis 2, wobei das organische Polymer kein Polyethylen ist.

4. Pigment gemäß einem der Ansprüche 1 bis 3, wobei das Verhältnis der Menge an Metalloxid der Beschichtung zur spezifischen Oberfläche des unbeschichteten Metallpigments in einem Bereich von 17,2 mg/m$^2$ bis 23 mg/m$^2$liegt

5. Pigment gemäß einem der Ansprüche 1 bis 4, wobei das Verhältnis der Menge organischem Polymer der anorganisch/organischen Hybridschicht zur spezifischen Oberfläche des unbeschichteten Metallpigments in einem Bereich von 4,6 mg/m$^2$ bis 9,7 mg/m$^2$ liegt

6. Pigment gemäß einem der Ansprüche 1 bis 5, wobei die Dicke der anorganisch/organischen Hybridschicht mindestens 40 % der Gesamtdicke der Beschichtung beträgt.

7. Pigment gemäß einem der Ansprüche 1 bis 6, wobei die Menge der anorganisch/organischen Hybridschicht mindestens 5 Gew.-% beträgt, bezogen auf das Gesamtgewicht des Pigments.

8. Pigment gemäß einem der Ansprüche 1 bis 7, wobei das mindestens eine Metalloxid der anorganisch/organischen Hybridschicht ausgewählt wird aus der Gruppe bestehend aus Siliciumoxid, Aluminiumoxid, Boroxid, Zirkoniumoxid, Ceroxid, Eisenoxid, Titanoxid, Chromoxid, Zinnoxid, Molybdänoxid, Vanadiumoxid, Zinkoxid, Magnesiumoxid und Mischungen davon, wobei der Begriff Metalloxid auch Oxidhydrate und Hydroxide umfasst.

9. Pigment gemäß einem der Ansprüche 1 bis 8, wobei auf mindestens einer anorganisch/organischen Hybridschicht mindestens ein vorkondensiertes Heteropolysiloxan aufgebracht wurde, wobei das mindestens eine vorkondensierte Heteropolysiloxan mindestens ein Aminosilan und mindestens ein Silan, das ausgewählt wird aus der Gruppe bestehend aus Alkylsilanen, Vinylsilanen und Arylsilanen, umfasst und das Heteropolysiloxan in vorkondensierter Form aufgebracht wurde.

10. Pigment gemäß einem der Ansprüche 1 bis 9, wobei mindestens 95 Gew.-% des Metalls des metallischen Substrats ausgewählt werden aus der Gruppe bestehend aus Aluminium, Eisen, Kupfer und Messing, bezogen auf das Gewicht des Metalls des metallischen Substrats ohne Sauerstoff.

11. Pigment gemäß einem der Ansprüche 1 bis 10, wobei mindestens ein Netzwerkbildner ausgewählt wird aus den Verbindungen gemäß Formel (NI) oder (NII),

$$R^{an1}{}_{xn1}R^{bn1}y_{n1}SiX_{(4-xn1-yn1)} \qquad (NI)$$

$$(R^{an1}O)_{xn2}(R^{bn1}O)_{yn2}MX_{(zn2-xn2-yn2)} \qquad (NII)\ ,$$

wobei die X unabhängig voneinander ausgewählt werden aus hydrolysierbaren Gruppen, nach deren Hydrolyse eine kovalente Bindung von organischem Netzwerkbildner mit dem anorganischen Netzwerk ausgebildet werden kann,
die $R^{an1}$ unabhängig voneinander ausgewählt werden aus reaktiven organischen Gruppen, die mit dem organischen Polymer kovalent verbindbar sind,
die $R^{bn1}$ unabhängig voneinander ausgewählt werden aus organischen Gruppen, die mit dem organischen Polymer kovalent verbindbar sein können,
M ausgewählt wird aus der Gruppe bestehend aus AI, Zr und Ti,
xn1eine ganze Zahl von 1 bis 3 ist, yn1 eine ganze Zahl von 0 bis (3-xn1) ist,
zn2 die formale Oxidationszahl von M ist, xn2 eine ganze Zahl von 1 bis (zn2-1) ist,
yn2 eine ganze Zahl von 0 bis (zn2-2) ist, und
xn2+yn2 $\leq$ zn2-1 ist.

12. Pigment gemäß einem der Ansprüche 1 bis 11, wobei die anorganisch/organische Hybridschicht mindestens ein Netzwerkbildner gemäß Formel (NI) umfasst,
wobei die X unabhängig voneinander ausgewählt werden aus der Gruppe bestehend aus C1-C4 Alkoxygruppen ohne Heteroatome in der Kohlenstoffkette, die $R^{an1}$unabhängig voneinander ausgewählt werden aus der Gruppe bestehend aus substituierten C1-C10 Alkylgruppen, wobei die Substituenten ausgewählt werden aus der Gruppe bestehend aus Acrylatgruppen, Methacrylatgruppen und Mischungen hiervon ,
die $R^{bn1}$ unabhängig voneinander ausgewählt werden aus der Gruppe bestehend aus C1-C24 Alkylgruppen, C6-C18 Arylgruppen, C7-C24 Alkylarylgruppen, C7-C24 Arylalkylgruppen, C5-C16 Cycloalkylgruppen und C6-C20 Alkylcycloalkylgruppen, wobei die vorgenannten Gruppen unsubstituiert sind und in den Kohlenstoffketten und Kohlenstoffringsystemen keine Heteroatome enthalten sind,
xn1eine ganze Zahl von 1 bis 3 ist und

yn1 eine ganze Zahl von 0 oder 1 ist.

13. Pigment gemäß einem der Ansprüche 1 bis 12, wobei das organische Polymer ausgewählt wird aus der Gruppe bestehend aus Polyacrylaten, Polymethacrylaten, Polyethern, Polyestern, Polyaminen, Polyamiden, Polyolen, Polyurethanen und Polyolefinen, wobei die Polyolefine kein Polyethylen umfassen.

14. Verfahren zur Herstellung von Metallpigmenten mit metallischem Substrat und Beschichtung, umfassend folgende Schritte:

   i) Umsetzen mindestens eines Metalloxidedukts, mindestens eines Edukts eines organischen Polymers und mindestens eines Netzwerkbildners in einer Flüssigphase unter Ausbildung einer Beschichtungszusammensetzung,
   ii) Aufbringen der Beschichtungszusammensetzung auf metallische Substrate unter Ausbildung einer anorganisch/organischen Hybridschicht,

   wobei die anorganisch/organische Hybridschicht mindestens ein anorganisches Netzwerk umfassend mindestens ein Metalloxid und mindestens ein organisches Polymer aufweisen und das anorganische Netzwerk und das organische Polymer kovalent miteinander verbunden sind,
   wobei das mindestens eine Metalloxid kein Oxidationsprodukt des metallischen Substrats darstellt und der Begriff Metalloxid Oxide, Hydroxide und Oxidhydrate der Metalle und Halbmetalle umfasst,
   der Netzwerkbildner zumindest teilweise mit dem Metalloxid und dem organischen Polymer verbunden ist und
   das Verhältnis der Menge an Metalloxid der anorganisch/organischen Hybridschicht zur spezifischen Oberfläche des unbeschichteten Metallpigments in einem Bereich von 16,1 mg/m$^2$ bis 25 mg/m$^2$ liegt und
   das Verhältnis der Menge organischem Polymer der anorganisch/organischen Hybridschicht zur spezifischen Oberfläche des unbeschichteten Metallpigments in einem Bereich von 3,9 mg/m$^2$ bis 10,1 mg/m$^2$ liegt.

15. Verfahren gemäß Anspruch 14, wobei auf mindestens eine anorganisch/organische Hybridschicht mindestens einer Beschichtungsschicht umfassend mindestens ein vorkondensiertes Heteropolysiloxan aufgebracht wird, wobei der Begriff Metalloxid auch Oxidhydrate und Hydroxide umfasst und das mindestens eine vorkondensierte Heteropolysiloxan mindestens ein Aminosilan und mindestens ein Silan umfasst, dass ausgewählt wird aus der Gruppe der Alkylsilane, Vinylsilane und Arylsilane.

16. Verfahren gemäß einem der Ansprüche 14 bis 15, wobei die Umsetzung der Edukte der anorganisch/organischen Hybridschicht entweder

   a) zunächst in einem pH-Wertbereich von 2,5 bis 6,8 und später in einem pH-Wertbereich von 7,2 bis 11,5 oder
   b) zunächst in einem pH-Wertbereich von 7,2 bis 11,5 und später in einem pH-Wertbereich von 2,5 bis 6,8

   durchgeführt wird.

17. Verwendung der Pigmente gemäß einem der Ansprüche 1 bis 13 in einem Kosmetikum, einem Kunststoff oder einem Beschichtungsmittel.

**Claims**

1. Pigment comprising a metallic substrate and at least one inorganic/organic hybrid layer,
   wherein the inorganic/organic hybrid layer comprises at least one metal oxide, at least one network former, and at least one organic polymer,
   wherein the at least one metal oxide does not constitute an oxidation product of the metallic substrate, and the term "metal oxide" comprises oxides, hydroxides, and oxide hydrates of the metals and semimetals,
   the network former is joined at least partially covalently to the metal oxide and to the organic polymer,
   wherein the ratio of the amount of metal oxide of the inorganic/organic hybrid layer to the specific surface area of the uncoated metal pigment is in a range from 16.1 mg/m$^2$ to 25 mg/m$^2$, and
   the ratio of the amount of organic polymer of the inorganic/organic hybrid layer to the specific surface area of the uncoated metal pigment is in a range from 3.9 mg/m$^2$ to 10.1 mg/m$^2$.

2. Pigment according to Claim 1, wherein the metallic substrate is platelet-shaped.

3. Pigment according to either of Claims 1 and 2, wherein the organic polymer is not polyethylene.

4. Pigment according to any of Claims 1 to 3, wherein the ratio of the amount of metal oxide of the coating to the specific surface area of the uncoated metal pigment is in a range from 17.2 mg/m$^2$ to 23 mg/m$^2$.

5. Pigment according to any of Claims 1 to 4, wherein the ratio of the amount of organic polymer of the inorganic/organic hybrid layer to the specific surface area of the uncoated metal pigment is in a range from 4.6 mg/m$^2$ to 9.7 mg/m$^2$.

6. Pigment according to any of Claims 1 to 5, wherein the thickness of the inorganic/organic hybrid layer is at least 40% of the total thickness of the coating.

7. Pigment according to any of Claims 1 to 6, wherein the amount of the inorganic/organic hybrid layer is at least 5 wt%, based on the total weight of the pigment.

8. Pigment according to any of Claims 1 to 7, wherein the at least one metal oxide of the inorganic/organic hybrid layer is selected from the group consisting of silicon oxide, aluminum oxide, boron oxide, zirconium oxide, cerium oxide, iron oxide, titanium oxide, chromium oxide, tin oxide, molybdenum oxide, vanadium oxide, zinc oxide, magnesium oxide, and mixtures thereof, wherein the term "metal oxide" also comprises oxide hydrates and hydroxides.

9. Pigment according to any of Claims 1 to 8, wherein at least one precondensed heteropolysiloxane has been applied to at least one inorganic/organic hybrid layer,
wherein the at least one precondensed heteropolysiloxane comprises at least one aminosilane and at least one silane selected from the group consisting of alkylsilanes, vinylsilanes, and arylsilanes, and the heteropolysiloxane has been applied in precondensed form.

10. Pigment according to any of Claims 1 to 9, wherein at least 95 wt% of the metal of the metallic substrate are selected from the group consisting of aluminum, iron, copper, and brass, based on the weight of the metal of the metallic substrate without oxygen.

11. Pigment according to any of Claims 1 to 10, wherein at least one network former is selected from the compounds of formula (NI) or (NII),

$$R^{an1}_{xn1}R^{bn1}_{yn1}SiX_{(4-xn1-yn1)} \qquad (NI)$$

$$(R^{an1}O)_{xn2}(R^{bn1}O)_{yn2}MX_{(zn2-xn2-yn2)} \qquad (NII) \, ,$$

wherein the Xs independently of one another are selected from hydrolyzable groups after whose hydrolysis a covalent bond of organic network former to the inorganic network can be formed,
the $R^{an1}$s independently of one another are selected from reactive organic groups which can be joined covalently to the organic polymer,
the $R^{bn1}$s independently of one another are selected from organic groups which can be joined covalently to the organic polymer,
M is selected from the group consisting of Al, Zr, and Ti,
xn1 is an integer from 1 to 3, yn1 is an integer from 0 to (3-xnl),
zn2 is the formal oxidation number of M, xn2 is an integer from 1 to (zn2-1),
yn2 is an integer from 0 to (zn2-2), and
xn2+yn2 $\leq$ zn2-1.

12. Pigment according to any of Claims 1 to 11, wherein the inorganic/organic hybrid layer comprises at least one network former of formula (NI),
wherein the Xs independently of one another are selected from the group consisting of C1-C4 alkoxy groups without heteroatoms in the carbon chain,
the $R^{an1}$s independently of one another are selected from the group consisting of substituted C1-C10 alkyl groups, wherein the substituents are selected from the group consisting of acrylate groups, methacrylate groups and mixtures thereof,
the $R^{bn1}$s independently of one another are selected from the group consisting of C1-C24 alkyl groups, C6-C18 aryl groups, C7-C24 alkylaryl groups, C7-C24 arylalkyl groups, C5-C16 cycloalkyl groups, and C6-C20 alkylcycloalkyl groups, wherein the aforesaid groups are unsubstituted and contain no heteroatoms in the carbon chains and carbon ring systems,
xn1 is an integer from 1 to 3, and
yn1 is an integer 0 or 1.

13. Pigment according to any of Claims 1 to 12, wherein the organic polymer is selected from the group consisting of polyacrylates, polymethacrylates, polyethers, polyesters, polyamines, polyamides, polyols, polyurethanes, and polyolefins, the polyolefins including no polyethylene.

14. Method for producing metal pigments comprising metallic substrate and coating, comprising the following steps:

   i) reacting at least one metal oxide reactant, at least one reactant of an organic polymer, and at least one network former in a liquid phase to form a coating composition,
   ii) applying the coating composition to metallic substrates to form an inorganic/organic hybrid layer, wherein the inorganic/organic hybrid layer comprises at least one inorganic network comprising at least one metal oxide and at least one organic polymer, and the inorganic network and the organic polymer are joined covalently to one another,

   wherein the at least one metal oxide does not constitute an oxidation product of the metallic substrate, and the term "metal oxide" comprises oxides, hydroxides, and oxide hydrates of the metals and semimetals,
   the network former is joined at least partially to the metal oxide and to the organic polymer, and
   the ratio of the amount of metal oxide of the inorganic/organic hybrid layer to the specific surface area of the uncoated metal pigment is in a range from 16.1 $mg/m^2$ to 25 $mg/m^2$, and
   the ratio of the amount of organic polymer of the inorganic/organic hybrid layer to the specific surface area of the uncoated metal pigment is in a range from 3.9 $mg/m^2$ to 10.1 $mg/m^2$.

15. Method according to Claim 14, wherein at least one coating layer comprising at least one precondensed heteropolysiloxane is applied to at least one inorganic/organic hybrid layer,
   wherein the term "metal oxide" also comprises oxide hydrates and hydroxides, and the at least one precondensed heteropolysiloxane comprises at least one aminosilane and at least one silane selected from the group of alkylsilanes, vinylsilanes, and arylsilanes.

16. Method according to either of Claims 14 and 15, wherein the reaction of the reactants of the inorganic/organic hybrid layer is carried out either

   a) first in a pH range from 2.5 to 6.8 and later in a pH range from 7.2 to 11.5, or
   b) first in a pH range from 7.2 to 11.5 and later in a pH range from 2.5 to 6.8.

17. Use of the pigments according to any of Claims 1 to 13 in a cosmetic, a plastic or a coating material.

**Revendications**

1. Pigment comprenant un substrat métallique et au moins une couche hybride inorganique/organique,
   la couche hybride inorganique/organique comprenant au moins un oxyde métallique, au moins un formateur de réseau et au moins un polymère organique,
   l'au moins un oxyde métallique ne représentant aucun produit d'oxydation du substrat métallique et le terme « oxyde métallique » comprenant des oxydes, des hydroxydes et des hydrates d'oxyde des métaux et des semi-métaux,
   le formateur de réseau étant lié au moins partiellement de manière covalente avec l'oxyde métallique et le polymère organique,
   le rapport de la quantité d'oxyde métallique de la couche hybride inorganique/organique sur la surface spécifique du pigment métallique non revêtu se situant dans une plage de 16,1 $mg/m^2$ à 25 $mg/m^2$ et
   le rapport de la quantité de polymère organique de la couche hybride inorganique/organique sur la surface spécifique du pigment métallique non revêtu se situant dans une plage de 3,9 $mg/m^2$ à 10,1 $mg/m^2$.

2. Pigment selon la revendication 1, le substrat métallique étant en forme de plaquette.

3. Pigment selon l'une quelconque des revendications 1 et 2, le polymère organique n'étant pas un polyéthylène.

**4.** Pigment selon l'une quelconque des revendications 1 à 3, le rapport de la quantité d'oxyde métallique du revêtement sur la surface spécifique du pigment métallique non revêtu se situant dans une plage de 17,2 mg/m$^2$ à 23 mg/m$^2$.

**5.** Pigment selon l'une quelconque des revendications 1 à 4, le rapport de la quantité de polymère organique de la couche hybride inorganique/organique sur la surface spécifique du pigment métallique non revêtu se situant dans une plage de 4,6 mg/m$^2$ à 9,7 mg/m$^2$.

**6.** Pigment selon l'une quelconque des revendications 1 à 5, l'épaisseur de la couche hybride inorganique/organique étant d'au moins 40 % de l'épaisseur totale du revêtement.

**7.** Pigment selon l'une quelconque des revendications 1 à 6, la quantité de la couche hybride inorganique/organique étant d'au moins 5 % en poids, par rapport au poids total du pigment.

**8.** Pigment selon l'une quelconque des revendications 1 à 7, l'au moins un oxyde métallique de la couche hybride inorganique/organique étant choisi dans le groupe constitué par l'oxyde de silicium, l'oxyde d'aluminium, l'oxyde de bore, l'oxyde de zirconium, l'oxyde de cérium, l'oxyde de fer, l'oxyde de titane, l'oxyde de chrome, l'oxyde d'étain, l'oxyde de molybdène, l'oxyde de vanadium, l'oxyde de zinc, l'oxyde de magnésium et des mélanges correspondants, le terme « oxyde métallique » comprenant également les hydrates d'oxyde et des hydroxydes.

**9.** Pigment selon l'une quelconque des revendications 1 à 8, au moins un hétéropolysiloxane précondensé ayant été appliqué sur au moins une couche hybride inorganique/organique, l'au moins un hétéropolysiloxane précondensé comprenant au moins un aminosilane et au moins un silane, qui est choisi dans le groupe constitué par les alkylsilanes, les vinylsilanes et les arylsilanes, et l'hétéropolysiloxane ayant été appliqué sous forme précondensée.

**10.** Pigment selon l'une quelconque des revendications 1 à 9, au moins 95 % en poids du métal du substrat métallique étant choisis dans le groupe constitué par l'aluminium, le fer, le cuivre et le laiton, par rapport au poids du métal du substrat métallique sans oxygène.

**11.** Pigment selon l'une quelconque des revendications 1 à 10, au moins un formateur de réseau étant choisi parmi les composés selon la formule (NI) et (NII) ,

$$R^{an1}_{xn1}R^{bn1}_{yn1}SiX_{(4-xn1-yn1)} \qquad \text{(NI)}$$

$$(R^{an1}O)_{xn2}(R^{bn1}O)_{yn2}MX_{(zn2-xn2-yn2)} \qquad \text{(NII)},$$

les X étant choisis indépendamment les uns des autres parmi des groupes hydrolysables, après l'hydrolyse desquels une liaison covalente du formateur de réseau organique avec le réseau inorganique peut être formée, les $R^{an1}$ étant choisis indépendamment les uns des autres parmi des groupes organiques réactifs qui peuvent être liés de manière covalente avec le polymère organique, les $R^{bn1}$ étant choisis indépendamment les uns des autres parmi des groupes organiques qui peuvent être liés de manière covalente avec le polymère organique, M étant choisi dans le groupe constitué par Al, Zr et Ti, xn1 étant un nombre entier de 1 à 3, yn1 étant un nombre entier de 0 à (3 - xn1), zn2 étant le nombre d'oxydation formel de M, xn2 étant un nombre entier de 1 à (zn2 - 1), yn2 étant un nombre entier de 0 à (zn2 - 2), et xn2 + yn2 $\leq$ zn2 - 1.

**12.** Pigment selon l'une quelconque des revendications 1 à 11, la couche hybride inorganique/organique comprenant au moins un formateur de réseau selon la formule (NI), les X étant choisis indépendamment les uns des autres dans le groupe constitué par des groupes $C_{1-4}$-alcoxy sans hétéroatomes dans la chaîne carbonée, les $R^{an1}$ étant choisis indépendamment les uns des autres dans le groupe constitué par des groupes $C_{1-10}$-alkyle substitués, les substituants étant choisis dans le groupe constitué par des groupes acrylate, des groupes méthacrylate et des mélanges correspondants, les $R^{bn1}$ étant choisis indépendamment les uns des autres dans le groupe constitué par des groupes $C_{1-24}$-alkyle, des groupes $C_{6-18}$-aryle, des groupes C7-24-alkylaryle, des groupes $C_{7-24}$-arylalkyle, des groupes $C_{5-16}$-cycloalkyle et des groupes $C_{6-20}$-alkylcycloalkyle, les groupes mentionnés précédemment étant non substitués et ne contenant aucun hétéroatome dans la chaîne carbonée et les systèmes cycliques carbonés, xn1 étant un nombre entier de 1 à 3 et

yn1 étant un nombre entier de 0 ou 1.

13. Pigment selon l'une quelconque des revendications 1 à 12, le polymère organique étant choisi dans le groupe constitué par les polyacrylates, les polyméthacrylates, les polyéthers, les polyesters, les polyamines, les polyamides, les polyols, les polyuréthanes et les polyoléfines, les polyoléfines ne comprenant aucun polyéthylène.

14. Procédé pour la préparation de pigments métalliques dotés d'un substrat métallique et d'un revêtement, comprenant les étapes suivantes :

i) transformation d'au moins un éduit d'oxyde métallique, d'au moins un éduit d'un polymère organique et d'au moins un formateur de réseau dans une phase liquide avec formation d'une composition de revêtement,
ii) application de la composition de revêtement sur des substrats métalliques avec formation d'une couche hybride inorganique/organique,
la couche hybride inorganique/organique présentant au moins un réseau inorganique comprenant au moins un oxyde métallique et au moins un polymère organique et le réseau inorganique et le polymère organique étant liés l'un à l'autre de manière covalente,
l'au moins un oxyde métallique ne représentant aucun produit d'oxydation du substrat métallique et le terme « oxyde métallique » comprenant des oxydes, des hydroxydes et des hydrates d'oxyde des métaux et des semi-métaux,
le formateur de réseau étant lié au moins partiellement avec l'oxyde métallique et le polymère organique et
le rapport de la quantité d'oxyde métallique de la couche hybride inorganique/organique sur la surface spécifique du pigment métallique non revêtu se situant dans une plage de 16,1 mg/m$^2$ à 25 mg/m$^2$ et
le rapport de la quantité de polymère organique de la couche hybride inorganique/organique sur la surface spécifique du pigment métallique non revêtu se situant dans une plage de 3,9 mg/m$^2$ à 10,1 mg/m$^2$.

15. Procédé selon la revendication 14, au moins une couche de revêtement comprenant au moins un hétéropolysiloxane précondensé étant appliquée sur au moins une couche hybride inorganique/organique, le terme « oxyde métallique » comprenant également les hydrates d'oxyde et les hydroxydes et l'au moins un hétéropolysiloxane précondensé comprenant au moins un aminosilane et au moins un silane, qui est choisi dans le groupe constitué des alkylsilanes, des vinylsilanes et des arylsilanes.

16. Procédé selon l'une quelconque des revendications 14 et 15, la transformation des éduits de la couche hybride inorganique/organique étant mise en œuvre

a) soit d'abord dans un domaine de valeur de pH de 2,5 à 6,8 et plus tard dans un domaine de valeur de pH de 7,2 à 11,5,
b) soit d'abord dans un domaine de valeur de pH de 7,2 à 11,5 et plus tard dans un domaine de valeur de pH de 2,5 à 6,8.

17. Utilisation des pigments selon l'une quelconque des revendications 1 à 13 dans un produit cosmétique, un plastique ou un agent de revêtement.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013064643 A1
- WO 2014048887 A1
- US 20080249209 A1
- CN 102516831
- DE 102005037611
- US 8574357 B2
- WO 1996038505 A1
- WO 2012130680 A1
- US 20070199478 A1

- US 20110179971 A1
- US 20100047199 A1
- EP 1613702 B1
- EP 2102294 B1
- EP 2128203 A1
- US 2010047199 A1
- US 5808125 A
- US 5679147 A
- US 5629400 A

**In der Beschreibung aufgeführte Nicht-Patentliteratur**

- Organofunctional alkoxysilanes in dilute aqueous solution: new accounts on the dynamic structural mutability. *Journal of Organometallic Chemistry,* 2001, vol. 625, 208-216